# EUROPEAN PATENT SPECIFICATION

(11) **EP 2 374 894 B1**
(45) Date of publication and mention of the grant of the patent: **17.09.2014**
(21) Application number: 11159899.1
(22) Date of filing: 26.05.1995
(51) Int. Cl.: C12N 15/866, C12N 15/62, C07K 14/11, A61K 39/145

(54) **A method for producing influenza hemagglutinin multivalent vaccines**
Verfahren zum Herstellen von hämaglutinin-multivalenten Influenza-Impfstoffen
Procédé pour la production de vaccins polyvalents contre l'hémagglutinine de la grippe

(43) Date of publication of application: 12.10.2011
(62) Divisional of application: 05076536.1
(73) Proprietor: Protein Sciences Corporation, Meriden, CT 06450 (US)
(72) Inventor: Smith, Gale Eugene, Gaithersburg, MD 28078-3401 (US); Volvovitz, Franklin, New Haven, CT 06457 (US); Wilkinson, Bethanie E., Middletown, CT 06457 (US); Voznesensky, Andrei I., West Hartford, CT 06107 (US); Hackett, Craig Stanway, Wallingford, CT 06492 (US)
(74) Representative: Van Someren, Petronella F. H. M.

(56) References cited:
- EP-A- 0 505 207
- EP-A- 0 546 787
- WO-A-88/07082
- WO-A-95/32286
- WO-A1-96/37624
- US-A- 4 659 669
- US-A- 4 752 473
- POSSEE R D: "CELL-SURFACE EXPRESSION OF INFLUENZA VIRUS HAEMAGGLUTININ IN INSECTCELLS USING A BACULOVIRUS VECTOR", VIRUS RESEARCH, AMSTERDAM, NL, vol. 5, no. 1, 1 July 1986 (1986-07-01), pages 43-59, XP000561792, ISSN: 0168-1702
- MATSUURA Y ET AL: "Baculovirus expression vectors: the requirements for high level expression of proteins, including glycoproteins", JOURNAL OF GENERAL VIROLOGY, SOCIETY FOR GENERAL MICROBIOLOGY, SPENCERS WOOD, GB, vol. 68, 1 May 1987 (1987-05-01), pages 1233-1250, XP000561791, ISSN: 0022-1317
- KURODA K ET AL: "EXPRESSION OF THE INFLUENZA VIRUS HEMAGGLUTININ IN INSECT CELLS BY A BACULOVIRUS VECTOR", EMBO JOURNAL, OXFORD UNIVERSITY PRESS, SURREY, GB, vol. 5, no. 6, 1 January 1986 (1986-01-01) , pages 1359-1366, XP000610287, ISSN: 0261-4189
- AYRES M D ET AL: "The complete DNA sequence of Autographa californica nuclear polyhedrosis virus", VIROLOGY, ACADEMIC PRESS,ORLANDO, US, vol. 202, no. 2, 1 January 1994 (1994-01-01), pages 586-605, XP002225673, ISSN: 0042-6822
- WILEY D C ET AL: "THE STRUCTURE AND FUNCTION OF THE HEMAGGLUTININ MEMBRANE GLYCOPROTEIN OF INFLUENZA VIRUS", ANNUAL REVIEW OF BIOCHEMISTRY, PALTO ALTO, CA, US, vol. 56, 1 January 1987 (1987-01-01), pages 365-394, XP000826411, ISSN: 0066-4154
- LUCKOW V A ET AL: "TRENDS IN THE DEVELOPMENT OF BACULOVIRUS EXPRESSION VECTORS", BIO/TECHNOLOGY, NATURE PUBLISHING CO. NEW YORK, US, vol. 6, no. 1, 1 January 1988 (1988-01-01) , pages 47-55, XP009004383, ISSN: 0733-222X
- HAWTIN R E ET AL: "Identification and preliminary characterization of a chitinase gene in the Autographa californica nuclear polyhedrosis virus genome.", VIROLOGY 1 OCT 1995, vol. 212, no. 2, 1 October 1995 (1995-10-01), pages 673-685, XP002486154, ISSN: 0042-6822
- POWER D C ET AL: "INFLUENZA A VIRUS VACCINES CONTAINING PURIFIED RECOMBINANT H3 HEMAGGLUTININ ARE WELL TOLERATED AND INDUCE PROTECTIVE IMMUNE RESPONSES IN HEALTHY ADULTS", JOURNAL OF INFECTIOUS DISEASES, CHICAGO, IL, vol. 171, no. 6, 1 June 1995 (1995-06-01), pages 1595-1599, XP000561789, ISSN: 0022-1899

### SEQUENCE LISTING

(1) GENERAL INFORMATION:
   (i) APPLICANT: MicroGeneSys, Inc.
   (ii) TITLE OF INVENTION: A METHOD FOR PRODUCING INFLUENZA HEMAGGLUTININ MULTIVALENT VACCINES
   (iii) NUMBER OF SEQUENCES: 32
   (iv) CORRESPONDENCE ADDRESS:
      (A) ADDRESSEE: Patrea L. Pabst
      (B) STREET: 2800 One Atlantic Center 1201 West Peachtree Street
      (C) CITY: Atlanta
      (D) STATE: GA
      (E) COUNTRY: USA
      (F) ZIP: 30309-3450
   (v) COMPUTER READABLE FORM:
      (A) MEDIUM TYPE: Floppy disk
      (B) COMPUTER: IBM PC compatible
      (C) OPERATING SYSTEM: PC-DOS/HS-POS
      (D) SOFTWARE: PatentIn Release #1.0, Version #1.25
   (vi) CURRENT APPLICATION DATA:
      (A) APPLICATION NUMBER: PCT/US95/06750
      (B) FILING DATE: 26-MAY-1995
      (C) CLASSIFICATION:
   (ix) TELECOMMUNICATION INFORMATION: (A) TELEPHONE: (404)-873-8794 (B) TELEFAX: (404) - 873-8795
(2) INFORMATION FOR SEQ ID NO:1:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Influenza virus
   (x) PUBLICATION INFORMATION:
      (A) AUTHORS: Davis, et al.
      (B) TITLE: Construction and Characterization of a Bacterial Clone Containing the Hemagglutinin Gene of the WSN Strain (HON1) of Influenza Virus
      (C) JOURNAL: Gene
      (D) VOLUME: 10
      (F) PAGES: 205-218
      (G) DATE: 1980
   (xi) SEQUENCE DESCRIPTION: SEQ ID NO:1:
      AGCAAAAGCA GG 12
(2) INFORMATION FOR SEQ ID NO:2:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
      GGGGGTACCC CCGGGAGCAA AAGCAGGGGA AAATAAAAA 39
(2) INFORMATION FOR SEQ ID NO:3:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION SEQ ID NO:3:
      CCCGGTACCT CAKATKCATA TTCTGCACTG CAAAG 35
(2) INFORMATION FOR SEQ ID NO:4:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION SEQ ID NO:4:
      GGGGGTACCC CCGGGGACAC AATATGTATA GGCTACCAT 39
(2) INFORMATION FOR SEQ ID NO:5:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
      CCCGGTACCT CAKATKCATA TTCTGCACTG CAAAG 35
(2) INFORMATION FOR SEQ ID NO:6:
   (i) SEQUENCE CHARACTERISTICS:
      (A) LENGTH: 1793 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Influenza virus
      (C) INDIVIDUAL ISOLATE: A/Bejing/32/92 rHA
   (ix) FEATURE
      (A) NAME/KEY: polyhedrin mRNA leader (partial)
      (B) LOCATION: 1 to 18
   (ix) FEATURE
      (A) NAME/KEY: coding region for AcNPV 61K protein signal sequence
      (B) LOCATION: 19 to 72
   (ix) FEATURE
      (A) NAME/KEY: smaI restriction site
      (B) LOCATION: 76 to 81
   (ix) FEATURE
      (A) NAME/KEY: coding region for mature rHA
      (B) LOCATION: 73 to 1728
   (ix) FEATURE
      (A) NAME/KEY: KpnI restriction site
      (B) LOCATION: 1771 to 1777
   (ix) FEATURE
      (A) NAME/KEY: BglII restriction site
      (B) LOCATION: 1776 to 1782
   (ix) FEATURE
      (A) NAME/KEY: unversal translation termination signal
      (B) LOCATION: 1783 to 1793
   (xi) SEQUENCE DESCRIPTION: SEQ ID NO:6:
   TAAAAAAACC TATAAATAAT GCCCTTGTAC AAATTGTTAA ACGTTTTGTG GTTGGTCGCC      60
   GTTTCTAACG CGATTCCCGG GGACTTTCCA GGAAATGACA ACAGCACAGC AACGCTGTGC     120
   CTGGGACATC ATGCAGTGCC AAACGGAACG CTAGTGAAAA CAATCACGAA TGATCAAATT    180
   GAAGTGACTA ATGCTACTGA GCTGGTTCAG AGTTCCTCAA CAGGTAGAAT ATGCGACAGT    240
   CCTCACCGAA TCCTTGATGG AAAAAACTGC ACACTGATAG ATGCTCTATT GGGAGACCCT    300
   CATTGTGATG GCTTCCAAAA TAAGGAATGG GACCTTTTTG TTGAACGCAG CAAAGCTTAC    360
   AGCAACTGTT ACCCTTATGA TGTACCGGAT TATGCCTCCC TTAGGTCACT AGTTGCCTCA    420
   TCAGGCACCC TGGAGTTTAT CAATGAAGAC TTCAATTGGA CTGGAGTCGC TCAGGATGGG    480
   GGAAGCTATG CTTGCAAAAG GGGATCTGTT AACAGTTTCT TTAGTAGATT GAATTGGTTG    540
   CACAAATCAG AATACAAATA TCCAGCGCTG AACGTGACTA TGCCAAACAA TGGCAAATTT    600
   GACAAATTGT ACATTTGGGG GGTTCACCAC CCGAGCACGG ACAGAGACCA AACCAGCCTA    660
   TATGTTCGAG CATCAGGGAG AGTCACAGTC TCTACCAAAA GAAGCCAACA AACTGTAACC    720
   CCGAATATCG GGTCTAGACC CTGGGTAAGG GGTCAGTCCA GTAGAATAAG CATCTATTGG    780
   ACAATAGTAA AACCGGGAGA CATACTTTTG ATTAATAGCA CAGGGAATCT AATTGCTCCT    840
   CGGGGTTACT TCAAAATACG AAATGGGAAA AGCTCAATAA TGAGGTCAGA TGCACCCATT    900
   GGCACCTGCA GTTCTGAATG CATCACTCCA AATGGAAGCA TTCCCAATGA CAAACCTTTT    960
   CAAAATGTAA ACAGGATCAC ATATGGGGCC TGCCCCAGAT ATGTTAAGCA AAACACTCTG   1020
   AAATTGGCAA CAGGGATGCG GAATGTACCA GAGAAACAAA CTAGAGGCAT ATTCGGCGCA   1080
   ATCGCAGGTT TCATAGAAAA TGGTTGGGAG GGAATGGTAG ACGGTTGGTA CGGTTTCAGG   1140
   CATCAAAATT CTGAGGGCAC AGGACAAGCA GCAGATCTTA AAAGCACTCA AGCAGCAATC   1200
   GACCAAATCA ACGGGAAACT GAATAGGTTA ATCGAGAAAA CGAACGAGAA ATTCCATCAA   1260
   ATCGAAAAAG AATTCTCAGA AGTAGAAGGG AGAATTCAGG ACCTCGAGAA ATATGTTGAA   1320
   GACACTAAAA TAGATCTCTG GTCTTACAAC GCGGAGCTTC TTGTTGCCCT GGAGAACCAA   1380
   CATACAATTG ATCTAACTGA CTCAGAAATG AACAAACTGT TTGAAAAAAC AAGGAAGCAA   1440
   CTGAGGGAAA ATGCTGAGGA CATGGGCAAT GGTTGCTTCA AAATATACCA CAAATGTGAC   1500
   AATGCCTGCA TAGGGTCAAT CAGAAATGGA ACTTATGACC ATGATGTATA CAGAGACGAA   1560
   GCATTAAACA ACCGGTTCCA GATCAAAGGT GTTGAGCTGA AGTCAGGATA CAAAGATTGG   1620
   ATCCTATGGA TTTCCTTTGC CATATCATGC TTTTTGCTTT GTGTTGTTTT GCTGGGGTTC   1680
   ATCATGTGGG CCTGCCAAAA AGGCAACATT AGGTGCAACA TTTGCATTTG AGTGTATTAA   1740
   TTAAAAACAC CCTTGTTTCT AGGATGATTC GGTACCAGAT CTTAATTAAT TAA          1793
(2) INFORMATION FOR SEQ ID NO:7:
   (i) SEQUENCE CHARACTERISTICS:
      (A) LENGTH: 570 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Influenza virus
      (C) INDIVIDUAL ISOLATE: A/Bejing/32/92 rHA
   (ix) FEATURE
      (A) NAME/KEY: AcNPV 61K protein signal sequence
      (B) LOCATION: 1 to 18
   fix) FEATURE
      (A) NAME/KEY: mature rHA
      (B) LOCATION: 19 to 552
   (xi) SEQUENCE DESCRIPTION: SEQ ID NO:7:
   Met Pro Leu Tyr Lys Leu Leu Asn Val Leu Trp Leu Val Ala Val Ser
   1 5 10 15
   Asn Ala Ile Pro Gly Asp Phe Pro Gly Asn Asp Asn Ser Thr Ala Thr
   20 25 30
   Leu Cys Leu Gly His His Ala Val Pro Asn Gly Thr Leu Val Lys Thr
   35 40 45
   Ile Thr Asn Asp Gln Ile Glu Val Thr Asn Ala Thr Glu Leu Val Gln
   50 55 60
   Ser Ser Ser Thr Gly Arg Ile Cys Asp Ser Pro His Arg Ile Leu Asp
   65 70 75 80
   Gly Lys Asn Cys Thr Leu Ile Asp Ala Leu Leu Gly Asp Pro His Cys
   85 90 95
   Asp Gly Phe Gln Asn Lys Glu Trp Asp Leu Phe Val Glu Arg Ser Lys
   100 105 110
   Ala Tyr Ser Asn Cys Tyr Pro Tyr Asp Val Pro Asp Tyr Ala Ser Leu
   115 120 125
   Arg Ser Leu Val Ala Ser Ser Gly Thr Leu Glu Phe Ile Asn Glu Asp
   130 135 140
   Phe Asn Trp Thr Gly Val Ala Gln Asp Gly Gly Ser Tyr Ala Cys Lys
   145 150 155 160
   Arg Gly Ser Val Asn Ser Phe Phe Ser Arg Leu Asn Trp Leu His Lys
   165 170 175
   Ser Glu Tyr Lys Tyr Pro Ala Leu Asn Val Thr Met Pro Asn Asn Gly
   180 185 190
   Lys Phe Asp Lys Leu Tyr Ile Trp Gly Val His His Pro Ser Thr Asp
   195 200 205
   Arg Asp Gln Thr Ser Leu Tyr Val Arg Ala Ser Gly Arg Val Thr Val
   210 215 220
   Ser Thr Lys Arg Ser Gln Gln Thr Val Thr Pro Asn Ile Gly Ser Arg
   225 230 235 240
   Pro Trp Val Arg Gly Gln Ser Ser Arg Ile Ser Ile Tyr Trp Thr Ile
   245 250 255
   Val Lys Pro Gly Asp Ile Leu Leu Ile Asn Ser Thr Gly Asn Leu Ile
   260 265 270
   Ala Pro Arg Gly Tyr Phe Lys Ile Arg Asn Gly Lys Ser Ser Ile Met
   275 280 285
   Arg Ser Asp Ala Pro Ile Gly Thr Cys Ser Ser Glu Cys Ile Thr Pro
   290 295 300
   Asn Gly Ser Ile Pro Asn Asp Lys Pro Phe Gln Asn Val Asn Arg Ile
   305 310 315 320
   Thr Tyr Gly Ala Cys Pro Arg Tyr Val Lys Gln Asn Thr Leu Lys Leu
   325 330 335
   Ala Thr Gly Met Arg Asn Val Pro Glu Lys Gln Thr Arg Gly Ile Phe
   340 345 350
   Gly Ala Ile Ala Gly Phe Ile Glu Asn Gly Trp Glu Gly Met Val Asp
   355 360 365
   Gly Trp Tyr Gly Phe Arg His Gln Asn Ser Glu Gly Thr Gly Gln Ala
   370 375 380
   Ala Asp Leu Lys Ser Thr Gln Ala Ala Ile Asp Gln Ile Asn Gly Lys
   385 390 395 400
   Leu Asn Arg Leu Ile Glu Lys Thr Asn Glu Lys Phe His Gln Ile Glu
   405 410 415
   Lys Glu Phe Ser Glu Val Glu Gly Arg Ile Gln Asp Leu Glu Lys Tyr
   420 425 430
   Val Glu Asp Thr Lys Ile Asp Leu Trp Ser Tyr Asn Ala Glu Leu Leu
   435 440 445
   Val Ala Leu Glu Asn Gln His Thr Ile Asp Leu Thr Asp Ser Glu Met
   450 455 460
   Asn Lys Leu Phe Glu Lys Thr Arg Lys Gln Leu Arg Glu Asn Ala Glu
   465 470 475 480
   Asp Met Gly Asn Gly Cys Phe Lys Ile Tyr His Lys Cys Asp Asn Ala
   485 490 495
   Cys Ile Gly Ser Ile Arg Asn Gly Thr Tyr Asp His Asp Val Tyr Arg
   500 505 510
   Asp Glu Ala Leu Asn Asn Arg Phe Gln Ile Lys Gly Val Glu Leu Lys
   515 520 525
   Ser Gly Tyr Lys Asp Trp Ile Leu Trp Ile Ser Phe Ala Ile Ser Cys
   530 535 540
   Phe Leu Leu Cys Val Val Leu Leu Gly Phe Ile Met Trp Ala Cys Gln
   545 550 555 560
   Lys Gly Asn Ile Arg Cys Asn Ile Cys Ile
   565 570
(2) INFORMATION FOR SEQ ID NO:8:
   (i) SEQUENCE CHARACTERISTICS:
      (A) LENGTH: 1766 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Influenza virus
      (C) INDIVIDUAL ISOLATE: A/Texas/36/91 rHA
   (ix) FEATURE
      (A) NAME/KEY: polyhedrin mRNA leader (partial)
      (B) LOCATION: 1 to 18
   (ix) FEATURE
      (A) NAME/KEY: coding region for AcNPV 61K protein signal peptide
      (B) LOCATION: 19 to 72
   (ix) FEATURE
      (A) NAME/KEY: SmaI restriction site
      (B) LOCATION: 76 to 81
   (ix) FEATURE
      (A) NAME/KEY: KpnI restriction site
      (B) LOCATION: 82 to 87
   (ix) FEATURE
      (A) NAME/KEY: SmaI restriction site
      (B) LOCATION: 88 to 93
   (ix) FEATURE
      (A) NAME/KEY: coding region for mature rHA
      (B) LOCATION: 73 to 1734
   (ix) FEATURE
      (A) NAME/KEY: KpnI restriction site
      (B) LOCATION: 1744 to 1749
   (ix) FEATURE
      (A) NAME/KEY: BglII restriction site
      (B) LOCATION: 1750 to 1755
   (ix) FEATURE
      (A) NAME/KEY: unversal translation termination signal
      (B) LOCATION: 1756 to 1766
   (xi) SEQUENCE DESCRIPTION: SEQ ID NO:8:
   TAAAAAAACC TATAAATAAT GCCCTTGTAC AAATTGTTAA ACGTTTTGTG GTTGGTCGCC     60
   GTTTCTAACG CGATTCCCGG GGGTACCCCC GGGGACACAA TATGTATAGG CTACCATGCG    120
   AACAACTCAA CCGACACTGT TGACACAGTA CTTGAGAAGA ACGTGACAGT GACACACTCT    180
   GTCAACCTAC TTGAGGACAG TCACAACGGA AAACTATGTC GACTAAAGGG AATAGCCCCA    240
   CTACAATTGG GTAATTGCAG CGTTGCCGGA TGGATCTTAG GAAACCCAAA ATGCGAATCA    300
   CTGTTTTCTA AGGAATCATG GTCCTACATT GCAGAAACAC CAAACCCTGA GAATGGAACA    360
   TGTTACCCAG GGTATTTCGC CGACTATGAG GAACTGAGGG AGCAATTGAG TTCAGTATCA    420
   TCATTCGAGA GATTCGAAAT ATTCCCCAAA GAAAGCTCAT GGCCCAACCA CACCGTAACC    480
   AAAGGAGTAA CGAGATCATG CTCCCATAAT GGGAAAAGCA GTTTTTACAG AAATTTGCTA    540
   TGGCTGACGG AGAAGAATGG CTTGTACCCA AATCTGAGCA AGTCCTATGT AAACAACAAA    600
   GAGAAAGAAG TCCTTGTACT ATGGGGTGTT CATCACCCGT CTAACATAAG GGACCAAAGG    660
   GCCATCTATC ATACAGAAAA TGCTTATGTC TCTGTAGTGT CTTCACATTA TAGCAGAAGA    720
   TTCACCCCAG AAATAGCAAA AAGACCCAAA GTAAGAGATC AAGAAGGAAG AATTAACTAC    780
   TACTGGACTC TGCTGGAACC CGGGGACACA ATAATATTTG AGGCAAATGG AAATCTAATA    840
   GCGCCATGGT ATGCTTTCGC ACTGAGTAGA GGCTTTGGGT CAGGAATCAT CACCTCAAAC    900
   GCATCAATGG ATGAATGTGA CGCGAAGTGT CAAACACCCC AGGGAGCTAT AAACAGTAGT    960
   CTTCCTTTCC AGAATGTACA CCCAGTCACA ATAGGAGAGT GTCCAAAGTA TGTCAGGAGT   1020
   ACAAAATTAA GGATGGTTAC AGGACTAAGG AACATCCCAT CCATTCAATC CAGAGGTTTG   1080
   TTTGGAGCCA TTGCCGGTTT CATTGAAGGG GGGTGGACTG GAATGATAGA TGGATGGTAT   1140
   GGTTATCATC ATCAGAATGA ACAAGGATCT GGCTATGCTG CGGACCAAAA AAGCACACAA   1200
   AATGCCATTA ACGGGATTAC AAACAAGGTG AATTCTGTAA TCGAGAAAAT GAACACTCAA   1260
   TTCACAGCTG TGGGCAAAGA ATTCAACAAA TTAGAAAGAA GGATGGAAAA CTTAAATAAA   1320
   AAAGTTGATG ATGGATTTCT GGACATTTGG ACATATAATG CAGAATTGTT GGTTCTACTG   1380
   GAAAATGGAA GGACTTTGGA TTTTCATGAC TCAAATGTGA AGAATCTGTA TGAGAAAGTA   1440
   AAAAGCCAAT TGAAGAATAA TGCCAAAGAA ATAGGGAACG GGTGTTTTGA ATTCTATCAC   1500
   AAGTGTAACA ATGAATGCAT GGAAAGTGTG AAAAATGGAA CTTATGACTA TCCAAAATAT   1560
   TCCGAAGAAT CAAAGTTAAA CAGGGGAAAA ATTGATGGAG TGAAATTGGA ATCAATGGGA   1620
   GTCTATCAGA TTCTGGCGAT CTACTCAACT GTCGCCAGTT CACTGGTGCT TTTGGTCTCC   1680
   CTGGGGGCAA TCAGCTTCTG GATGTGTTCT AATGGGTCTT TGCAGTGCAG AATATGAATC   1740
   TGAGGTACCA GATCTTAATT AATTAA                                        1766
(2) INFORMATION FOR SEQ ID NO:9:
   (i) SEQUENCE CHARACTERISTICS:
      (A) LENGTH: 572 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Influenza virus
      (C) INDIVIDUAL ISOLATE: A/Texas/36/91 rHA
   (ix) FEATURE
      (A) NAME/KEY: AcNPV 61K protein signal sequence
      (B) LOCATION: 1 to 18
   (ix) FEATURE
      (A) NAME/KEY: mature rHA
      (B) LOCATION: 19 to 554
   (xi) SEQUENCE DESCRIPTION: SEQ ID NO:9:
   Met Pro Leu Tyr Lys Leu Leu Asn Val Leu Trp Leu Val Ala Val Ser
   1 5 10 15
   Asn Ala Ile Pro Gly Gly Thr Pro Gly Asp Thr Ile Cys Ile Gly Tyr
   20 25 30
   His Ala Asn Asn Ser Thr Asp Thr Val Asp Thr Val Leu Glu Lys Asn
   35 40 45
   Val Thr Val Thr His Ser Val Asn Leu Leu Glu Asp Ser His Asn Gly
   50 55 60
   Lys Leu Cys Arg Leu Lys Gly Ile Ala Pro Leu Gln Leu Gly Asn Cys
   65 70 75 80
   Ser Val Ala Gly Trp Ile Leu Gly Asn Pro Lys Cys Glu Ser Leu Phe
   85 90 95
   Ser Lys Glu Ser Trp Ser Tyr Ile Ala Glu Thr Pro Asn Pro Glu Asn
   100 105 110
   Gly Thr Cys Tyr Pro Gly Tyr Phe Ala Asp Tyr Glu Glu Leu Arg Glu
   115 120 125
   Gln Leu Ser Ser Val Ser Ser Phe Glu Arg Phe Glu Ile Phe Pro Lys
   130 135 140
   Glu Ser Ser Trp Pro Asn His Thr Val Thr Lys Gly Val Thr Arg Ser
   145 150 155 160
   Cys Ser His Asn Gly Lys Ser Ser Phe Tyr Arg Asn Leu Leu Trp Leu
   165 170 175
   Thr Glu Lys Asn Gly Leu Tyr Pro Asn Leu Ser Lys Ser Tyr Val Asn
   180 185 190
   Asn Lys Glu Lys Glu Val Leu Val Leu Trp Gly Val His His Pro Ser
   195 200 205
   Asn Ile Arg Asp Gln Arg Ala Ile Tyr His Thr Glu Asn Ala Tyr Val
   210 215 220
   Ser Val Val Ser Ser His Tyr Ser Arg Arg Phe Thr Pro Glu Ile Ala
   225 230 235 240
   Lys Arg Pro Lys Val Arg Asp Gln Glu Gly Arg Ile Asn Tyr Tyr Trp
   245 250 255
   Thr Leu Leu Glu Pro Gly Asp Thr Ile Ile Phe Glu Ala Asn Gly Asn
   260 265 270
   Leu Ile Ala Pro Trp Tyr Ala Phe Ala Leu Ser Arg Gly Phe Gly Ser
   275 280 285
   Gly Ile Ile Thr Ser Asn Ala Ser Met Asp Glu Cys Asp Ala Lys Cys
   290 295 300
   Gln Thr Pro Gln Gly Ala Ile Asn Ser Ser Leu Pro Phe Gln Asn Val
   305 310 315 320
   His Pro Val Thr Ile Gly Glu Cys Pro Lys Tyr Val Arg Ser Thr Lys
   325 330 335
   Leu Arg Met Val Thr Gly Leu Arg Asn Ile Pro Ser Ile Gln Ser Arg
   340 345 350
   Gly Leu Phe Gly Ala Ile Ala Gly Phe Ile Glu Gly Gly Trp Thr Gly
   355 360 365
   Met Ile Asp Gly Trp Tyr Gly Tyr His His Gln Asn Glu Gln Gly Ser
   370 375 380
   Gly Tyr Ala Ala Asp Gln Lys Ser Thr Gln Asn Ala Ile Asn Gly Ile
   385 390 395 400
   Thr Asn Lys Val Asn Ser Val Ile Glu Lys Met Asn Thr Gln Phe Thr
   405 410 415
   Ala Val Gly Lys Glu Phe Asn Lys Leu Glu Arg Arg Met Glu Asn Leu
   420 425 430
   Asn Lys Lys Val Asp Asp Gly Phe Leu Asp Ile Trp Thr Tyr Asn Ala
   435 440 445
   Glu Leu Leu Val Leu Leu Glu Asn Gly Arg Thr Leu Asp Phe His Asp
   450 455 460
   Ser Asn Val Lys Asn Leu Tyr Glu Lys Val Lys Ser Gln Leu Lys Asn
   465 470 475 480
   Asn Ala Lys Glu Ile Gly Asn Gly Cys Phe Glu Phe Tyr His Lys Cys
   485 490 495
   Asn Asn Glu Cys Met Glu Ser Val Lys Asn Gly Thr Tyr Asp Tyr Pro
   500 505 510
   Lys Tyr Ser Glu Glu Ser Lys Leu Asn Arg Gly Lys Ile Asp Gly Val
   515 520 525
   Lys Leu Glu Ser Met Gly Val Tyr Gln Ile Leu Ala Ile Tyr Ser Thr
   530 535 540
   Val Ala Ser Ser Leu Val Leu Leu Val Ser Leu Gly Ala Ile Ser Phe
   545 550 555 560
   Trp Met Cys Ser Asn Gly Ser Leu Gln Cys Arg Ile
   565 570
(2) INFORMATION FOR SEQ ID NO:10:
   (i) SEQUENCE CHARACTERISTICS:
      (A) LENGTH: 1799 base pairs
      (B) TYPE: nucleic acid
      (C) STRANDEDNESS: single
      (D) TOPOLOGY: linear
   (ii) MOLECULE TYPE: RNA (genomic)
   (iii) HYPOTHETICAL: NO
   (iv) ANTI-SENSE: NO
   (vi) ORIGINAL SOURCE:
      (A) ORGANISM: Influenza virus
      (c) INDIVIDUAL ISOLATE: B/Panam/45/90 rHA
   (ix) FEATURE
      (A) NAME/KEY: polyhedrin mRNA leader (partial)
      (B) LOCATION: 1 to 18
   (ix) FEATURE
      (A) NAME/KEY: coding region for HA signal peptide sequence
      (B) LOCATION: 19 to 69
   (ix) FEATURE
      (A) NAME/KEY: SmaI restriction site
      (B) LOCATION; 22 to 27
   (ix) FEATURE
      (A) NAME/KEY: coding region for mature rHA
      (B) LOCATION: 70 to 1773
   (ix) FEATURE
      (A) NAME/KEY: KpnI restriction site
      (B) LOCATION: 1777 to 1782
   (ix) FEATURE
      (A) NAME/KEY: BglII restriction site
      (B) LOCATION: 1783 to 1788
   (ix) FEATURE
      (A) NAME/KEY: unversal translation termination signal
      (B) LOCATION: 1789 to 1799
   (xi) SEQUENCE DESCRIPTION: SEQ ID NO:10:
   TAAAAAAACC TATAAATAAT GCCCGGGAAG GCAATAATTG TACTACTCAT GGTAGTAACA     60
   TCCAACGCAG ATCGAATCTG CACTGGGATA ACATCTTCAA ACTCACCTCA TGTGGTCAAA    120
   ACAGCTACTC AAGGGGAAGT CAATGTGACT GGTGTGATAC CACTGACAAC AACACCAACA    180
   AAATCTCATT TTGCAAATCT AAAAGGAACA AAGACCAGAG GGAAACTATG CCCAAACTGT    240
   CTCAACTGCA CAGATCTGGA TGTGGCCTTG GGCAGACCAA TGTGTGTGGG GACCACACCT    300
   TCGGCAAAAG CTTCAATACT CCACGAAGTC AGACCTGTTA CATCCGGGTG CTTTCCTATA    360
   ATGCACGACA GAACAAAAAT CAGACAGCTA CCCAATCTTC TCAGAGGATA TGAAAATATC    420
   AGATTATCAA CCCAAAACGT TATCAACGCA GAAAGAGCAC CAGGAGGACC CTACAGACTT    480
   GGAACCTCAG GATCTTGCCC TAACGTTACC AGTAGAGACG GATTCTTCGC AACAATGGCT    540
   TGGGCTGTCC CAAGGGACAA CAAAACAGCA ACGAATCCAC TAACAGTAGA AGTACCATAC    600
   ATTTGTACCA AAGGAGAAGA CCAAATTACT GTTTGGGGGT TCCATTCTGA TAACAAAATC    660
   CAAATGAAAA ACCTCTATGG AGACTCAAAT CCTCAAAAGT TCACCTCATC TGCCAATGGA    720
   GTAACCACAC ATTATGTTTC TCAGATTGGT GGCTTCCCAA ATCAAACAGA AGACGGAGGG    780
   CTACCACAAA GCGGCAGAAT TGTTGTTGAT TACATGGTGC AAAAACCTGG GAAAACAGGA    840
   ACAATTGTCT ATCAAAGAGG TGTTTTGTTG CCTCAAAAGG TGTGGTGCGC AAGTGGCAGG    900
   AGCAAGGTAA TAAAAGGGTC CTTGCCTTTA ATTGGTGAAG CAGATTGCCT TCACGAAAAA    960
   TACGGTGGAT TAAACAAAAG CAAGCCTTAC TACACAGGAG AACATGCAAA AGCCATAGGA   1020
   AATTGCCCAA TATGGGTGAA AACACCTTTG AAGCTTGCCA ATGGAACCAA ATATAGACCT   1080
   CCTGCAAAAC TATTAAAGGA AAGGGGTTTC TTCGGAGCTA TTGCTGGTTT CTTAGAAGGA   1140
   GGATGGGAAG GAATGATTGC AGGTTGGCAC GGATACACAT CTCATGGAGC ACATGGAGTG   1200
   GCAGTGGCAG CAGACCTTAA GAGTACGCAA GAAGCCATAA ACAAGATAAC AAAAAATCTC   1260
   AATTCTTTGA GTGAGCTAGA AGTAAAGAAT CTTCAAAGAC TAAGTGGTGC CATGGATGAA   1320
   CTCCACAACG AAATACTCGA GCTGGATGAG AAAGTGGATG ATCTCAGAGC TGACACAATA   1380
   AGCTCGCAAA TAGAGCTTGC AGTCTTGCTT TCCAACGAAG GAATAATAAA CAGTGAAGAT   1440
   GAGCATCTAT TGGCACTTGA GAGAAAACTA AAGAAAATGC TGGGTCCCTC TGCTGTAGAC   1500
   ATAGGGAATG GATGCTTCGA AACCAAACAC AAGTGCAACC AGACCTGCTT AGACAGGATA   1560
   GCTGCTGGCA CCTTTAATGC AGGAGAATTT TCTCTTCCCA CTTTTGATTC ACTGAATATT   1620
   ACTGCTGCAT CTTTAAATGA TGATGGATTG GATAATCATA CTATACTGCT CTACTACTCA   1680
   ACTGCTGCTT CTAGTTTGGC TGTAACATTG ATGATAGCTA TTTTTATTGT TTATATGGTC   1740
   TCCAGAGACA ATGTTTCTTG TTCCATCTGT CTGTGAGGTA CCAGATCTTA ATTAATTAA    1799
(2) INFORMATION FOR SEQ ID NO:11:
   (i) SEQUENCE CHARACTERISTICS:
      (A) LENGTH: 585 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Influenza virus
      (c) INDIVIDUAL ISOLATE: E/Panama/45/90 rHA
   (ix) FEATURE
      (A) NAME/KEY: HA signal peptide
      (B) LOCATION: 1 to 17
   (ix) FEATURE
      (A) NAME/KEY: mature rHA
      (B) LOCATION: 18 to 568
   (xi) SEQUENCE DESCRIPTION: SEQ ID NO:11:
   Met Pro Gly Lys Ala Ile Ile Val Leu Leu Met Val Val Thr Ser Asn
   1               5                   10                  15
   Ala Asp Arg Ile Cys Thr Gly Ile Thr Ser Ser Asn Ser Pro His Val
               20                  25                  30
   Val Lys Thr Ala Thr Gln Gly Glu Val Asn Val Thr Gly Val Ile Pro
           35                  40                  45
   Leu Thr Thr Thr Pro Thr Lys Ser His Phe Ala Asn Leu Lys Gly Thr
       50                  55                  60
   Lys Thr Arg Gly Lys Leu Cys Pro Asn Cys Leu Asn Cys Thr Asp Leu
   65                  70                  75                  80
   Asp Val Ala Leu Gly Arg Pro Met Cys Val Gly Thr Thr Pro Ser Ala
                   85                  90                  95
   Lys Ala Ser Ile Leu His Glu Val Arg Pro Val Thr Ser Gly Cys Phe
               100                 105                 110
   Pro Ile Met His Asp Arg Thr Lys Ile Arg Gln Leu Pro Asn Leu Leu
           115                 120                 125
   Arg Gly Tyr Glu Asn Ile Arg Leu Ser Thr Gln Asn Val Ile Asn Ala
       130                 135                 140
   Glu Arg Ala Pro Gly Gly Pro Tyr Arg Leu Gly Thr Ser Gly Ser Cys
   145                 150                 155                 160
   Pro Asn Val Thr Ser Arg Asp Gly Phe Phe Ala Thr Met Ala Trp Ala
                   165                 170                 175
   Val Pro Arg Asp Asn Lys Thr Ala Thr Asn Pro Leu Thr Val Glu Val
               180                 185                 190
   Pro Tyr Ile Cys Thr Lys Gly Glu Asp Gln Ile Thr Val Trp Gly Phe
           195                 200                 205
   His Ser Asp Asn Lys Ile Gln Met Lys Asn Leu Tyr Gly Asp Ser Asn
       210                 215                 220
   Pro Gln Lys Phe Thr Ser Ser Ala Asn Gly Val Thr Thr His Tyr Val
   225                 230                 235                 240
   Ser Gln Ile Gly Gly Phe Pro Asn Gln Thr Glu Asp Gly Gly Leu Pro
                   245                 250                 255
   Gln Ser Gly Arg Ile Val Val Asp Tyr Met Val Gln Lys Pro Gly Lys
               260                 265                 270
   Thr Gly Thr Ile Val Tyr Gln Arg Gly Val Leu Leu Pro Gln Lys Val
           275                 280                 285
   Trp Cys Ala Ser Gly Arg Ser Lys Val Ile Lys Gly Ser Leu Pro Leu
       290                 295                 300
   Ile Gly Glu Ala Asp Cys Leu His Glu Lys Tyr Gly Gly Leu Asn Lys
   305                 310                 315                 320
   Ser Lys Pro Tyr Tyr Thr Gly Glu His Ala Lys Ala Ile Gly Asn Cys
                   325                 330                 335
   Pro Ile Trp Val Lys Thr Pro Leu Lys Leu Ala Asn Gly Thr Lys Tyr
               340                 345                 350
   Arg Pro Pro Ala Lys Leu Leu Lys Glu Arg Gly Phe Phe Gly Ala Ile
           355                 360                 365
   Ala Gly Phe Leu Glu Gly Gly Trp Glu Gly Met Ile Ala Gly Trp His
       370                 375                 380
   Gly Tyr Thr Ser His Gly Ala His Gly Val Ala Val Ala Ala Asp Leu
   385                 390                 395                 400
   Lys Ser Thr Gln Glu Ala Ile Asn Lys Ile Thr Lys Asn Leu Asn Ser
                   405                 410                 415
   Leu Ser Glu Leu Glu Val Lys Asn Leu Gln Arg Leu Ser Gly Ala Met
               420                 425                 430
   Asp Glu Leu His Asn Glu Ile Leu Glu Leu Asp Glu Lys Val Asp Asp
           435                 440                 445
   Leu Arg Ala Asp Thr Ile Ser Ser Gln Ile Glu Leu Ala Val Leu Leu
       450                 455                 460
   Ser Asn Glu Gly Ile Ile Asn Ser Glu Asp Glu His Leu Leu Ala Leu
   465                 470                 475                 480
   Glu Arg Lys Leu Lys Lys Met Leu Gly Pro Ser Ala Val Asp Ile Gly
                   485                 490                 495
   Asn Gly Cys Phe Glu Thr Lys His Lys Cys Asn Gln Thr Cys Leu Asp
               500                 505                 510
   Arg Ile Ala Ala Gly Thr Phe Asn Ala Gly Glu Phe Ser Leu Pro Thr
           515                 520                 525
   Phe Asp Ser Leu Asn Ile Thr Ala Ala Ser Leu Asn Asp Asp Gly Leu
       530                 535                 540
   Asp Asn His Thr Ile Leu Leu Tyr Tyr Ser Thr Ala Ala Ser Ser Leu
   545                 550                 555                 560
   Ala Val Thr Leu Met Ile Ala Ile Phe Ile Val Tyr Met Val Ser Arg
                   565                 570                 575
   Asp Asn Val Ser Cys Ser Ile Cys Leu
               580                 585
(2) INFORMATION FOR SEQ ID NO:12:
   (i) SEQUENCE CHARACTERISTICS:
      (A) LENGTH: 1811 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Influenza virus
      (C) INDIVIDUAL ISOLATE: B/Netherlands/13/94 rHA
   (ix) FEATURE
      (A) NAME/KEY: polyhedrin mRNA leader (partial)
      (B) LOCATION: 1 to 18
   (ix) FEATURE
      (A) NAME/KEY: coding region for AcNPV 61K protein signal sequence
      (B) LOCATION: 19 to 72
   (ix) FEATURE
      (A) NAME/KEY: SmaI restriction site
      (B) LOCATION: 76 to 81
   (ix) FEATURE
      (A) NAME/KEY: coding region for mature rHA
      (B) LOCATION: 73 to 1785
   (ix) FEATURE
      (A) NAME/KEY: KpnI restriction site
      (B) LOCATION: 1789 to 1794
   (ix) FEATURE
      (A) NAME/KEY: BglII restriction site
      (B) LOCATION: 1795 to 1800
   (ix) FEATURE
      (A) NAME/KEY: unversal translation termination signal
      (B) LOCATION: 1801 to 1811
   (xi) SEQUENCE DESCRIPTION: SEQ ID NO:12:
   TAAAAAAACC TATAAATAAT GCCCTTGTAC AAATTGTTAA ACGTTTTGTG GTTGGTCGCC      60
   GTTTCTAACG CGATTCCCGG GGATCGAATC TGCACTGGGA TAACATCTTC AAAATCACCT     120
   CATGTAGTCA AAACAGCTAC TCAAGGGGAG GTCAATGTGA CTGGTGTGAT ACCACTGACG     180
   ACAACACCAA CAAAATCTCA TTTTGCAAAT CTCAAAGGAA CAAAGACCAG AGGGAAACTA     240
   TGCCCAAACT GTCTCAACTG CACAGATCTG GATGTGGCCT TGGGCAGACC AATGTGTGTG     300
   GGGATCACAC CTTCGGCAAA AGCTTCAATA CTCCACGAAG TCAGACCTGT TACATCCGGG     360
   TGCTTTCCTA TAATGCATGA CAGAACAAAA ATCAGACAGC TACCCAATCT TCTCAGAGGA     420
   TATGAAAACA TCAGACTATC AACCCAAAAC GTTATCAACG CAGAAAAGGC ACCAGGAGGA     480
   CCCTACAGAC TTGGAACCTC AGGATCTTGC CCTAACGTTA CCAGTAGAAC CGGATTCTTC     540
   GCAACAATGG CTTGGGCTGT CCCAAGGGAC AACAAAACAG CAACGAATCC ACTAACAGTA     600
   GAAGTACCAT ACATTTGTAC GAAAGGAGAA GACCAAATTA CTGTTTGGGG GTTCCATTCT     660
   GATAACAAAA CCCAAATGAA AAACCTCTAT GGAGACTCAA ATCCTCAAAA GTTCACCTCA     720
   TCTGCCAATG GAGTAACCAC ACATTATGTT TCTCAGATTG GTGGCTTCCC AGATCAAACA     780
   GAAGACGGAG GACTACCACA AAGCGGCAGA ATTGTTGTTG ATTACATGGT GCAAAAACCT     840
   GGGAAAACAG GAACAATTGT CTATCAAAGA GGTATTTTGT TGCCTCAAAA GGTGTGGTGC    900
   GCAAGTGGCA GGAGCAAGGT AATAAAAGGG TCCTTGCCTT TAATTGGTGA AGCAGATTGC     960
   CTTCACGAAA AATACGGTGG ATTAAACAAA AGCAAGCCTT ACTACACAGG AGAACATGCA    1020
   AAAGCCATAG GAAATTGCCC AATATGGGTG AAAACACCTT TGAAGCTTGC CAATGGAACC    1080
   AGATATAGAC CTCCTGCAAA ACTATTAAAG GAAAGGGGTT TCTTCGGAGC TATTGCTGGT    1140
   TTCTTAGAAG GAGGATGGGA AGGAATGATT GCAGGTTGGC ACGGATACAC ATCTCACGGG    1200
   GCACATGGAG TGGCAGTGGC AGCAGACCTT AAGAGTACGC AAGAAGCCAT AAACAAGATA    1260
   ACAAAAAATC TCAATTCTTT GAGTGAGCTA GAAGTAAAGA ACCTTCAAAG ACTAAGTGGT    1320
   GCCATGGATG AACTCCACAA CGAAATACTC GAGCTGGATG AGAAAGTGGA TGATCTCAGA    1380
   GCTGACACAA TAAGCTCGCA AATAGAGCTT GCAGTCTTAC TTTCCAACGA AGGAATAATA    1440
   AACAGTGAAG ATGAGCATCT ATTGGCACTT GAGAGAAAAC TAAAGAAAAT GCTGGGTCCC    1500
   TCTGCTGTAG ACATAGGGAA TGGATGCTTC GAAACAAAAC ACAAGTGCAA CCAGACCTGC    1560
   TTAGACAGGA TAGCTGCTGG CACCTTTAAT GCAGGAGAAT TTTCTCTTCC CACTTTTGAT    1620
   TCACTGAATA TTACTGCTGC ATCTTTAAAT GATGATGGAT TGGATAATCA TACTATACTG    1680
   CTCTACTACT CAACTGCTGC TTCTAGTTTG GCTGTAACAT TGATGATAGC TATTTTTATT    1740
   GTTTATATGG TCTCCAGAGA CAATGTTTCT TGTTCCATCT GTCTGTGAGG TACCAGATCT    1800
   TAATTAATTA A                                                         1811
(2) INFORMATION FOR SEQ ID NO:13:
   (i) SEQUENCE CHARACTERISTICS:
      (A) LENGTH: 589 amino acide
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Influenza virus
      (C) INDIVIDUAL ISOLATE: B/Netherlands/13/94 rHA
   (ix) FEATURE
      (A) NAME/KEY: AcNPV 61K protein signal sequence
      (B) LOCATION: 1 to 18
   (ix) FEATURE
      (A) NAME/KEY: mature rHA
      (B) LOCATION: 19 to 571
   (xi) SEQUENCE DESCRIPTION: SEQ ID NO:13:
   Met Pro Leu Tyr Lys Leu Leu Asn Val Leu Trp Leu Val Ala Val Ser
   1               5                   10                  15
   Asn Ala Ile Pro Gly Asp Arg Ile Cys Thr Gly Ile Thr Ser Ser Lys
               20                  25                  30
   Ser Pro His Val Val Lys Thr Ala Thr Gln Gly Glu Val Asn Val Thr
           35                  40                  45
   Gly Val Ile Pro Leu Thr Thr Thr Pro Thr Lys Ser His Phe Ala Asn
       50                  55                  60
   Leu Lys Gly Thr Lys Thr Arg Gly Lys Leu Cys Pro Asn Cys Leu Asn
   65                  70                  75                  80
   Cys Thr Asp Leu Asp Val Ala Leu Gly Arg Pro Met Cys Val Gly Ile
   85 90 95
   Thr Pro Ser Ala Lys Ala Ser Ile Leu His Glu Val Arg Pro Val Thr
   100 105 110
   Ser Gly Cys Phe Pro Ile Met His Asp Arg Thr Lys Ile Arg Gln Leu
   115 120 125
   Pro Asn Leu Leu Arg Gly Tyr Glu Asn Ile Arg Leu Ser Thr Gln Asn
   130 135 140
   Val Ile Asn Ala Glu Lys Ala Pro Gly Gly Pro Tyr Arg Leu Gly Thr
   145 150 155 160
   Ser Gly Ser Cys Pro Asn Val Thr Ser Arg Thr Gly Phe Phe Ala Thr
   165 170 175
   Met Ala Trp Ala Val Pro Arg Asp Asn Lys Thr Ala Thr Asn Pro Leu
   180 185 190
   Thr Val Glu Val Pro Tyr Ile Cys Thr Lys Gly Glu Asp Gln Ile Thr
   195 200 205
   Val Trp Gly Phe His Ser Asp Asn Lys Thr Gln Met Lys Asn Leu Tyr
   210 215 220
   Gly Asp Ser Asn Pro Gln Lys Phe Thr Ser Ser Ala Asn Gly Val Thr
   225 230 235 240
   Thr His Tyr Val Ser Gln Ile Gly Gly Phe Pro Asp Gln Thr Glu Asp
   245 250 255
   Gly Gly Leu Pro Gln Ser Gly Arg Ile Val Val Asp Tyr Met Val Gln
   260 265 270
   Lys Pro Gly Lys Thr Gly Thr Ile Val Tyr Gln Arg Gly Ile Leu Leu
   275 280 285
   Pro Gln Lys Val Trp Cys Ala Ser Gly Arg Ser Lys Val Ile Lys Gly
   290 295 300
   Ser Leu Pro Leu Ile Gly Glu Ala Asp Cys Leu His Glu Lys Tyr Gly
   305 310 315 320
   Gly Leu Asn Lys Ser Lys Pro Tyr Tyr Thr Gly Glu His Ala Lys Ala
   325 330 335
   Ile Gly Asn Cys Pro Ile Trp Val Lys Thr Pro Leu Lys Leu Ala Asn
   340 345 350
   Gly Thr Arg Tyr Arg Pro Pro Ala Lys Leu Leu Lys Glu Arg Gly Phe
   355 360 365
   Phe Gly Ala Ile Ala Gly Phe Leu Glu Gly Gly Trp Glu Gly Met Ile
   370 375 380
   Ala Gly Trp His Gly Tyr Thr Ser His Gly Ala His Gly Val Ala Val
   385 390 395 400
   Ala Ala Asp Leu Lys Ser Thr Gln Glu Ala Ile Asn Lys Ile Thr Lys
   405 410 415
   Asn Leu Asn Ser Leu Ser Glu Leu Glu Val Lys Asn Leu Gln Arg Leu
   420 425 430
   Ser Gly Ala Met Asp Glu Leu His Asn Glu Ile Leu Glu Leu Asp Glu
   435 440 445
   Lys Val Asp Asp Leu Arg Ala Asp Thr Ile Ser Ser Gln Ile Glu Leu
   450 455 460
   Ala Val Leu Leu Ser Asn Glu Gly Ile Ile Asn Ser Glu Asp Glu His
   465 470 475 480
   Leu Leu Ala Leu Glu Arg Lys Leu Lys Lys Met Leu Gly Pro Ser Ala
   485 490 495
   Val Asp Ile Gly Asn Gly Cys Phe Glu Thr Lys His Lys Cys Asn Gln
   500 505 510
   Thr Cys Leu Asp Arg Ile Ala Ala Gly Thr Phe Asn Ala Gly Glu Phe
   515 520 525
   Ser Leu Pro Thr Phe Asp Ser Leu Asn Ile Thr Ala Ala Ser Leu Asn
   530 535 540
   Asp Asp Gly Leu Asp Asn His Thr Ile Leu Leu Tyr Tyr Ser Thr Ala
   545 550 555 560
   Ala Ser Ser Leu Ala Val Thr Leu Met Ile Ala Ile Phe Ile Val Tyr
   565 570 575
   Met Val Ser Arg Asp Asn Val Ser Cys Ser Ile Cys Leu
   580 585
(2) INFORMATION FOR SEQ ID NO:14:
   (i) SEQUENCE CHARACTERISTICS:
      (A) LENGTH: 1757 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Influenza virus
      (C) INDIVIDUAL ISOLATE: A/Shandong/9/93 rHA
   (ix) FEATURE
      (A) NAME/KEY: polyhedrin mRNA leader (partial)
      (B) LOCATION: 1 to 18
   (ix) FEATURE
      (A) NAME/KEY : coding region for AcNPV 61K protein signal sequence
      (B) LOCATION: 19 to 72
   (ix) FEATURE
      (A) NAME/KEY: smaI restriction site
      (B) LOCATION. 76 to 81
   (ix) FEATURE
      (A) NAME/KEY: coding region for mature rHA
      (B) LOCATION: 73 to 1728
   (ix) FEATURE
      (A) NAME/KEY: KpnI restriction site
      (B) LOCATION: 1735 to 1740
   (ix) FEATURE
      (A) NAME/KEY: EglII restriction site
      (B) LOCATION: 1741 to 1746
   (ix) FEATURE
      (A) NAME/KEY: unversal translation termination signal
      (B) LOCATION: 1747 to 1757
   (xi) SEQUENCE DESCRIPTION; SEQ ID NO:14:
   TAAAAAAACC TATAAATAAT GCCCTTGTAC AAATTGTTAA ACGTTTTGTG GTTGGTCGCC     60
   GTTTCTAACG CGATTCCCGG GCAAGACCTT CCAGGAAATG ACAACAGCAC AGCAACGCTG    120
   TGCCTGGGAC ATCATGCAGT GCCAAACGGA ACGCTAGTGA AAACAATCAC GAATGATCAA    180
   ATTGAAGTGA CTAATGCTAC TGAGTTGGTT CAGAGTTCCT CAACAGGTAG AATATGCGGC    240
   AGTCCTCACC GAATCCTTGA TGGAAAAAAC TGCACACTGA TAGATGCTCT ATTGGGAGAC    300
   CCTCATTGTG ATGGCTTCCA AAATAAGGAA TGGGACCTTT TTGTTGAACG CAGCAAAGCT    360
   TACAGCAACT GTTACCCTTA TGATGTGCCG GATTATGCCT CCCTTAGGTC ACTAGTTGCC    420
   TCATCAGGCA CCCTGGAGTT TATCAATGAA GACTTCAATT GGACTGGAGT CGCTCAGGAT    480
   GGGGGAAGCT ATGCTTGCAA AAGAGGATCT GTTAACAGTT TCTTTAGTAG ATTGAATTGG    540
   TTGCACAAAT TAGAATACAA ATATCCAGCG CTGAACGTGA CTATGCCAAA CAATGGCAAA    600
   TTTGACAAAT TGTACATTTG GGGGGTTCAC CACCCGAGCA CGGACAGTGA CCAAACCAGC    660
   CTATATGTTC GAGCATCAGG GAGAGTCACA GTCTCTACCA AAAGAAGCCA ACAAACTGTA    720
   ACCCCGAATA TCGGGTCTAG ACCCTGGGTA AGGGGTCAGT CCAGTAGAAT AAGCATCTAT    780
   TGGACAATAG TAAAACCGGG AGACATACTT TTGATTGATA GCACAGGGAA TCTAATTGCT    840
   CCTCGGGGTT ACTTCAAAAT ACGAAATGGG AAAAGCTCAA TAATGAGGTC AGATGCACCC    900
   ATTGGCAACT GCAGTTCTGA ATGCATCACT CCAAATGGAA GCATTCCCAA TGACAAACCT    960
   TTTCAAAATG TAAACAGAAT CACATATGGG GCCTGCCCCA GATATGTTAA GCAAAACACT   1020
   CTGAAATTGG CAACAGGGAT GCGGAATGTA CCAGAGAAAC AAACTAGAGG CATATTCGGC   1080
   GCAATCGCAG GTTTCATAGA AAATGGTTGG GAGGGAATGG TAGACGGTTG GTACGGTTTC   1140
   AGGCATCAAA ATTCTGAGGG CACAGGACAA GCAGCAGATC TTAAAAGCAC TCAAGCAGCA   1200
   ATCGACCAAA TCAACGGGAA ACTGAATAGG TTAATCGAGA AAACGAACGA GAAATTCCAT   1260
   CAAATCGAAA AAGAATTCTC AGAAGTAGAA GGGAGAATTC AGGACCTCGA GAAATATGTT   1320
   GAAGACACTA AAATAGATCT CTGGTCTTAC AACGCGGAGC TTCTTGTTGC CCTGGAGAAC   1380
   CAACATACAA TTGATCTAAC TGACTCAGAA ATGAACAAAC TGTTTGAAAA AACAAGGAAG   1440
   CAACTGAGGG AAAATGCTGA GGACATGGGC AATGGTTGCT TCAAAATATA CCACAAATGT   1500
   GACAATGCCT GCATAGGGTC AATCAGAAAT GGAACTTATG ACCATGATGT ATACAGAGAC   1560
   GAAGCATTAA ACAACCGGTT CCAGATCAAA GGTGTTGAGC TGAAGTCAGG ATACAAAGAT   1620
   TGGATCCTAT GGATTTCCTT TGCCATATCA TGCTTTTTGC TTTGTGTTGT TTTGCTGGGG   1680
   TTCATCATGT GGGCCTGCCA AAAAGGCAAC ATTAGGTGCA ACATTTGCAT TTGAGGTACC   1740
   AGATCTTAAT TAATTAA                                                  1757
(2) INFORMATION FOR SEQ ID NO:15:
   (i) SEQUENCE CHARACTERISTICS:
      (A) LENGTH: 571 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Influenza virus
      (C) INDIVIDUAL ISOLATE: A/Shandong/9/93 rHA
   (ix) FEATURE
      (A) NAME/KEY: AcNPV 61K protein signal sequence
      (B) LOCATION: 1 to 18
   (ix) FEATURE
      (A) NAME/KEY: mature rHA
      (B) LOCATION: 19 to 553
   (xi) SEQUENCE DESCRIPTION: SEQ ID NO:15:
   Met Pro Leu Tyr Lys Leu Leu Asn Val Leu Trp Leu Val Ala Val Ser
   1               5                   10                  15
   Asn Ala Ile Pro Gly Gln Asp Leu Pro Gly Asn Asp Asn Ser Thr Ala
               20                  25                  30
   Thr Leu Cys Leu Gly His His Ala Val Pro Asn Gly Thr Leu Val Lys
           35                  40                  45
   Thr Ile Thr Asn Asp Gln Ile Glu Val Thr Asn Ala Thr Glu Leu Val
       50                  55                  60
   Gln Ser Ser Ser Thr Gly Arg Ile Cys Gly Ser Pro His Arg Ile Leu
   65                  70                  75                  80
   Asp Gly Lys Asn Cys Thr Leu Ile Asp Ala Leu Leu Gly Asp Pro His
                   85                  90                  95
   Cys Asp Gly Phe Gln Asn Lys Glu Trp Asp Leu Phe Val Glu Arg Ser
               100                 105                 110
   Lys Ala Tyr Ser Asn Cys Tyr Pro Tyr Asp Val Pro Asp Tyr Ala Ser
           115                 120                 125
   Leu Arg Ser Leu Val Ala Ser Ser Gly Thr Leu Glu Phe Ile Asn Glu
       130                 135                 140
   Asp Phe Asn Trp Thr Gly Val Ala Gln Asp Gly Gly Ser Tyr Ala Cys
   145 150 155 160
   Lys Arg Gly Ser Val Asn Ser Phe Phe Ser Arg Leu Asn Trp Leu His
   165 170 175
   Lys Leu Glu Tyr Lys Tyr Pro Ala Leu Asn Val Thr Met Pro Asn Asn
   180 185 190
   Gly Lys Phe Asp Lys Leu Tyr Ile Trp Gly Val His His Pro Ser Thr
   195 200 205
   Asp Ser Asp Gln Thr Ser Leu Tyr Val Arg Ala Ser Gly Arg Val Thr
   210 215 220
   Val Ser Thr Lys Arg Ser Gln Gln Thr Val Thr Pro Asn Ile Gly Ser
   225 230 235 240
   Arg Pro Trp Val Arg Gly Gln Ser Ser Arg Ile Ser Ile Tyr Trp Thr
   245 250 255
   Ile Val Lys Pro Gly Asp Ile Leu Leu Ile Asp Ser Thr Gly Asn Leu
   260 265 270
   Ile Ala Pro Arg Gly Tyr Phe Lys Ile Arg Asn Gly Lys Ser Ser Ile
   275 280 285
   Met Arg Ser Asp Ala Pro Ile Gly Asn Cys Ser Ser Glu Cys Ile Thr
   290 295 300
   Pro Asn Gly Ser Ile Pro Asn Asp Lys Pro Phe Gln Asn Val Asn Arg
   305 310 315 320
   Ile Thr Tyr Gly Ala Cys Pro Arg Tyr Val Lys Gln Asn Thr Leu Lys
   325 330 335
   Leu Ala Thr Gly Met Arg Asn Val Pro Glu Lys Gln Thr Arg Gly Ile
   340 345 350
   Phe Gly Ala Ile Ala Gly Phe Ile Glu Asn Gly Trp Glu Gly Met Val
   355 360 365
   Asp Gly Trp Tyr Gly Phe Arg His Gln Asn Ser Glu Gly Thr Gly Gln
   370 375 380
   Ala Ala Asp Leu Lys Ser Thr Gln Ala Ala Ile Asp Gln Ile Asn Gly
   385 390 395 400
   Lys Leu Asn Arg Leu Ile Glu Lys Thr Asn Glu Lys Phe His Gln Ile
   405 410 415
   Glu Lys Glu Phe Ser Glu Val Glu Gly Arg Ile Gln Asp Leu Glu Lys
   420 425 430
   Tyr Val Glu Asp Thr Lys Ile Asp Leu Trp Ser Tyr Asn Ala Glu Leu
   435 440 445
   Leu Val Ala Leu Glu Asn Gln His Thr Ile Asp Leu Thr Asp Ser Glu
   450 455 460
   Met Asn Lys Leu Phe Glu Lys Thr Arg Lys Gln Leu Arg Glu Asn Ala
   465 470 475 480
   Glu Asp Met Gly Asn Gly Cys Phe Lys Ile Tyr His Lys Cys Asp Asn
   485 490 495
   Ala Cys Ile Gly Ser Ile Arg Asn Gly Thr Tyr Asp His Asp Val Tyr
   500 505 510
   Arg Asp Glu Ala Leu Asn Asn Arg Phe Gln Ile Lys Gly Val Glu Leu
   515 520 525
   Lys Ser Gly Tyr Lys Asp Trp Ile Leu Trp Ile Ser Phe Ala Ile Ser
   530 535 540
   Cys Phe Leu Leu Cys Val Val Leu Leu Gly Phe Ile Met Trp Ala Cys
   545 550 555 560
   Gln Lys Gly Asn Ile Arg Cys Asn Ile Cys Ile
   565 570
(2) INFORMATION FOR SEQ ID NO:16:
   (i) SEQUENCE CHARACTERISTICS:
      (A) LENGTH: 1814 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Influenza virus
      (C) INDIVIDUAL ISOLATE: B/Shanhai/4/94 rHA
   (ix) FEATURE
      (A) NAME/KEY: polyhedrin mRNA leader (partial)
      (B) LOCATION: 1 to 18
   (ix) FEATURE
      (A) NAME/KEY: coding region for AcNPV 61K protein signal sequence
      (B) LOCATION: 19 to 72
   (ix) FEATURE
      (A) NAME/KEY: SmaI restriction site
      (B) LOCATION: 76 to 81
   (ix) FEATURE
      (A) NAME/KEY: RpnI restriction site
      (B) LOCATION: 82 to 87
   (ix) FEATURE
      (A) NAME/KEY: coding region for mature rHA
      (B) LOCATION: 73 to 1794
   (ix) FEATURE
      (A) NAME/KEY: unversal translation termination signal
      (B) LOCATION: 1804 to 1814
   (xi) SEQUENCE DESCRIPTION: SEQ ID NO:16:
   TAAAAAAACC TATAAATAAT GCCCTTGTAC AAATTGTTAA ACGTTTTGTG GTTGGTCGCC     60
   GTTTCTAACG CGATTCCCGG GGGTACCGAT CGAATCTGCA CTGGGATAAC ATCTTCAAAC    120
   TCACCTCATG TGGTCAAAAC AGCTACTCAA GGGGAGGTCA ATGTGACTGG TGTGATACCA    180
   GTGACAACAA CACCAACAAA ATCTCATTTT GCAAATCTCA AAGGAACAAA GACCAGAGGG    240
   AAACTATGCC CAAACTGTCT CAACTGCACA GATCTGGATG TGGCCTTGGG CAGACCAATG    300
   TGTGTGGGGA CCACACCTTC GGCAAAAGCT TCAATACTCC ACGAAGTCAG ACCTGTTACA    360
   TCCGGGTGCT TTCCTATAAT GCACGACAGA ACAAAAATCA GACAGCTACC CAATCTTCTC    420
   AGAGGATATG AAAATATCAG ATTATCAACC CAAAACGTTA TCAACGCAGA AAAGGCACCA    480
   GGAGGACCCT ACAGACTTGG AACCTCAGGA TCTTGCCCTA ACGCTACCAG TAGAAGCGGA    540
   TTTTTCGCAA CAATGGCTTG GGCTGTCCCA AGGGACAACA ACAAAACAGC AACGAATCCA    600
   CTAACAGTAG AAGTACCATA CATTTGCACA AAAGGAGAAG ACCAAATTAC TGTTTGGGGG    660
   TTCCATTCTG ATAACAAACC CCAAATGAAA AACCTCTATG GAGACTCAAA TCCTCAAAAG    720
   TTCACCTCAT CTGCTAATGG AGTAACCACA CATTATGTTT CTCAGATTGG CGGCTTCCCA    780
   GATCAAACAG AAGACGGAGG GCTACCACAA AGCGGCAGAA TTGTTGTTGA TTACATGGTG    840
   CAAAAACCTG GGAAGACAGG AACAATTGTC TATCAGAGAG GTGTTTTGTT GCCTCAAAAG    900
   GTGTGGTGCG CTAGTGGCAG GAGCAAAGTA ATAAAAGGGT CCTTGCCTTT AATTGGTGAA    960
   GCAGATTGCC TTCACGAAAA ATACGGTGGA TTAAACAAAA GCAAGCCTTA CTACACAGGA   1020
   GAACATGCAA AAGCCATAGG AAATTGCCCA ATATGGGTGA AAACACCTTT GAAGCTTGCC   1080
   AATGGAACCA AATATAGACC TCCTGCAAAA CTATTAAAGG AAAGGGGTTT CTTCGGAGCT   1140
   ATTGCTGGTT TCTTAGAAGG AGGATGGGAA GGAATGATTG CAGGTTGGCA CGGATACACA   1200
   TCTCACGGAG CACATGGAGT GGCAGTGGCA GCAGACCTTA AGAGTACGCA AGAAGCCATA   1260
   AACAAGATAA CAAAAAATCT CAATTCTTTG AGTGAGCTAG AAGTAAAGAA TCTTCAAAGG   1320
   CTAAGTGGTG CCATGGATGA ACTCCACAAC GAAATACTCG AGCTGGATGA GAAAGTGGAT   1380
   GATCTCAGAG CTGACACAAT AAGCTCGCAA ATAGAACTTG CAGTCTTGCT TTCCAACGAA   1440
   GGAATAATAA ACAGTGAAGA TGAGCATCTA TTGGCACTTG AGAGAAAACT AAAGAAAATG   1500
   CTGGGTCCCT CTGCTGTAGA CATAGGAAAT GGATGCTTCG AAACCAAACA CAAGTGCAAC   1560
   CAGACCTGCT TAGACAGGAT AGCTGCTGGC ACCTTTAATG CGGGAGAATT TTCTCTTCCC   1620
   ACTTTTGATT CACTGAATAT TACTGCTGCA TCTTTAAATG ATGATGGATT GGATAACCAT   1680
   ACTATACTGC TCTACTACTC AACTGCTGCT TCTAGTTTGG CGGTAACATT GATGATAGCT   1740
   ATTTTTATTG TTTATATGGT CTCCAGAGAC AATGTTTCTT GCTCCATCTG TCTGTGAGGA   1800
   TCTTAATTAA TTAA                                                     1814
(2) INFORMATION FOR SEQ ID NO:17:
   (i) SEQUENCE CHARACTERISTICS:
      (A) LENGTH: 592 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Influenza virus
      (C) INDIVIDUAL ISOLATE: B/Shanhai/4/94 rHA
   [ix] FEATURE
      (A) NAME/KEY: AcNPV 61K protein signal peptide
      (B) LOCATION: 1 to 16
   (ix) FEATURE
      (A) NAME/KEY: mature rHA
      (B) LOCATION: 19 to 574
   (xi) SEQUENCE DESCRIPTION: SEQ ID NO:17:
   Met Pro Leu Tyr Lys Leu Leu Asn Val Leu Trp Leu Val Ala Val Ser
   1               5                   10                  15
   Asn Ala Ile Pro Gly Gly Thr Asp Arg Ile Cys Thr Gly Ile Thr Ser
               20                  25                  30
   Ser Asn Ser Pro His Val Val Lys Thr Ala Thr Gln Gly Glu Val Asn
           35                  40                  45
   Val Thr Gly Val Ile Pro Leu Thr Thr Thr Pro Thr Lys Ser His Phe
       50                  55                  60
   Ala Asn Leu Lys Gly Thr Lys Thr Arg Gly Lys Leu Cys Pro Asn Cys
   65                  70                  75                  80
   Leu Asn Cys Thr Asp Leu Asp Val Ala Leu Gly Arg Pro Met Cys Val
                   85                  90                  95
   Gly Thr Thr Pro Ser Ala Lys Ala Ser Ile Leu His Glu Val Arg Pro
               100                 105                 110
   Val Thr Ser Gly Cys Phe Pro Ile Met His Asp Arg Thr Lys Ile Arg
           115                 120                 125
   Gln Leu Pro Asn Leu Leu Arg Gly Tyr Glu Asn Ile Arg Leu Ser Thr
       130                 135                 140
   Gln Asn Val Ile Asn Ala Glu Lys Ala Pro Gly Gly Pro Tyr Arg Leu
   145                 150                 155                 160
   Gly Thr Ser Gly Ser Cys Pro Asn Ala Thr Ser Arg Ser Gly Phe Phe
                   165                 170                 175
   Ala Thr Met Ala Trp Ala Val Pro Arg Asp Asn Asn Lys Thr Ala Thr
               180                 185                 190
   Asn Pro Leu Thr Val Glu Val Pro Tyr Ile Cys Thr Lys Gly Glu Asp
           195                 200                 205
   Gln Ile Thr Val Trp Gly Phe His Ser Asp Asn Lys Pro Gln Met Lys
       210                 215                 220
   Asn Leu Tyr Gly Asp Ser Asn Pro Gln Lys Phe Thr Ser Ser Ala Asn
   225                 230                 235                 240
   Gly Val Thr Thr His Tyr Val Ser Gln Ile Gly Gly Phe Pro Asp Gln
                   245                 250                 255
   Thr Glu Asp Gly Gly Leu Pro Gln Ser Gly Arg Ile Val Val Asp Tyr
               260                 265                 270
   Met Val Gln Lys Pro Gly Lys Thr Gly Thr Ile Val Tyr Gln Arg Gly
           275                 280                 285
   Val Leu Leu Pro Gln Lys Val Trp Cys Ala Ser Gly Arg Ser Lys Val
       290                 295                 300
   Ile Lys Gly Ser Leu Pro Leu Ile Gly Glu Ala Asp Cys Leu His Glu
   305                 310                 315                 320
   Lys Tyr Gly Gly Leu Asn Lys Ser Lys Pro Tyr Tyr Thr Gly Glu His
                   325                 330                 335
   Ala Lys Ala Ile Gly Asn Cys Pro Ile Trp Val Lys Thr Pro Leu Lys
               340                 345                 350
   Leu Ala Asn Gly Thr Lys Tyr Arg Pro Pro Ala Lys Leu Leu Lys Glu
           355                 360                 365
   Arg Gly Phe Phe Gly Ala Ile Ala Gly Phe Leu Glu Gly Gly Trp Glu
       370                 375                 380
   Gly Met Ile Ala Gly Trp His Gly Tyr Thr Ser His Gly Ala His Gly
   385                 390                 395                 400
   Val Ala Val Ala Ala Asp Leu Lys Ser Thr Gln Glu Ala Ile Asn Lys
                   405                 410                 415
   Ile Thr Lys Asn Leu Asn Ser Leu Ser Glu Leu Glu Val Lys Asn Leu
               420                 425                 430
   Gln Arg Leu Ser Gly Ala Met Asp Glu Leu His Asn Glu Ile Leu Glu
           435                 440                 445
   Leu Asp Glu Lys Val Asp Asp Leu Arg Ala Asp Thr Ile Ser Ser Gln
       450                 455                 460
   Ile Glu Leu Ala Val Leu Leu Ser Asn Glu Gly Ile Ile Asn Ser Glu
   465                 470                 475                 480
   Asp Glu His Leu Leu Ala Leu Glu Arg Lys Leu Lys Lys Met Leu Gly
                   485                 490                 495
   Pro Ser Ala Val Asp Ile Gly Asn Gly Cys Phe Glu Thr Lys His Lys
               500                 505                 510
   Cys Asn Gln Thr Cys Leu Asp Arg Ile Ala Ala Gly Thr Phe Asn Ala
           515                 520                 525
   Gly Glu Phe Ser Leu Pro Thr Phe Asp Ser Leu Asn Ile Thr Ala Ala
       530                 535                 540
   Ser Leu Asn Asp Asp Gly Leu Asp Asn His Thr Ile Leu Leu Tyr Tyr
   545                 550                 555                 560
   Ser Thr Ala Ala Ser Ser Leu Ala Val Thr Leu Met Ile Ala Ile Phe
                   565                 570                 575
   Ile Val Tyr Met Val Ser Arg Asp Asn Val Ser Cys Ser Ile Cys Leu
               580                 585                 590
(2) INFORMATION FOR SEQ ID NO:16:
   (i) SEQUENCE CHARACTERISTICS:
      (A) LENGTH: 1802 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Influenza virus
      (C) INDIVIDUAL ISOLATE: B/Harbin/7/94 rHA
   (ix) FEATURE
      (A) NAME/KEY: polyhedrin mRNA leader (partial)
      (B) LOCATION: 1 to 18
   (ix) FEATURE
      (A) NAME/KEY: coding region for HA signal peptide sequence
      (B) LOCATION: 19 to 69
   (ix) FEATURE
      (A) NAME/KEY: smaI restriction site
      (B) LOCATION: 22 to 27
   (ix) FEATURE
      (A) NAME/KEY: coding region for mature rHA
      (B) LOCATION: 70 to 1776
   (ix) FEATURE
      (A) NAME/KEY: KpnI restriction site
      (B) LOCATION: 1780 to 1785
   (ix) FEATURE
      (A) NAME/KEY: BglII restriction site
      (B) LOCATION: 1786 to 1791
   (ix) FEATURE
      (A) NAME/KEY: unversal translation termination signal
      (B) LOCATION: 1792 to 1802
   (xi) SEQUENCE DESCRIPTION: SEQ ID NO:18:
(2) INFORMATION FOR SEQ ID NO:19:
   (i) SEQUENCE CHARACTERISTICS:
      (A) LENGTH: 586 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) PRAGMENT TYPE: N-terminal
   (vi) ORIGINAL SOURCE:
      (A) ORGANISM: Influenza virus
      (C) INDIVIDUAL ISOLATE: B/Harbin/7/94 rHA
   (ix) FEATURE
      (A) NAME/KEY: HA signal peptide
      (B) LOCATION: 1 to 17
   (ix) FEATURE
      (A) NAME/KEY: mature rHA
      (B) LOCATION: 18 to 569
   (xi) SEQUENCE DESCRIPTION: SEQ ID NO:19:
   Met Pro Gly Lys Ala Ile Ile Val Leu Leu Met Val Val Thr Ser Asn
   1               5                   10                  15
   Ala Asp Arg Ile Cys Thr Gly Ile Thr Ser Ser Asn Ser Pro His Val
               20                  25                  30
   Val Lys Thr Ala Thr Gln Gly Glu Val Asn Val Thr Gly Val Ile Pro
           35                  40                  45
   Leu Thr Thr Thr Pro Thr Lys Ser His Phe Ala Asn Leu Lys Gly Thr
       50                  55                  60
   Lys Thr Arg Gly Lys Leu Cys Pro Asn Cys Leu Asn Cys Thr Asp Leu
   65                  70                  75                  80
   Asp Val Ala Leu Gly Arg Pro Met Cys Val Gly Thr Thr Pro Ser Ala
                   85                  90                  95
   Lys Ala Ser Ile Leu His Glu Val Arg Pro Val Thr Ser Gly Cys Phe
               100                 105                 110
   Pro Ile Met His Asp Arg Thr Lys Ile Arg Gln Leu Pro Asn Leu Leu
           115                 120                 125
   Arg Gly Tyr Glu Asn Ile Arg Leu Ser Thr Gln Asn Val Ile Asn Ala
       130                 135                 140
   Glu Lys Ala Pro Gly Gly Pro Tyr Arg Leu Gly Thr Ser Gly Ser Cys
   145                 150                 155                 160
   Pro Asn Ala Thr Ser Arg Ser Gly Phe Phe Ala Thr Met Ala Trp Ala
                   165                 170                 175
   Val Pro Arg Asp Asp Asn Lys Thr Ala Thr Asn Pro Leu Thr Val Glu
               180                 185                 190
   Val Pro Tyr Val Cys Thr Glu Gly Glu Asp Gln Ile Thr Val Trp Gly
           195                 200                 205
   Phe His Ser Asp Asn Lys Ala Gln Met Lys Asn Leu Tyr Gly Asp Ser
       210                 215                 220
   Asn Pro Gln Lys Phe Thr Ser Ser Ala Asn Gly Val Thr Thr His Tyr
   225                 230                 235                 240
   Val Ser Gln Ile Gly Gly Phe Pro Asp Gln Thr Glu Asp Gly Gly Leu
                   245                 250                 255
   Pro Gln Ser Gly Arg Ile Val Val Asp Tyr Met Val Gln Lys Pro Gly
               260                 265                 270
   Lys Thr Gly Thr Ile Val Tyr Gln Arg Gly Val Leu Leu Pro Gln Lys
           275                 280                 285
   Val Trp Cys Ala Ser Gly Arg Ser Lys Val Ile Lys Gly Ser Leu Pro
       290                 295                 300
   Leu Ile Gly Glu Ala Asp Cys Leu His Glu Lys Tyr Gly Gly Leu Asn
   305                 310                 315                 320
   Lys Ser Lys Pro Tyr Tyr Thr Gly Glu His Ala Lys Ala Ile Gly Asn
                   325                 330                 335
   Cys Pro Ile Trp Val Lys Thr Pro Leu Lys Leu Ala Asn Gly Thr Lys
               340                 345                 350
   Tyr Arg Pro Pro Ala Lys Leu Leu Lys Glu Arg Gly Phe Phe Gly Ala
           355                 360                 365
   Ile Ala Gly Phe Leu Glu Gly Gly Trp Glu Gly Met Ile Ala Gly Trp
       370                 375                 380
   His Gly Tyr Thr Ser His Gly Ala His Gly Val Ala Val Ala Ala Asp
   385                 390                 395                 400
   Leu Lys Ser Thr Gln Glu Ala Ile Asn Lys Ile Thr Lys Asn Leu Asn
                   405                 410                 415
   Ser Leu Ser Glu Leu Glu Val Lys Asn Leu Gln Arg Leu Ser Gly Ala
               420                 425                 430
   Met Asp Glu Leu His Asn Glu Ile Leu Glu Leu Asp Glu Lys Val Asp
           435                 440                 445
   Asp Leu Arg Ala Asp Thr Ile Ser Ser Gln Ile Glu Leu Ala Val Leu
       450                 455                 460
   Leu Ser Asn Glu Gly Ile Ile Asn Ser Glu Asp Glu His Leu Leu Ala
   465                 470                 475                 480
   Leu Glu Arg Lys Leu Lys Lys Met Leu Gly Pro Ser Ala Val Asp Ile
                   485                 490                 495
   Gly Asn Gly Cys Phe Glu Thr Lys His Lys Cys Asn Gln Thr Cys Leu
               500                 505                 510
   Asp Arg Ile Ala Ala Gly Thr Phe Asn Ala Gly Glu Phe Ser Leu Pro
           515                 520                 525
   Thr Phe Asp Ser Leu Asn Ile Thr Ala Ala Ser Leu Asn Asp Asp Gly
       530                 535                 540
   Leu Asp Asn His Thr Ile Leu Leu Tyr Tyr Ser Thr Ala Ala Ser Ser
   545                 550                 555                 560
   Leu Ala Val Thr Leu Met Ile Ala Ile Phe Ile Val Tyr Met Val Ser
                   565                 570                 575
   Arg Asp Asn Val Ser Cys Ser Ile Cys Leu
               580                 585
(2) INFORMATION FOR SEQ ID NO:20:
   (i) SEQUENCE CHARACTERISTICS:
      (A) LENGTH: 1757 base pairs
      (B) TYPE: nucleic acid
      (C) STRANDEDNESS: single
      (D) TOPOLOGY: linear
   (ii) MOLECULE TYPE: DNA (genomic)
   liii) HYPOTHETICAL: NO
   (iv) ANTI-SENSE: NO
   (vi) ORIGINAL SOURCE:
      (A) ORGANISM: Influenza virus
      (C) INDIVIDUAL ISOLATE: A/Johannesburg/33/94 rHA
   (ix) FEATURE
      (A) NAME/KEY: polyhedrin mRNA leader (partial)
      (B) LOCATION: 1 to 18
   (ix) FEATURE
      (A) NAME/KEY: coding region for AcNPV 61K protein signal peptide
      (B) LOCATION: 19 to 72
   (ix) FEATURE
      (A) NAME/KEY: SmaI restriction site
      (B) LOCATION: 76 to 81
   (ix) FEATURE
      (A) NAME/KEY: coding-region for mature rHA
      (B) LOCATION: 73 to 1731
   (ix) FEATURE
      (A) NAME/KEY: KpnI restriction site
      (B) LOCATION: 1735 to 1740
   (ix) FEATURE
      (A) NAME/KEY: BglII restriction site
      (B) LOCATION: 1741 to 1747
   (ix) FEATURE
      (A) NAME/KEY: unversal translation termination signal
      (B) LOCATION: 1747 to 1757
   (xi) SEQUENCE DESCRIPTION: SEQ ID NO:20:
   TAAAAAAACC TATAAATAAT GCCCTTGTAC AAATTGTTAA ACGTTTTGTG GTTGGTCGCC     60
   GTTTCTAACG CGATTCCCGG GCAGGACCTT CCAGGAAATG ACAACAGCAC AGCAACGCTG    120
   TGCCTGGGAC ACCATGCAGT GCCAAACGGA ACGCTAGTGA AAACAATCAC GAATGATCAA    180
   ATTGAAGTGA CTAATGCTAC TGAGCTGGTT CAGAGTTCCC CAACAGGTAG AATATGCGAC    240
   AGTCCTCACC GAATCCTTGA TGGAAAGAAC TGCACACTGA TAGATGCTCT ATTGGGAGAC    300
   CCTCATTGTG ATGGCTTCCA AAATAAGGAA TGGGACCTTT TTGTTGAACG CAGCAAAGCT    360
   TACAGCAACT GTTACCCTTA TGATGTGCCG GATTATGCCT CCCTTAGGTC ACTAGTTGCC    420
   TCATCAGGCA CCCTGGAGTT TATCAACGAA AACTTCAATT GGACTGGAGT CGCTCAGGAT    480
   GGGAAAAGCT ATGCTTGCAA AAGGGGATCT GTTAACAGTT TCTTTAGTAG ATTGAATTGG    540
   TTGCACAAAT TAGAATACAA ATATCCAGCG CTGAACGTGA CTATGCCAAA CAATGGCAAA    600
   TTTGACAAAT TGTACATTTG GGGGGTTCAC CACCCGAGCA CGGACAGTGA CCAAACCAGC    660
   CTATATGTCC GAGCATCAGG GAGAGTCACA GTCTCTACCA AAAGAAGCCA ACAAACTGTA    720
   ATCCCGGATA TCGGGTATAG ACCATGGGTA AGGGGTCAGT CCAGTAGAAT AGGCATCTAT    780
   TGGACAATAG TAAAACCGGG AGACATACTT TTGATTAATA GCACAGGGAA TCTAATTGCT    840
   CCTCGGGGTT ACTTCAAAAT ACGAAATGGG AAAAGCTCAA TAATGAGGTC AGATGCACCC    900
   ATTGGCAACT GCAGTTCTGA ATGCATCACT CCAAATGGAA GCATTCCCAA TGACAAACCT    960
   TTTCAAAATG TAAACAGGAT CACATATGGG GCCTGCCCCA GATATGTTAA GCAAAACACT   1020
   CTGAAATTGG CAACAGGGAT GCGGAATGTA CCAGAGAAAC AAACTAGAGG CATATTCGGC   1080
   GCAATCGCAG GTTTCATAGA AAATGGTTGG GAGGGAATGG TAGACGGTTG GTACGGTTTC   1140
   AGGCATCAAA ATTCTGAGGG CACAGGACAA GCTGCAGATC TTAAAAGCAC TCAAGCAGCA   1200
   ATCGACCAAA TCAACGGGAA ACTGAATAGG TTAGTCGAGA AAACGAACGA GAAATTCCAT   1260
   CAAATCGAAA AAGAATTCTC AGAAGTAGAA GGGAGAATTC AGGACCTCGA GAAATATGTT   1320
   GAAGACACTA AAATAGATCT CTGGTCTTAC AATGCGGAGC TTCTTGTTGC TCTGGAGAAC   1380
   CAACATACAA TTGATCTAAC TGACTCAGAA ATGAACAAAC TGTTTGAAAG AACAAGGAAG   1440
   CAACTGAGGG AAAATGCTGA GGACATGGGC AATGGTTGTT TCAAAATATA CCACAAATGT   1500
   GACAATGCCT GCATAGGGTC AATCAGAAAT GGAACTTATG ACCATGATGT ATACAGAGAC   1560
   GAAGCATTAA ACAACCGGTT CCAGATCAAA GGTGTTGAGC TGAAGTCAGG ATACAAAGAT   1620
   TGGATTCTAT GGATTTCCTT TGCCATATCA TGCTTTTTGC TTTGTGTTGT TTTGCTTGGG   1680
   TTCATCATGT GGGCCTGCCA AAAAGGCAAC ATTAGGTGCA ACATTTGCAT TTGAGGTACC   1740
   AGATCTTAAT TAATTAA                                                  1757
(2) INFORMATION FOR SEQ ID NO:21:
   (i) SEQUENCE CHARACTERISTICS:
      (A) LENGTH: 571 amino acids
      (B) TYPE: amino acid
      (C) STRANDEDNESS: single
      (D) TYPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Influenza virus
      (C) INDIVIDUAL ISOLATE: A/Johannesburg/33/94 rHA
   (ix) FEATURE
      (A) NAME/KEY: AcNPV 61K protein signal sequence
      (B) LOCATION: 1 to 18
   (ix) FEATURE
      (A) NAME/KEY: mature rHA
      (B) LOCATION: 19 to 569
   (xi) SEQUENCE DESCRIPTION: SEQ ID NO:21:
   Met Pro Leu Tyr Lys Leu Leu Asn Val Leu Trp Leu Val Ala Val Ser
   1               5                   10                  15
   Asn Ala Ile Pro Gly Gln Asp Leu Pro Gly Asn Asp Asn Ser Thr Ala
               20                  25                  30
   Thr Leu Cys Leu Gly His His Ala Val Pro Asn Gly Thr Leu Val Lys
           35                  40                  45
   Thr Ile Thr Asn Asp Gln Ile Glu Val Thr Asn Ala Thr Glu Leu Val
       50                  55                  60
   Gln Ser Ser Pro Thr Gly Arg Ile Cys Asp Ser Pro His Arg Ile Leu
   65                  70                  75                  80
   Asp Gly Lys Asn Cys Thr Leu Ile Asp Ala Leu Leu Gly Asp Pro His
                   85                  90                  95
   Cys Asp Gly Phe Gln Asn Lys Glu Trp Asp Leu Phe Val Glu Arg Ser
               100                 105                 110
   Lys Ala Tyr Ser Asn Cys Tyr Pro Tyr Asp Val Pro Asp Tyr Ala Ser
           115                 120                 125
   Leu Arg Ser Leu Val Ala Ser Ser Gly Thr Leu Glu Phe Ile Asn Glu
       130                 135                 140
   Asn Phe Asn Trp Thr Gly Val Ala Gln Asp Gly Lys Ser Tyr Ala Cys
   145                 150                 155                 160
   Lys Arg Gly Ser Val Asn Ser Phe Phe Ser Arg Leu Asn Trp Leu His
                   165                 170                 175
   Lys Leu Glu Tyr Lys Tyr Pro Ala Leu Asn Val Thr Met Pro Asn Asn
               180                 185                 190
   Gly Lys Phe Asp Lys Leu Tyr Ile Trp Gly Val His His Pro Ser Thr
           195                 200                 205
   Asp Ser Asp Gln Thr Ser Leu Tyr Val Arg Ala Ser Gly Arg Val Thr
       210                 215                 220
   Val Ser Thr Lys Arg Ser Gln Gln Thr Val Ile Pro Asp Ile Gly Tyr
   225                 230                 235                 240
   Arg Pro Trp Val Arg Gly Gln Ser Ser Arg Ile Gly Ile Tyr Trp Thr
                   245                 250                 255
   Ile Val Lys Pro Gly Asp Ile Leu Leu Ile Asn Ser Thr Gly Asn Leu
               260                 265                 270
   Ile Ala Pro Arg Gly Tyr Phe Lys Ile Arg Asn Gly Lys Ser Ser Ile
           275                 280                 285
   Met Arg Ser Asp Ala Pro Ile Gly Asn Cys Ser Ser Glu Cys Ile Thr
       290                 295                 300
   Pro Asn Gly Ser Ile Pro Asn Asp Lys Pro Phe Gln Asn Val Asn Arg
   305                 310                 315                 320
   Ile Thr Tyr Gly Ala Cys Pro Arg Tyr Val Lys Gln Asn Thr Leu Lys
                   325                 330                 335
   Leu Ala Thr Gly Met Arg Asn Val Pro Glu Lys Gln Thr Arg Gly Ile
               340                 345                 350
   Phe Gly Ala Ile Ala Gly Phe Ile Glu Asn Gly Trp Glu Gly Met Val
           355                 360                 365
   Asp Gly Trp Tyr Gly Phe Arg His Gln Asn Ser Glu Gly Thr Gly Gln
   370 375 380
   Ala Ala Asp Leu Lys Ser Thr Gln Ala Ala Ile Asp Gln Ile Asn Gly
   385 390 395 400
   Lys Leu Asn Arg Leu Val Glu Lys Thr Asn Glu Lys Phe His Gln Ile
   405 410 415
   Glu Lys Glu Phe Ser Glu Val Glu Gly Arg Ile Gln Asp Leu Glu Lys
   420 425 430
   Tyr Val Glu Asp Thr Lys Ile Asp Leu Trp Ser Tyr Asn Ala Glu Leu
   435 440 445
   Leu Val Ala Leu Glu Asn Gln His Thr Ile Asp Leu Thr Asp Ser Glu
   450 455 460
   Met Asn Lys Leu Phe Glu Arg Thr Arg Lys Gln Leu Arg Glu Asn Ala
   465 470 475 480
   Glu Asp Met Gly Asn Gly Cys Phe Lys Ile Tyr His Lys Cys Asp Asn
   485 490 495
   Ala Cys Ile Gly Ser Ile Arg Asn Gly Thr Tyr Asp His Asp Val Tyr
   500 505 510
   Arg Asp Glu Ala Leu Asn Asn Arg Phe Gln Ile Lys Gly Val Glu Leu
   515 520 525
   Lys Ser Gly Tyr Lys Asp Trp Ile Leu Trp Ile Ser Phe Ala Ile Ser
   530 535 540
   Cys Phe Leu Leu Cys Val Val Leu Leu Gly Phe Ile Met Trp Ala Cys
   545 550 555 560
   Gln Lys Gly Asn Ile Arg Cys Asn Ile Cys Ile
   565 570
(2) INFORMATION FOR SEQ ID NO:22:
   (i) SEQUENCE CHARACTERISTICS:
      (A) LENGTH: 39 bases
      (B) TYPE: nucleic acid
      (C) STRANDEDNESS: single
      (D) TOPOLOGY: linear
   (xi) SEQUENCE DESCRIPTION: SEQ ID NO:22:
      TGGTTGGTCG CCGTTTCTAA CGCGATTCCC GGGGGTACC 39
(2) INFORMATION FOR SEQ ID NO:23:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:23:
   Trp Leu Val Ala Val Ser Asn Ala Ile Pro Gly Gly Thr
   1 5 10
(2) INFORMATION FOR SEQ ID NO:24:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (xi) SEQUENCE DESCRIPTION: SEQ ID NO:24:
      TGGTTAGTCG CCGTGTCCTG CAGGCCAGAG AGGCCTTGGT ACC 43
(2) INFORMATION FOR SEQ ID NO:25:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:25:
      GTCGCCGTGT CCAACGCG 18
(2) INFORMATION FOR SEQ ID NO:26:
   (i) SEQUENCE CHARACTERISTICS:
      (A) LENGTH: 1B base pairs
      (B) TYPE: nucleic acid
      (C) STRANDEDNESS: single
      (D) TOPOLOGY: linear
   (xi) SEQUENCE DESCRIPTION: SEQ ID NO:26:
      TAATTGGCCA GAGAGGCC 18
(2) INFORMATION FOR SEQ ID NO:27:
   (i) SEQUENCE CHARACTERISTICS:
      (A) LENGTH: 39 bases
      (B) TYPE: nucleic acid
      (C) STRANDEDNESS: single
      (D) TOPOLOGY: linear
   (xi) SEQUENCE DESCRIPTION: SEQ ID NO:27:
      GGGGGATCCG GTACCAGCAA AAGCAGGGGA TAATTCTAT 39
(2) INFORMATION FOR SEQ ID NO:28:
   (i) SEQUENCE CHARACTERISTICS:
      (A) LENGTH: 38 bases
      (B) TYPE: nucleic acid
      (C) STRANDEDNESS: single
      (D) TOPOLOGY: linear
   (xi) SEQUENCE DESCRIPTION: SEQ ID NO:28:
      GGGGGTACCC CCGGGGACTT TCCAGGAAAT GACAACAG 38
(2) INFORMATION FOR SEQ ID NO:29:
   (i) SEQUENCE CHARACTERISTICS:
      (A) LENGTH: 44 bases
      (B) TYPE: nucleic acid
      (C) STRANDEDNESS: single
      (D) TOPOLOGY: linear
   (xi) SEQUENCE DESCRIPTION: SEQ ID NO:29:
      CCCGGTACCG AATCATCCTA GAAACAAGGG TGTTTTTAAT TAAT 44
(2) INFORMATION FOR SEQ ID NO.30:
   (i) SEQUENCE CHARACTERISTICS:
      (A) LENGTH: 47 bases
      (B) TYPE: nucleic acid
      (C) STRANDEDNESS: single
      (D) TOPOLOGY: linear
   (xi) SEQUENCE DESCRIPTION: SEQ ID NO:30:
      GGGGAATTCG GTACCCCCGG GAAGGCAATA ATTGTACTAC TCATGGT 47
(2) INFORMATION FOR SEQ ID NO:31:
   (i) SEQUENCE CHARACTERISTICS:
      (A) LENGTH: 36 bases
      (B) TYPE: nucleic acid
      (C) STRANDEDNESS: single
      (D) TOPOLOGY: linear
   (xi) SEQUENCE DESCRIPTION: SEQ ID NO:31:
      GGTACCCCCG GGGATCGAAT CTGCACTGGG ATAACA 36
(2) INFORMATION FOR SEQ ID NO:32:
   (i) SEQUENCE CHARACTERISTICS:
      (A) LENGTH: 50 bases
      (B) TYPE: nucleic acid
      (C) STRANDEDNESS: single
      (D) TOPOLOGY: linear
   (xi) SEQUENCE DESCRIPTION: SEQ ID NO:32:
      GGGGAATTCG GATCCGGTAC CTCACAGACA GATGGARCAA CAAACATTGT 50

## Description

### Background of the invention

The present invention is generally in the area of recombinant influenza vaccines.

Epidemic Influenza occurs annually and is a cause of significant morbidity and mortality worldwide. Children have the highest attack rate, and are largely responsible for transmission of influenza viruses In the community. The elderly and persons with underlying health problems are at increased risk for complications and hospitalization from influenza infection. In the United States alone, more than 10,000 deaths occurred during each of seven influenza seasons between 1956 and 1988 due to pneumonia and influenza, and greater than 40,000 deaths were reported for each of two seasons (Update: Influenza Activity - United States and Worldwide, and Composition of the 1992-1993 Influenza Vaccine, Morbidity_and Mortality Weekly Report. U.S. Department of Health and Human Services, Public Health Service, 41/No. 18:315-323,1992.) influenza viruses are highly pleomorphic particles composed of two surface glycoproteins, hemagglutinin (HA) and neuraminidase (NA). The HA mediates attachment of the virus to the host cell and viral-call membrane fusion during penetration of the virus into the cell. The influenza virus genome consists of eight single-stranded negative-sense RNA segments of which the fourth largest segment encodes the HA gene. The influenza viruses are divided into types A, B and C based on antigenic differences. Influenza A viruses are described by a nomenclature which includes the sub-type or type, geographic origin, strain number, and year of isolation, for example, A/Beljing/353/89. There are at least 13 sub-types of HA (H1-H13) and nine subtypes of NA (N1-N9). All subtypes are found in birds, but only H1-H3 and N1-N2 are found in humans, swine and horses (Murphy and Webster, "Orthomyxo-viruses", in Virology, ed. Fields, B.N., Knipe, D.M., Chanock, R.M., 1091-1152 (Raven Press, New York, (1990)).

Antibodies to HA neutralize the virus and form the basis for natural immunity to infection by influenza (Clements, "influenza Vaccines", in Vaccines: New Approaches to Immunological Problems, ed. Ronald W. Ellis, pp.129-150 (Butterworth-Heinemann, Stoneham, MA 1992)). Antigenic variation in the HA molecule is responsible for frequent outbreaks to influenza and for limited control of infection by immunization.

The three-dimensional structure of HA and the interaction with its cellular receptor, sialic acid, has been extensively studied (Wilson, et al, "Structure of the hemagglutinin membrane glycoprotein of influenza virus at 3A* resolution" Nature 289:366-378 (1981); Weis, et al, "Structure of the influenza virus hemagglutinin complexed with its receptor, sialic acid" Nature, 333:426-431 (1988); Murphy and Webster, 1990). The HA molecule is present in the virion as a trimer. Each monomer exists as two chains, HA1 and HA2, linked by a single disulfide bond. Infected host cells produce a precursor glycosylated polypeptide (HAO) with a molecular weight of about 85,000, which is subsequently cleaved into HA1 and HA2.

The presence of influenza HA-specific neutralizing IgG and IgA antibody is associated with resistance to infection and illness (Clements, 1992). Inactivated whole virus or partially purified (split subunit) influenza vaccines are standardized to the quantity of HA from each strain. Influenza vaccines usually include 7 to 25 micrograms HA from each of three strains of influenza.

The role of the other major surface glycoprotein, NA, in protective immunity of antibody or T-cell responses against influenza has not been defined. Neuraminidase is very labile to the process of purification and storage (Murphy and Webster, 1990) and the quantity of NA in the current influenza vaccines is not standardized. Purified HA but not NA vaccine prevents disease in animals challenged with influenza (Johansson, et al, "Purified influenza virus hemagglutinin and neuraminidase are equivalent in stimulation of antibody response but induce contrasting types of immunity to infection" J. Virology, 63:1239-1246 (1989)). An experimental vaccine based on neuraminidase antigen was not found to be protective in a human trial (Orga et al, J. Intact. Dis. 135:499-506 (1977)).

Licensed influenza vaccines consist of formalin-inactivated whole or chemically split subunit preparations from two influenza A subtype (H1N1 and H3N2) and one influenza B subtype viruses. Prior to each influenza season, the U.S. Food and Drug Administration's Vaccines and Related Biologicals Advisory Committee recommends the composition of a trivalent influenza vaccine for the upcoming season. The 1992-93 vaccine contained A/Texas/36/91-like (H1N1). A/Beijing/353/89-like(H3N2), and B/Panama/45/90 viruses. The FDA has advised that the 1993-94 influenza vaccine should contain the same Texas and Panama strains and a new influenza A Beijing strain (A/Beijing/32/92).

Vaccination of high-risk persons each year before the influenza season is the most effective measure for reducing the impact of influenza. Limitations of the currently available vaccines include low use rates; poor efficacy in the elderly and in young children; production in eggs; antigenic variation; and adverse reactions.

The Center for Disease Control (CDC) estimates that less than 30% of the individuals at high-risk for influenza are vaccinated each year (MMWR, 1992). The current inactivated vaccines achieve a high rate of protection against disease among normal healthy adults when the antigens of the vaccine and those of the circulating influenza viruses are closely related. Among the elderly, the rate of protection against illness is much lower, especially for those who are institutionalized (Clements, 1992). In a recent study by Powers and Belshe, J. Inf. Dis. 167:584-592 (1993), significant antibody responses to a trivalent subvirion Influenza vaccine were observed in less than 30 percent of subjects 65 years old or older.

Seed viruses for influenza A and B vaccines are naturally occurring strains that replicate to high titers in the allantoic cavity of chicken eggs. Alternatively, the strain for the influenza A component is a reassortant virus with the correct surface antigen genes. A reassortant virus is one that, due to segmentation of the viral genome, has characteristics of each parental strain. When more than one influenza viral strains infect a cell, these viral segments mix to create progeny virion containing various assortments of genes from both parents.

Protection with current whole or split influenza vaccines is shart-lived and wanes as antigenic drift occurs in epidemic strains of influenza. Influenza viruses undergo antigenic drift as a result of immune selection of viruses with amino acid sequence changes in the hemagglutinin molecule. Ideally, the vaccine strains match the influenza virus strains causing disease. The current manufacturing process for influenza vaccines, however, is limited by propagation of the virus in embryonated chicken eggs. Not all influenza virus strains replicate well in eggs; thus the viruses must be adapted or viral reassortants constructed. Extensive heterogeneity occurs In the hemagglutinin of egg-grown influenza viruses as compared to primary isolates from infected individuals grown in mammalian cells (Wang, et al, Virol. 171: 275-279 (1989); Rajakumar, et al, Proc. Natl. Acad. Sci. USA 87:4154-4158 (1990)). The changes in HA during the selection and manufacture of influenza vaccines can result in a mixture of antigenically distinct subpopulations of virus. The viruses in the vaccine may therefore differ from the variants within the epidemic strains, resulting in suboptimal levels of protection.

Kuroda et al. EMBO J. 5(6) (1986) 1359-1366 disposes the cloning of a cDNA sequence of the influenza (fowl plague) virus haemagglutinin gene being inserted into the BamH1 site of the pAc373 polyhedrin vector. Partial purification of HA by immunoprecipitation is described. Immunization of chicken with insect cells transfected with baculovirus and expressing HA leads to a protective immune response.

EP-A-0 546 787 describes the construction of an HA expression vector, wherein expression of the HA gene is under the control of the polyhedron promoter. The HA glycoprotein was partially purified and used to immunize guinea-pigs.

Possee R.D., Virus Research 5 (1986) 43-59 describes expression of HA under control of the polyhedron promoter. Matsuura et al., J. General. Virol. 68 (1987) 1233-1250 also relates to expression of recombinant HA of influenza A virus using a baculovirus expression system.

It is known from Ayres et al., Virology 202(2) (1994) 586-605 that the Autographa califormica Nuclear Polyhedrosis Virus contains ten proteins with putative signal sequences located within their first 50 amino acids.

immediate hypersensitivity reactions can occur in persons with severe egg allergy due to residual egg protein in the vaccine. The 1976 swine influenza vaccine was associated with an increased frequency of Guillain-Barré syndrome. Subsequent vaccines prepared from other influenza strains have, thus far, not been observed to Increase the occurrence of this rare disease.

A method of producing an influenza vaccine that does not require propagation in eggs would result in a a purer product that would be less likely to cause an adverse immune reaction. In addition, a purer vaccine preparation would not require virus inactivation or organic extraction of Viral membrane components, thereby avoiding denaturation of antigenic epitopes and safety concerns due to residual chemicals in the vaccine.

In addition, an influenza vaccine produced in the absence of egg propagation would avoid the genetic heterogeneity that occurs during adaptation and passage through eggs. This would result in a vaccine that is better matched with influenza epidemic strains, resulting in improved efficacy.

It is therefore an object of the present invention to provide a method of producing an influenza vaccine that does not require replication in eggs.

It is a further object of the present invention to provide a method of producing an influenza vaccine that is rapid and cost-efficient, highly purified and allows production of vaccines from primary sources of influenza.

### Summary of the invention

A method of preparing a recombinant influenza hemagglutinin protein by expression in insect cells using a baculovirus expression system is provided. The resulting protein is a substantially pure, recombinant, glycosylated influenza haemagglutinin (rHAo), that is purified to at least 95% and is immunogenic, induces a protective immune response when used as a vaccine and further comprises a chitinase signal peptide. It is useful in making a multivalent influenza vaccine based on a mixture of recombinant hemagglutinin antigens cloned from influenza viruses having epidemic potential. The recombinant hemagglutinin proteins are full length, uncleaved (HAO) glycoproteins including both the HA1 and HA2 subunits (HAO) purified under non-denaturing conditions to 95% or greater purity, preferably 99% purity.

A process for cloning influenza hemagglutinin genes from influenza A and B viruses using specially designed oligonucleotide probes and polymerase chain reaction (PCR) methodology is also disclosed. In the preferred embodiment, the cloned HA genes are modified by deletion of the nucleotides encoding the natural hydrophobic signal peptide sequences and replacement with a new baculovirus signal peptide, to yield a sequence encoding the signal peptide immediately abutting the hemagglutinin. These chimeric genes are Introduced into baculovirus expression vectors so that the baculovirus polyhedrin promoter directs the expression of recombinant HA proteins in infected insect cells. The* 18 amino acid baculovirus signal peptide directs the translation of rHA into the insect cell glycosylation pathway and is not present on the mature rHA glycoprotein. In the preferred embodiment, a vector is designed that does not encode any intervening amino acids between the signal peptide and hemagglutinin protein.

This methodology can be extended to all types of influenza viruses, including but not limited to the prevalent A (H1N1) sub-type, the A(H3N2) sub-type, and the B type that infect humans, as well as the influenza viruses which infect other mammalian and avian species.

A general approach for the efficient extraction and purification of recombinant HA protein produced in insect cells is disclosed for the purification of rHA proteins from A sub-types and B type influenza viruses. The recombinant vaccine can be developed from primary sources of influenza, for example, nasal secretions from infected individuals, rather than from virus adapted to and cultured in chicken eggs. This allows rapid development of vaccine directly from epidemic strains of influenza and avoids the problems arising from adaptation of the virus for culture in eggs, as well as patient reaction to egg contamination in the resulting vaccine.

Examples demonstrate the formulation and clinical efficacy of vaccine in an immunizing dosage form including purified rHA antigens from three strains of influenza virus recommended by the FDA for the 1993/1994 and 1994/1995 influenza epidemic seasons. Functional immunity was measured using assays that quantitate antibodies that bind to influenza hemagglutinin, that block the ability of influenza virus to agglutinate red blood cells, or that neutralize the influenza virus. Protective immune responses with rHA vaccines were measured in animals that are susceptible to influenza infection or in human challenge studies.

### Brief Description of the Drawings

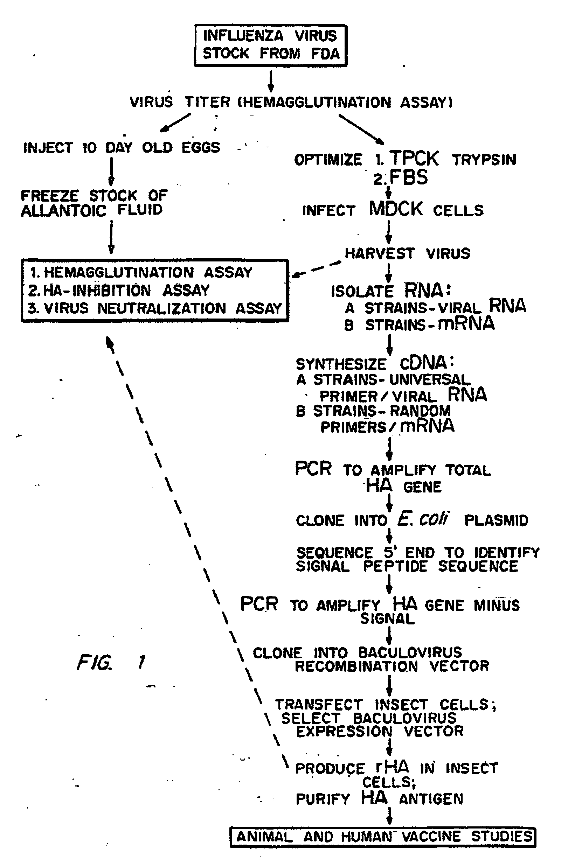

Figure 1 is a schematic of the cloning of HA genes from influenza A strains from purified viral RNA preparations, purification of expressed rHA, and biological characterization of rHA. Abbreviations: FDA, Food and Drug Administration; MDCK, Madin Darby Canlne Kidney; TPCK, tosylphenylalanyl chloromethylketone; RNA, ribonucleic acid; cDNA, complementary deoxyribonucleic acid; HA, hemagglutinin; FBS, Fetal Bovine Serum; PCR, Polymerase Chain Reaction; and BV, Baculovirus.

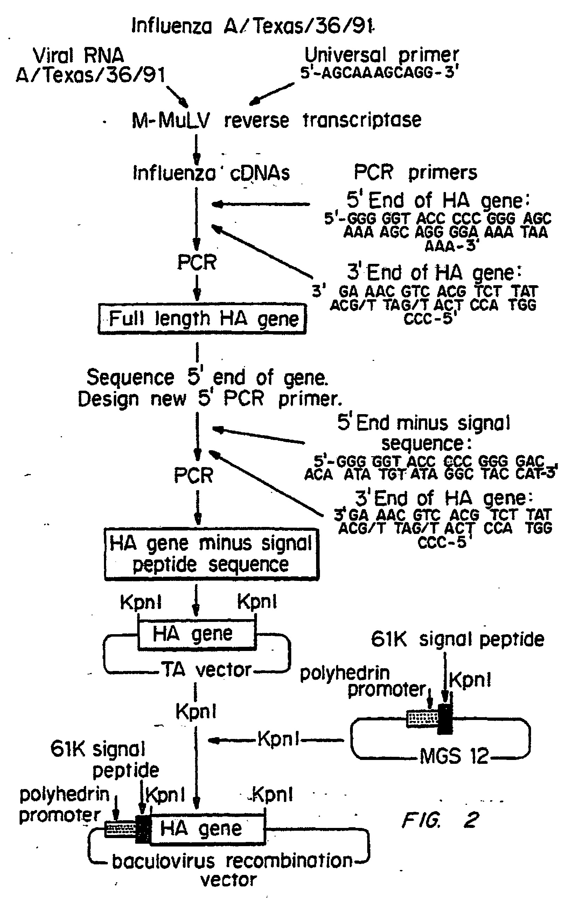

Figure 2 Is a more detailed schematic of the method of Figure 1 applied to the cloning and expression of the HA gene of the influenza A/Texas/36/91 strain. Influenza HA gene was obtained from RNA purified from MDCK cells infected with influenza A/Texas/36/91 using reverse transcriptase and universal primer (SEQ ID NO. 1) followed by two rounds of PCR amplification and cloning. As shown, in the first round of PCR reactions, 5' end primer SEQ ID NO. 2 and 3' end primer SEQ ID NO. 3 were used. In the second round of PCR reactions, 5' end primer SEQ ID NO. 4 and 3' end primer SEQ ID NO. 5 were used. A baculovirus recombination vector was constructed containing the polyhedrin promoter and a signal peptide sequence from the baculovirus 61 K gene (a baculovirus gene that encodes a signal peptide having a molecular weight of approximately 61,000), followed by the complete coding sequences for the mature HA protein. This recombination vector was then used to make a baculovirus expression vector that produces HA from this strain of the virus.

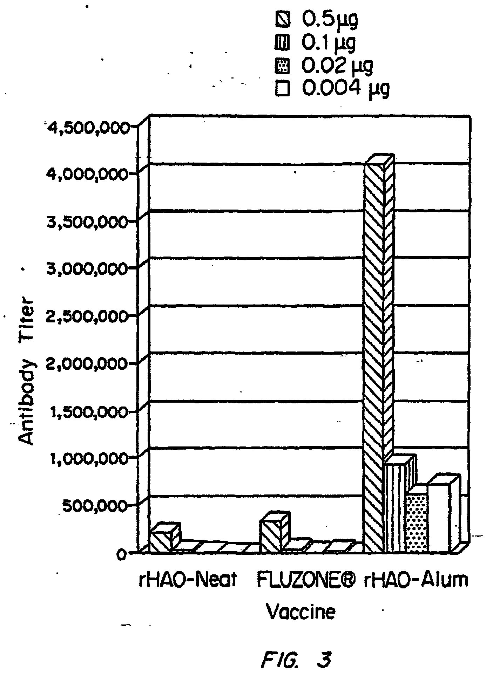

Figure 3 is a graph of the anti-HA immune response in mice, day 42, n=5, graphing antibody titer for rHA0-neat; Fluzone® vaccine, and rHA0-alum, at dosages of 0.5 µg (dark bars), 0.1 µg (shaded bars), 0.02 µg (dotted bars), and 0.004 µg (open bars).

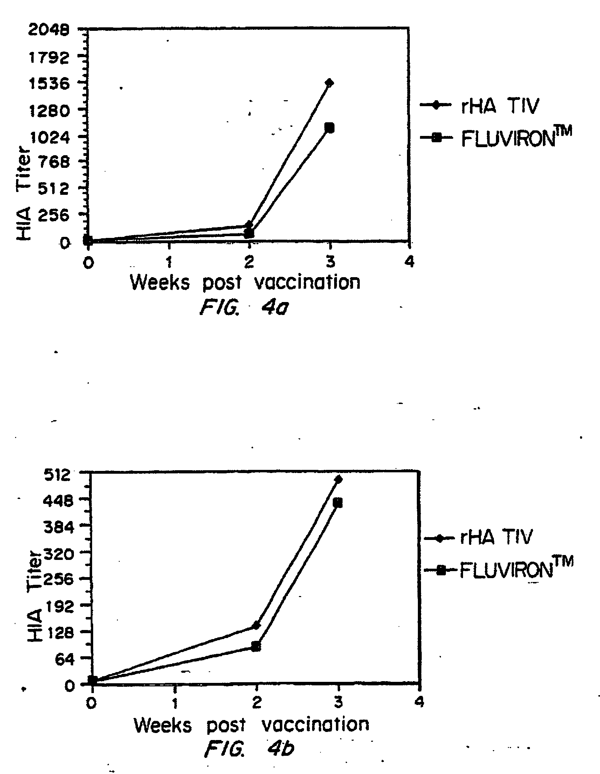

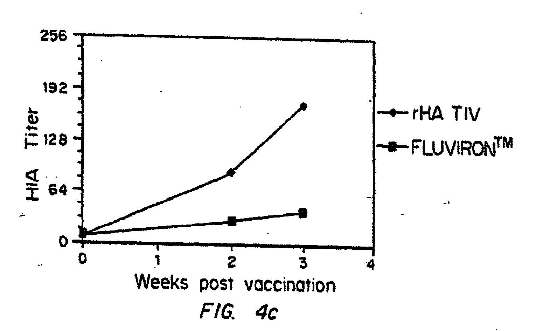

Figures 4a, 4b, and 4c are graphs of the anti-HA immune response in mice immunized with rHA or licensed trivalent vaccine, 1994-1995 formula, weeks post vaccination versus HIA titer, for HAI A/Texas/36/91 (Figure 4a), HAI A/Shangdong/9/93 (Figure 4b), and HAI B/Panama/45/90 (Figure 4c), rHA (diamonds) and FLUVIRON® attenuated vaccine cultured in eggs (squares).

### Detailed Description of the Invention

A method of preparing a recombinant influenza vaccine is described. A full length, uncleaved (HAO), hemagglutinin antigen from an influenza virus is produced with baculovirus expression vectors in cultured insect cells and purified under non-denaturing conditions. Two or more purified hemagglutinin antigens from influenza A and/or influenza B strains are mixed together to produce a multivalent influenza vaccine. The recombinant antigens may be combined with an adjuvant carrier for increased efficacy.

The use of recombinant DNA technology to produce influenza vaccines offers several advantages: a recombinant DNA influenza vaccine can be produced under safer and more stringently controlled conditions; propagation with infectious influenza in eggs is not required; recombinant HA protein can be more highly purified, virtually eliminating side effects due to contaminating proteins; purification procedures for recombinant HA do not have to include virus inactivation or organic extraction of viral membrane components, therefore avoiding denaturation of antigens and additional safety concerns due to residual chemicals in the vaccine; production of HA via recombinant DNA technology provides an opportunity to avoid the genetic heterogeneity which occurs during adaptation and passage through eggs, which should make it possible to better match vaccine stains with influenza epidemic stains, resulting in improved efficacy; and a recombinant approach may also allow for strain selection later in the year, thereby allowing time for selections based on more reliable epidemiological data.

### Baculovirus Expression System.

Baculoviruses are DNA viruses in the family *Baculoviridae.* These viruses are known to have a narrow host-range that is limited primarily to Lepidopteran species of insects (butterflies and moths). The baculovirus *Autographa califomica* Nuclear Polyhedrosis Virus (AcNPV), which has become the prototype baculovirus, replicates efficiently in susceptible cultured insect cells. AcNPV has a double-stranded closed circular DNA genome of about 130,000 basepairs and is well characterized with regard to host range, molecular biology, and genetics.

Many baculoviruses, including AcNPV, form large protein crystalline occlusions within the nucleus of infected cells. A single polypeptide, referred to as a polyhedrin, accounts for approximately 95% of the protein mass of these occlusion bodies. The gene for polyhedrin is present as a single copy in the AcNPV viral genome. Because the polyhedrin gene is not essential for virus replication in cultured cells, it can be readily modified to express foreign genes. The foreign gene sequence is inserted into the AcNPV gene just 3' to the polyhedrin promoter sequence such that it is under the transcriptional control of the polyhedrin promoter.

Recombinant baculoviruses that express foreign genes are constructed by way of homologous recombination between baculovirus DNA and chimeric plasmids containing the gene sequence of interest. Recombinant viruses can be detected by virtue of their distinct plaque morphology and plaque-purified to homogeneity.

Baculoviruses are particularly well-suited for use as eukaryotic cloning and expression vectors. They are generally safe by virtue of their narrow host range Which is restricted to arthropods. The U.S. Environmental Protection Agency (EPA) has approved the use of three baculovirus species for the control of insect pests. AcNPV has been applied to crops for many years under EPA Experimental Use Permits.

AcNPV wild type and recombinant viruses replicate in a variety of Insect cells, including continuous cell lines derived from the fall armyworm, *Spodoptera frugiperda* (Lepidoptera; Noctuidae). *S. frugiperda* cells have a population doubling time of 18 to 24 hours and can be propagated in monolayer or in free suspension cultures.

Recombinant HA proteins can be produced in, but not limited to, cells derived from the Lepidopteran species *Spodoptera frugiperda.* Other insect cells that can be infected by baculovirus, such as those from the species *Bombix mori, Galleria mellanoma, Trichplusia ni, or Lamanthria dispar,* could also be used as a suitable substrate to produce recombinant HA proteins.

The most preferred host cell line for protein production from recombinant baculoviruses is Sf900+. Another preferred hostcell line for protein production from recombinant baculoviruses is Sf9. Sf900+and Sf9 are non-transformed, non- tumorigenic continuous cell lines derived from the fall armyworm, *Spodoptera frugiperda* (Lepidoptera; Noctuidae). Sf900+ and Sf9 cells are propagated at 26±2°C without carbon dioxide supplementation. The culture medium used for Sf9 cells is TNMFH, a simple mixture of salts, vitamins, sugars and amino acids, supplemented with 10% fetal bovine serum. Aside from fetal bovine serum, no other animal derived products (i.e, trypsin, etc.) are used in cell propagation. Serum free culture medium (available as Sf900 culture media, Gibco BRL, Gaithersburg, MD) can also be used to grow Sf9 cells and is preferred for propagation of Sf900+ cells.

Sf9 cells have a population doubling time of 18-24 hours and can be propagated in monolayer or in free suspension cultures. *S. frugiperda* cells have not been reported to support the replication of any known mammalian viruses.

It will be understood by those skilled in the art.thatthe expression vector is not limited to a baculovirus expression system. The recombinant HA proteins can also be expressed in other expression vectors such as Entomopox viruses (the poxviruses of insects), cytoplasmic polyhedrosis viruses (CPV), and transformation of insect cells with the recombinant HA gene or genes constitutive expression.

### Isolation of Influenza strains.

One or more influenza strains are isolated from individuals infected with the disease. Preferably, the influenza strains are those identified by the Food and Drug Administration (FDA) or CDC to have epidemic potential for the subsequent influenza season. An advantage of the method described herein is that clinical samples, such as nasal secretions, from patients infected with influenza can be used as a direct source of virus. Alternatively, they can be obtained from the FDA or CDC.

### Propagation of Influenza strains.

The strains are then propagated in cells producing high viral titers, such as Madin Darby Canine Kidney (MD*CK) cells (available from the American Type Culture Collection under accession number ATCC CCL34). For example, MDCK cells are infected in the presence of tosylphenylalanyl chloromethylketone (TPCK) partially inactivated trypsin and fetal bovine serum concentrations optimized to produce the highest titers of first passage virus. The MDCK cells are infected with the influenza strains at a low multiplicity of infection (0.1 to 0.5) as determined by a standard HA assay (Rosen, "Hemagglutination with Animal Viruses" in Fundamental. Techniques in Virology, ed. K. Habel and N.P. Salzman, pp. 276-28-(Academic Press, New York 1969). The infected cells are incubated at 33°C for 48 hours, and the media assayed for virus production using the hemagglutination activity assay. The conditions yielding the highest HA activity are then used to prepare large stocks of influenza virus.

### Purification of Virus.

Viral particles produced from the first passage are purified from the media using a known purification method such as sucrose density gradient centrifugation. For example, virus is harvested 24-48 hours post infection by centrifuging media of influenza infected MDCK cells. The resulting viral pellet is resuspended in buffer and centrifuged through a buffered sucrose gradient. The Influenza virus band is harvested from the 40-45% sucrose region of the gradient, diluted with buffer and pelleted by centrifugation at 100,000 x g. The purified virus pellet is resuspended in buffer and stored at - 70°C.

### Cloning of Influenza Hemagglutinin Genes.

An overview of the methods for cloning HA genes is provided in Figure 1. Basically, cells are infected with the influenza strain to be cloned. Virus is harvested from the cell media and either viral RNA, for Influenza A strains, or mRNA, for influlenza B strains, is isolated. Viral RNA (-RNA) is extracted from purified virions and analyzed on formaldehyde agarose gels using standard procedures. cDNA Is synthesized, using either an universal primer system for the viral RNA from the Influenza A strains or random primers for the mRNA from influenza B strains. Plus-standard complimentary DNA (cDNA) is made using a universal oligonucleotide primer (5'-AGCAAAAGCAGG-3' (SEQ ID NO. 1)) which is homologous to all hemagglutinin RNA segments in influenza A and B viruses (Davis et al, "construction and characterization of a bacterial clone containing the hemagglutinin gene of the WSN strain (H0N1) of influenza virus" Gene, 10: 205-218 (1980)). Primers are designed that are homologous to conserved regions at the 5' and 3' end of influenza hemagglutinin genes. Both 5' and 3' primers also have restriction enzyme sites at the ends that are not found within the hemagglutinin genes.

The appropriate influenza A or B primers and influenza cDNA are mixed and the hemagglutinin gene segments amplified using standard PCR procedures. The resulting double-stranded DNA fragments contain entire mature hemagglutinin coding sequences. The polymerase chain reaction ("PCR") is used to amplify the total HA gene, which is then cloned Into a suitable bacterial host such as *E*. *coli.* The 5' ends are sequenced to identify the signal peptide of the HA genes, then PCR is used to amplify the HA genes minus the signal peptide. This is then subcloned into a plasmid transfer vector containing the AcNPV polyhedrin promoter. The resulting transfer vectors contain the following 5'->3' sequences: Polyhedrin promoterfrom the baculovirus *A*. *califomica* NPV, an ATG translational start codon, a 61 K baculovirus signal peptide, the coding sequences for mature hemagglutinin, the natural hemagglutinin translational termination codon, the polyhedrin RNA polyadenylation signal, and flanking baculovirus DNA.

A purified chimeric transfer plasmid DNA containing a cloned hemagglutinin gene is then mixed with AcNPV wild type DNA, co-precipitated with calcium and transfected into *S*. *frugiperda* cells. Recombinant baculoviruses are selected on the basis of plaque morphology and further purified by additional rounds of plaque-purification. Cloned recombinant baculoviruses are screened for hemagglutinin expression and a single baculovirus expression vector is selected to produce a Master Virus Bank.

### Influenza A Strains:

HA genes from influenza A strains are cloned from purified viral RNA preparations. Viral RNA is extracted from 100-200 microliters of purified influenza A virions containing 1,000-2,000 hemagglutination units (HAU) of influenza. One HAU is the amount of virus that will agglutinate 50% of the red blood cells in the standard agglutination assay (Rosen, 1968). The virions are treated with proteinase K to digest protein, then the viral RNA is extracted with equal volumes of phenol and chloroform, and precipitated with ethanol in the presence of tRNA carrier. The viral RNA is resuspended in buffer and digested with RNAse-free DNAse to remove any contaminating DNA, then the extraction and precipitation steps repeated. Viral RNA (vRNA) is then analyzed using formaldehyde agarose gels as described by Maniatis, et al. Molecular Cloning: A Laboratory Manual. pp. 86-96 and 366-367 (Cold Spring Harbor Lab., Cold Spring, N.Y. 1982).

### Influenza B Strains:

HA genes from influenza B strains are cloned from total messenger RNA (mRNA) extracted from cells infected with the influenza B-strain. Total DNA is then extracted from the infected cells, The harvested cells are lysed in the presence of guanidinium thiocyanate and total cell RNA is purified, using, for example, the RNA Extraction Kit from Pharmacia Biotech Inc. (Piscataway, NJ) Total mRNA is extracted from cellular RNA using Oligo-(dT)-cellulose spun columns, using, for example, the mRNA Purification Kitfrom Pharmacia Biotech Inc.

### Expression and Processing of Recombinant Hemagglutinin In Insect Cells.

Recombinant hemagglutinin antigens are expressed at high levels in *S. frugiperda* cells Infected with AcNPV-hemagglutinin vectors. The primary gene product is unprocessed, full length hemagglutinin (rHAO) and is not secreted but remains associated with peripheral membranes of infected calls. This recombinant HAO is a 68,000 molecular weight protein which is glycosylated with N-linked, high-man nose type glycans distinctfrom the glycans produced by expression of the viral proteins in mammalian or avian cells. There is evidence that rHAO forms trimers past-translationally which accumulate in cytoplasmic membranes.

### Vectors for Expression of HAO and other Proteins

HAO is a better vaccine due to its superior stability as compared to the HA1/HA2 complex, and maintains correct folding during purification and storage. The superior stability is particularly apparent with the B strains, resulting in titers that are about five fold greater than obtained with commercially available attenuated B strains.

As described below in the examples, when the HA genes were cloned in pMGS12 via restriction sites, the HA mature signal peptide was removed and replaced with the baculovirus chitinase signal peptide, referred to as the 61 kD signal peptide. Since the HA gene is connected to the chitinase signal peptide through a cleavage site, there are between three and five amino acids, depending on the restriction site selected, between the mature HAO protein and the 61 kD signal peptide. Although not a problem with the A strains of influenza, the B strain HAO expressed with the additional amino acids did not fold properly.

Two ways to overcome this problem were daveloped. The first is to use a new vector, pMGS3, which does not encode the 61 kD signal peptide. HAO with its native signal peptide is cloned into the vector and expressed. When characterized by SDS-PAGE, B strain HAO expressed in this vector shows better glycosylation and processing than when expressed in pMGS12. The HAO folded so well that it can be quantitatively converted to HA1/HA2. Unfortunately, as determined by Western blotting, the yield is not as high. The second method increases the yield by using the 61 kD signal peptide in pMGS12 to guide expression where the HAO gene was inserted without the use of restriction enzymes. The new vector, including the 61 kD signal peptide and HAO gene, without sequence encoding extraneous intervening amino acids, is referred to as pMGS27.

pMGS27 can be used for cloning and expression of any gene in a baculovirus expression system. The target gene, instead of being cloned into the vector by restriction and ligation, is cloned into the vector by annealing. Reagents are available from Clontech in their PCR-direct Cloning System. pMGS27 was designed so that it can be linearized at the end of the chitinase signal peptide coding region, and two long single-stranded tails created by treating the linearized pMGS27 with T4 DNA polymerase plus dATP.

The target gene is amplified using polymerase chain reaction ("PCR") or reverse transcriptase-PCR ("RT-PCR") with a pair of oligonucleotides designed to create single-stranded tails that are complementary to the tails of the treated pMGS27, after the PCR fragment has been treated with T4 DNA polymerase and dTTP. A simple annealing can then combine the two molecules into a circular plasmid which is ready to transform the host. Besides being quicker and simpler than the traditional restriction-ligation method of cloning a HA gene into pMCS12, the pMGS27 has the important advantage that it does not yield extra amino acids encoded by the restriction sites created between the chitinase signal peptide and the mature HA protein. These extra amino acids can sometimes create difficulties such that signal peptidase cannot cleave the signal or that the encoded protein does not fold correctly, as in the case of the B strain HA.

### Purification of Recombinant HAO.

Several days post infection, rHAO can be selectively extracted from the peripheral membranes of AcNPV-hemagglutinin infected cells with a non-denaturing, nonionic detergent or other methods known to those skilled in the art for purification of recombinant proteins from insect cells, including, but not limited to affinity or gel chromatography, and antibody binding. The detergent soluble rHA0 can be further purified using DEAE Ion exchange and lentil lectin affinity chromatography, or other equivalent methods known to those skilled in the art.

In a preferred embodiment, the rHAO is purified using a procedure that Is more gentle and results in higher yield of the rHAO from B strains of influenza. This procedure is generally as follows:
The HAO protein which forms an Integral part of the membrane of the insect cells is separated from the soluble proteins, the peripheral membrane proteins and the majority of the DNA and RNA by extraction of the cells in a relatively viscous alkaline solution, where an alkaline pH is defined as between about 9.5 and 10.5. Viscosity is increased through the inclusion of sucrose in a concentration of approximately 250 mM. A disulfide-reducing agent, for example, β-mercaptoethanol, is included in a concentration effective to prevent disulfide linking of proteins in the mixture. The cells are suspended in the extraction buffer, homogenized, and then centrifuged. The pellet is washed by homogenization in a low ionic strength buffer containing a disulfide-reducing agent at an alkaline pH (conductivity is generally less than 1 mS, pH 10.5) and the pellet centrifuged. The HAO is then extracted from the pellet in a buffer containing between 0.3 and 1.5% detergent such as Triton, an amount of disaggregating agent effect to prevent complex formation due to charge interactions, such as between 0.3 and 1.0 M betaine or paurine, at an alkaline pH (9.5 is preferred). The HAO In the supernatant is then purified by anion exchange chromatography followed by cation exchange chromatography. The HAO is applied to the anion exchange column, for example, DEAE or Q-Sepharose® (an agarose bead column with quaternary amine groups), in the same buffer as extracted but diluted at least 1:2 with additional buffer, after equilibration of the column in buffer containing approximately 1/10th the concentration of detergent and disulfide-reducing agent. The HAO is then eluted by lowering the pH to approximately 8,5. The eluted HAO is applied to a cation exchange column in essentially the same buffer. Contaminants are eluted by lowering the pH to approximately 7.4, then eluting the HAO by increasing the salt concentration to 0.15 M NaCl,

This preferred method of purification is described in detail as follows.

Preparation of the recombinant HA-containing membrane fraction. Recombinant HA expressing cells (6.2 g of cells from 0.34 L of culture) are suspended at 100 mg/mL in ice-cold 100 mM sodium pyrophosphate, 100 mM sodium chloride, 250 mM sucrose, 0.1 % β-mercaptoethanol, pH 10.5. The cells are disrupted using a Polytron® homogenizer (Brinkman Instruments Inc., Westbury, NY) at a setting of 4 for 2 min. Alkaline pH of the homogenization medium is needed to increase the solubility of the contaminating proteins and to increase the purity of the membrane preparation. The homogenate is centrifuged for 30 min. at 9,200 g. The supernatant Is discarded and the pellet collected. Preparation of the membrane fraction is followed by a low-ionic strength wash step. The pellet is resuspended to the original volume in the ice-cold 0.1 % β-mercaptoethanol, 10.5, and homogenized using a Polytron®) homogenizer at a setting of 4 for 2 min. The homogenate is centrifuged for 30 min. at 9,200 g. The supernatant is discarded and the pellet collected. This low-ionic strength wash removes additional portion of the peripheral membrane proteins, The preparation of the membrane fraction results In the considerable enrichment in the recombinant HA and in the removal of contaminating nuclei acids.

**Extraction of the recombinant HA.** The recombinant HA is then selectively extracted from the membrane pellet under conditions that do not denature the antigen. The membrane pellet is homogenized in 41 mL of ice-cold 10 mM ethanolamine pH 9.5,1% Triton N101, 0.1% β-mercaptoethanal, 25 mM NaCl, 400 mM betaine using a Polytron homogenizer at a setting of 4 for 2 min. After incubation for 40 min. at 23°C, the mixture is centrifuged for 30 min. at 9,200 g. The supernatant containing recombinant HA is decanted and diluted two-fold with the same bluffer.

Proteins are analyzed by SDS polyacrylamide gel electrophoresis. Samples are disrupted In a boiling water bath for 10 minutes in the presence of 2% sodium dodecyl sulfate (SDS) and 5% β-mercaptoethanol, then electrophoresed on an 11 % polyacrylamide gel in the presence of 0.1 % SDS, then stained with Coomassie blue.

**Chromatographic purification.** Chromatographic purification of the recombinant HA was simplified and expensive affinity chromatography on Lentil Lectin Sepharose was eliminated from the process by substitution with a two-step chromatographic purification process which results in a highly purified recombinant HA antigen that is non-denatured and suitable as a component of an influenza vaccine for human use. The chromatography gel matrices used are Pharmacia Q-Sepharose® Fast Flow and CM-Sepharose Fast Flow®.

**Anion-exchange chromatography.** All chromatography is performed at room temperature. The recombinant HA-containing extract prepared as described above is applied at 1 mL/min to Pharmacia Q-Sepharose Fast Flow® (5 mL in a C10/10 Pharmacia column) equilibrated with 10 mM ethanolamine pH 9.5, 0.1% Tritan® N101, 0.01% β-mercaptoethanol, 25 mM NaCl, 400 mM betaine. The column is then washed with the equilibration buffer until the UV absorbance of the effluent returns to the baseline. Under these conditions recombinant HA binds to the column while part of the contaminants flow through. Partially purified recombinant HA is then eluted with 30 mM diethanolamine pH 8.5, 0.1% Triton® N101, 0.01% β-mercaptoethanol, 25 mM NaCl, 400 mM betaine.

**Cation exchange chromatography.** The Q-Sepharose eluate (23 mL) is diluted two-fold with 30 mM diethanolamine pH 8.5, 0.1% Triton® N101, 0.01 % β-mercaptoethanol, 10 mM NaCl, 400 mM betaine. The column is then washed with 35 mL of 10 mM sodium phosphate pH 7.4, 0.1% Triton® N101, 0.01 % β-mercaptoethanol, 10 mM NaCl, 400 mM betaine. This treatment elutes the contaminants from the column while recombinant HA remains bound to the CM Sepharose. The detergent is then removed by washing the column with 10 mM sodium phosphate pH 7.4,10 mM NaCl until the UV absorbance of the effluent returned to the baseline. Pu rifled recombinant HA is eluted with phosphate buffer saline, pH 7.5 (PBS).

Purified rHAO is resuspended in an isotonic, buffered solution. Following the removal of the detergent, purified rHAO will efficiently agglutinate red blood cells.

### Structural and Biological Properties of Recombinant HA0.

rHA0 is purified to at least 95% purity, more preferably 99% purity. This migrates predominantly as a single major polypeptide of 68,000 molecular weight on an SDS-polyacrylamide gel. The quaternary structure of purified recombinant HA0 antigen was examined by electron microscopy, trypsin resistance, density sedimentation analysis, and ability to agglutinate red blood cells. These data show that recombinant HAO forms trimers, which assemble into rosettes.

Purified rHA0 does not agglutinate cells priorto removal of detergent, suggesting that the antigen must form complexes (rosettes) in orderto cross-linkchicken red blood cells. The quantitative ability of purified rHAO to agglutinate cells is used as a measure of lot-to-lot consistency of the antigen. One hemagglutinin unit is defined as the quantity of antigen required to achieve 50% agglutination in a standard hemagglutinin assay with chicken red blood cells. Comparative data shows that purified rHA0 antigens agglutinate red blood cells with an efficiency comparable to that observed with whole influenza visions.

The recombinant HA0 can be cleaved at the disulfide bond, causing a conformation change that results in the formation of two chains, HA1 and HA2 as described by Carr, C.M. and Kim, P.S., "A Spring-loaded Mechanism for the Conformational Change of Influenza Hemagglutin", Cell 73:823-832 (1993). Cleavage of recombinant HA0 is described in more detail below in Example 6. It is believed that, upon cleavage of natural HA0 into HA1 and HA2, the chains become infectious by acquiring the ability to fuse with a cell, thereby creating an improved immune response. The processing of antigens such as influenza hemagglutin occurs by the binding of antigenic peptides to major histocompatility (MHC) molecules. The antigen/MHC complex is recognized by T cells to initiate an immune response as described in the review by Harding and Geuze, Current Opinion in Cell Biology 5:596-605 (1993). The rHAO produced in a baculovirus, however, is highly stable and Immunogenic as the intact molecule. Comparison of the sugar molecules on the HAO expressed in insect cells shows that the glycans are different from those when the HAO is expressed in mammalian or avian cells.

### Production of Fusion Proteins

Fusion proteins consisting of the HAO fused to a second antigenic protein can be made where the antigenicity of the second protein is low or there are advantages to eliciting an immunogenic response to multiple antigens. An example of a preferred second antigen is the neuraminidase produced by influenza. The antigen can consist of a cellular, viral, or bacterial protein, or antigenic portion thereof including at least five to eight amino acids. Other antigens include hepatitis B antigen, HIV antigens, and carcinoembryonic antigen. An "immune response" as used herein, refers to either a humoral response, measured by the production of antibody to the antigen, or a cellular response, measured by the elicitation of a T cell mediated response to the antigen. In some cases a "linker" of non-antigenic amino acids may be inserted between the HA and the antigen, to further enhance antigenicity of the antigen as compared to the HA. The process involves constructing a DNA plasmid forfusing target antigen genes to full-length or fragments of the influenza virus HA gene, using oligonucleotide probes and polymerase chain reaction (PCR) methodology.

The HA-target antigen fusion genes are modified for proper expression in insect cells by deletion of the natural hydrophobic signal peptide sequences and replacement with a new baculovirus signal peptide. The fusion gene is introduced into a baculovirus expression vector so that the baculovirus polyhedron promoter directs the transcription of the fusion proteins in infected insect cells. The 18 amino acid baculovirus signal peptide directs the translation of the HA-target antigen fusion polypeptide Into the insect cell glycosylation pathway and is not present on the mature fusion protein.

For example, Plasmid pA9440, which contains the A/Beijing/32/92 strain HA gene in the pMGS12 baculovirus transfer plasmid described below, was used as a template for the amplification of the HA gene by polymerase chain reaction (PCR) using the protocol recommended by the supplier (Gene Amp PCR cloning kit, Perkin Elmer Cetus). The PCR reaction mixture (100 µl) contained 20 µmol of primers designed to anneal to portions of the HA gene. The 5' and 3' primers were designed with restriction endonuclease sites at the ends that are not found within the HA gene. The 5' PCR primer (O-567) for the HA0 and HA1 fragments begins 52 base pairs downstream from the 5' end of the natural HA gene coding sequences, deleting the natural signal peptide sequence, and adds a Smal site immediately 5' to the HA coding sequences. The 5' PCR primer (O-651) for the HA2 fragment begins at nucleotide 1108 of the natural HA gene, immediately following the codon encoding the arginine residue that is removed during cleavage of HA0 to HA1 and HA2. The 3' PCR primer (O-680) for the HA0 and HA2 fragments was designed to add a Kpnl site immediately following the HA coding sequences, removing the natural stop codon. The 3' PCR primer for HA1 (O-679) truncates the gene immediately prior to the arginine residue removed during HA0 cleavage. Amplification of the HA gene fragment was carried out for 30 cycles each consisting of 1 min. at 94°C for denaturation, 2 min. at 55°C for annealing of the primers, and 2 min. at 72°C for extension. The resulting amplified HA gene fragments were electrophoresed on agarose gels, purified from the gel using a GeneClean kit (Bio 101, Inc.), and ligated into a plasmid designed to accept PCR-generated fragments (pCRII; Invitrogen). Thus, plasmids pB142, pB144, and pB330, which contain the HA0, HA1, or HA2 gene fragments, respectively, were obtained.

The HA gene fragments were removed from plasmids pB142, pB144, and pB330 with *Sma*I and *Kpn*I restriction enzymes and then subcloned by standard recombinant DNA techniques (Sambrook et al., 1989) into the AcNPV transfer plasmid pMGS12. The pMGS12 plasmid contains, from 5' to 3', the AcNPV polyhedron promoter, an ATG initiation codon, the sequence for a cleavable signal peptide from a 61,000 molecular weight baculovirus glycoprotein (61 K), *Sma*I and *Kpn*I restriction enzyme cloning sites, and a TAA universal stop codon sequence. Flanking these regulatory regions is DNA from the EcoRI I fragment from the AcNPV genome (Summers and Smith, "A manual of methods for baculovirus vectors and insect cell culture procedures". Texas Agricultural Experimental Station Bulletin No. 1555 (1987). The cloned HA PCR fragments were excised from the pCRII cloning vector with *Sma*I and *Kpn*I, purified with agarose gel electrophoresis and the GeneClean kit, and ligated into pMGS12 that had also been digested with *Sma*I and KpnI. The resulting AcNPV transfer plasmids, pB879, pB1201, and pB1205, contained the coding regions for HA0, HA1, or HA2, respectively, linked in frame with the cleavable baculovirus signal peptide from the 61K gene and the polyhedron promoter. The pB879, pB1201; and pB1205 AcNPV transfer plasmids may be used to fuse HA0, HA1, or HA2 to any gene of interest.

The second step in the construction of HA-CEA fusion gene transfer plasmids was to insert the CEA coding sequences into the HA-encoding constructs. Restriction endonuclease recognition/cleavage sites for *Sma*I and *Kpn*I were placed at both ends of the C EA gene through PCR amplification of plasmid pA9080. The 5' PCR primer, O-649, begins 82 base pairs from the 5' end of the gene, deleting the natural CEA signal peptide sequence. The 3' PCR primer, 0-650, was designed to delete the last 72 basepairs at the 3' end of the gene which codes for the hydrophobic C-terminal region sequence. Amplification of the CEA gene fragment was carried out for 30 cycles, each consisting of 1 min. at 94°C for denaturation, 2 min. at 55°C for reannealing, and 2 min. at 72°C for extension. The resulting amplified CEA gene fragment was electrophoresed on an agarose gel, purified with the GeneClean procedure, and ligated Into pCRII (Invitrogen) according to the man ufacturers' instructions. The resulting plasmid, pB806, contains the CEA gene without its natural signal peptide, C-terminal hydrophobic domain, or stop codon, but with both *Sma*I and *Kpn*I sites at both ends of the gene.

A large-scale plasmid prep was performed with the pB806 plasmid, and the DNA was digested either with *Sma* I or *Kpn* I. The CEA-encoding fragments were purified with agarose gel electrophoresis and the GeneGlean kit, and the purified fragments were ligated into each of the three HA-encoding constructs (pB879, pB1201, or pB1205) digested with the same restriction enzyme. For example, CEA-encoding fragments with SmaI-cut ends were ligated into the HA0-, HA1-, and HA2-encoding constructs (pB879, pB1201, and pB1205, respectively) cut with *Sma*I to create plasmids pB1250, pB1555, and pB1584, respectively. CEA-encoding fragments with *Kpn*I-cut ends were ligated into the HA0-, HA1-, and HA2-encoding constructs cut with KpnI to create pB1264, pB1564, and pB1593. insertion of the CEA gene at the Smal site placed the CEA coding sequences downstream of the HA coding sequences. For all constructs, the PCR primer were designed such that the EA gene was inserted in-frame with HA, and the fusion gene translation would be terminated at the universal translation termination signal (TAATTAATTAA) (Sequence ID No. 4) in the pMGS12 vector sequences downstream of the *Kpn*I site.

This construct may be improved by deletion of intervening amino acids, either between the signal peptide and HAO, as described below, or between the HAO and the fusion gene, to enhance folding and immunogenicity.

### Formulation and Packaging of Vaccines

The rHA can be formulated and packaged, alone or in combination with other influenza antigens, using methods and materials known to those skilled in the art for influenza vaccines. I n a preferred embodiment, HA proteins from two A strains and one B strain are combined to form a multivalent vaccine.

In a particularly preferred embodiment, the HAs are combined with an adjuvant, in an amount effective to enhance the immunogenic response against the HA proteins. At this time, the only adjuvant widely used in humans has been alum (aluminum phosphate or aluminum hydroxide). Saponin and its purified component Quil A, Freund's complete adjuvant and other adjuvants used in research and veterinary applications have toxicities which limit their potential use In human vaccines. However, new chemically defined preparations such as muramyl dipeptide, mono-phosphoryl lipid A, phospholipid conjugates such as those described by Goodman-Snitkoff et al. J. Immunol. 147: 410-415 (1991) encapsulation of the protein within a proteoliposome as described by Miller et al., J. Exp. Med. 176:1739-1744 (1992), and encapsulation of the protein In lipid vesicles such as Novasome™ lipid vesicles (Micro Vescular Systems, Inc., Nashua, NH) should also be useful.

In the preferred embodiment, the vaccine is packaged In a a single dosage for immunization by parenteral (i. e., intramuscular, intradermal or subcutaneous) administration or nasopharyngeal (i.e., intranasal) administration. The effective dosage is determined as described in the following examples. The carrier is usually water or a buffered saline, with or without a preservative. The antigen may be lyophilized for suspension at the time of administration or in solution.

The carrier may also be a polymeric delayed release system. Synthetic polymers are particularly useful in the formulation of a vaccine to effect the controlled release of antigens. An early example of this was the polymerization of methyl methacrylate into spheres having diameters less than one micron to form so-called nano particles, reported by Kreuter, J., Microcapsules and Nanoparticles in Medicine and Pharmacology, M. Donbrow (Ed). CRC Press, p. 125-148. The antibody response as well as the protection against infection with influenza virus was significantly better than when antigen was administered in combination with aluminum hydroxide. Experiments with other particles have demonstrated that the adjuvant effect of these polymers depends on particle size and hydrophobicity.

Microencapsulation has been applied to the injection of microencapsulated pharmaceuticals to give a controlled release. A number of factors contribute to the selection of a particular polymer for microencapsulation. The reproducibility of polymer synthesis and the microencapsulation process, the cost of the microencapsulation materials and process, the toxicological profile, the requirements for variable release kinetics and the physicochemical compatibility of the polymer and the antigens are all factors that must be considered. Examples of useful polymers are polycarbonates, polyesters, polyurethanes, polyorthoesters and polyamides, particularly those that are biodegradable.

A frequent choice of a carrier for pharmaceuticals and more recently for antigens is poly (d,I-lactide-co-gly-colide) (PLGA). This Is a biodegradable polyester that has a long history of medical use in erodible sutures, bone plates and other temporary prostheses, where it has not exhibited any toxicity. A wide variety of pharmaceuticals including peptides and antigens have been formulated into PLGA microcapsules. A body of data has accumulated on the adaptation of PLGA for the controlled release of antigen, for example, as reviewed by Eldridge, J.H., et al. Current Topics in Microbiology and Immunology. 1989, 146: 59-66. The entrapment of antigens in PLGA microspheres of 1 to 10 microns in diameter has been shown to have a remarkable adjuvant effect when administered orally. The PLGA microencapsulation process uses a phase separation of a water-in-oil emulsion. The compound of Interest is prepared as an aqueous solution and the PLGA is dissolved in a suitable organic solvents such as methylene chloride and ethyl acetate. These two Immiscible solutions are co-emulsified by high-speed stirring. A non-solvent for the polymer is then added, causing precipitation of the polymer around the aqueous droplets to form embryonic microcapsules. The microcapsules are collected, and stabilized with one of an assortment of agents (polyvinyl alcohol (PVA), gelatin, alginates, polyvinylpyrrolidone (PVP), methyl cellulose) and the solvent removed by either drying in vacuo or solvent extraction.

The present invention will be further understood by reference to the following nonlimiting examples.

### Example 1: Propagation and Purification of Influenza Viruses.

The following influenza vaccine strains were obtained from the FDA in chicken egg allantoic fluid:
A/Beijing/353/89-like(H3N2)
A/Beijing/32/92-like(H3N2)
A/Texas/36/91-like(H1N1)
B/Panama/45/90

To propagate the original stock of influenza virus obtained from the FDA, MDCK cells were Infected in the presence of TPCK-treated trypsin (Sigma Chemical Co., St. Louis, MO) and fetal bovine serum concentrations optimized to produce the highest titers of first passage virus. The MDCK cells were infected with the influenza strains at a low multiplicity of infection (0.1 to 0.5) as determined by a standard HAassay (Rosen, "Hemagglutination with Animal Viruses" in Fundamental Techniques in virology, ed. K. Habel and N.P. Salzman, pp. 276-28 (Academic Press, New York 1969)). The infected cells were incubated at 33°C for 48 h. and mediawas assayed for virus production using the bemagg luti nation activity assay. The conditions yielding the highest HA activity were used to prepare large stocks of Influenza virus. The optimum concentrations of TPCK trypsin and fetal bovine serum for the above influenza viruses are listed in Table 1.

**Table 1. Optimum Concentration of TPCK Trypsin and Fetal Bovine Serum.**

| | **A/Beijing/ 353/89** | **A/Beijing/ 32/92** | **A/Texas/ 36/91** | **B/Panama /45/90** |
|---|---|---|---|---|
| **% Fetal Bovine Serum** | 0.25% | 0.25% | 0.25% | 5.0% |
| **Amount TPCK Treated Trypsin** | 45 µ/ml | 45 µg/ml | 45 µ/ml | 3 µ/ml |

Purification of influenza Virus: Virus was harvested 24-28 hours post infection from 10 T175 tissue culture flasks by clarifying media (1,000 x g for 10 minutes) of influenza infected MDCK cells. The virus was pelleted from the media at 100,000 x g for 1 hour. The resulting viral pellet was resuspended in 1 ml phosphate buffered saline (PBS) pH 7.4 and centrifuged through a 20 ml 20-60% (W/v) sucrose gradient in PBS. The influenza virus band was harvested from the 40-45% sucrose region of the gradient, diluted with PBS and pelleted at 100,000 x g. The purified virus pellet was resuspended in 0.5 ml PBS stored at -70°C.

### Example 2: Cloning of Influenza A/Texas/36/91 Ha gene.

A specific example of the cloning step for one of the influenza HA genes is shown in Figure 2. Viral RNA was extracted as described above from influenza A/Texas/36/91, obtained from the CDC. The universal primer complementary to the 3' end of influenza RNA segments 5'-AGCAAAAGCAGG-3' (SEQ ID NO. 1) was used with murine Meloney Leukemia Virus (M-MuLV) reverse transcriptase to produced influenza cDNAs. Purified viral RNA or mRNA (5 µg) was used as a template to make cDNA utilizing M-MuLV reverse transcriptase supplied in the First-Strand cDNA Synthesis Kit by Pharmacia Inc. The primer used for cDNA of viral RNA from influenza A strains was a synthetic oligonucleotide primer (5'-AGCAAAAGCAGG-3') (SEQ ID NO. 1), which is homologous to the 3' end of all HA gene virion segments.

Amplification of HA genes from cDNA was done by polymerase chain reaction (PCR) using standard reaction conditions (Gene Amp kits; Cetus/Perkin Elmer, Norwalk, CT). The PCR reaction mixture (100 µl) contained 20 pmol of primers specific for 5' and 3' ends of the HA gene of Influenza A (H3) or A (H1) or influenza B strains as determined by consensus sequences found in GenBank DNA data files, as shown in Table 2. Amplification was carried out for 30 cycles with each cycle consisting of 1 minute of denaturation at 94°C, 2 minutes at 55°C for reanealing and 3 minutes at 72°C for extension. The PCR products were analyzed on 0.8% agarose gels for correct size before cloning.

PCR primers from the 5' end of the HA gene: 5'-GGG GGT ACC CCC GGG AGC AAA AGC AGG GGA AAA TAA AAA-3' (SEQ ID NO. 2) and 3' end of the HA gene: 5'-GA AAC GTC ACG TCT TAT ACG/T TAG/T ACT CCA TGG CCC-3' (SEQ ID NO. 3) were used in the PCR to yield the full length HA gene.

A new 5' PCR primer was designed from the 5' end of the gene: 5' end minus signal sequence: 5'-GGG GGT ACC CCC GGG GAC ACA ATA TGT ATA GGC TAG CAT-3' (SEQ ID NO. 4) and the 3' end of the gene: 5'-GA AAC GTC ACG TCT TAT ACG/T TAG/T ACT CCA TGG CCC-3' (SEQ ID NO. 5). These were used in PCR to yield the HA gene minus the signal peptide sequence. This was then inserted into the TA vector cleaved with *Kpn*I. The 61K signal peptide for baculovirus expression and the polyhedrin promoter were then inserted into the TA vector containing the HA gene minus influenza signal peptide sequence. The resulting baculovirus recombination vector contains the polyhedrin promoter, 61K baculovirus signal peptide, and HA gene for Influenza A/Texas/36/91.

HA genes from influenza B strains were cloned from total messenger RNA (mRNA) extracted from MDCK cells infected with the influenza B-strain B/Panama/45/90. Total RNA was prepared from 5 T175 flasks of infected cells. The harvested cells were lysed in the presence of guan idinium thiocyanate and total cell RNAwas purified as described above. Total mRNA was extracted from cellular RNA using Oligo-(dT)-cellulose spun columns as described above.

The primer used for mRNAfrom influenza B strains was a random oligonucleotide DNA primer (Pharmacia, Inc.).

**Table 2. Primers Used for PCR Amplification.**

| A/Beijing/32/93 | |
|---|---|
| 5' end gene (SEQ ID NO. 27) | |
| 5' end minus HA signal peptide (SEQ ID NO. 28) | |
| 3' end (SEQ ID NO. 29) | |
| | |

| A/Texas/36/91 | |
|---|---|
| 5' end gene (SEQ ID NO. 2) | |
| 5' end minus HA signal peptide (SEQ ID NO. 4) | |
| 3' end (SEQ ID NO. 3) | |
| B/Panama/ 45/90 | |
| 5' end gene (SEQ ID NO. 30) | |
| S' end minus HA signal peptide (SEQ ID NO. 31) | |
| 3' end (SEQ ID NO. 32) | |

An example of cDNA synthesis products used virus A/Texas/36/91 viral RNA as template. The location of the cDNA segments that code for the influenza proteins could be determined as follows. Purified viral RNA was combined in the reaction mixture with the universal single stranded DNA primer 5'-AGCAAAAGCAGG-3' (SEQ ID NO. 1). This primer is complementary to the 3' end of onfluenza virion segments, as described above. The reaction also contained the addition of [α-³²P]dCTP tovizualize the cDNA products wich were separated on 1.5% alkaline hydrolysis gel 5Maniatis, et al, 1982) and exposed to X-OMAT-AR film.

### Example 3: Cloning HA Genes Into Bacterial Plasmids.

The PCR amplified rHA genes were cloned into a pUC-like plasmid vector using the TA Cloning System (Invitrogen, Inc.). The presence of HA genes were verified by restriction enzyme digest analysis of plasmid DNA purified by standard procedures (Maniatis, et al, 1982). The 5' end of the rHA genes were then analyzed by DNA sequencing and new primers were designed to remove the sequences coding for the hydrophobic signal peptides at the N-terminus HA proteins. The specific 5' and 3' oligonucleotide primers listed in Table 2 were then used to amplify cDNA products by PCR and cloned into *E*. *coli TA* plasmid vectors (Invitrogen, Inc.) using standard cloning methods. The resulting DNA clones contained coding sequences for the mature HAs.

The rHA genes from A/Texas/36/91, A/Beijing/353/89, A/Beijing/32/92, and B/Panama/45/90 were subcloned by standard procedures (Maniatis et al, 1982) into baculovirus expression vectors. The HA genes were removed from the TA cloning plasmids with the appropriate restriction enzymes and the purified HA DNA fragment inserted into a baculovirus recombination plasmid. The resulting bacterial clones were screened for ampicillin resistance and then cut with restriction enzymes to release the inserted HA gene to confirm is presence. The recombination plasmids containing HA genes were purified on cesium chloride-ethidium bromide gradients (Maniatis, et al, 1982). The 5' end of the plasmids were sequenced to determine the presence of the correct baculovirus signals (AcNPV polyhedrin promoter, ATG translational start signal and baculovirus signal peptide sequence) and proper HA coding sequence in the correct reading frame. The DNA sequences at the 5' end of the HA genes and flanking AcNPV polyhedrin promoter and baculovirus signal peptide (first 18 amino acids of each amino acid sequence) are shown as SEQUENCE LISTINGS.

SEQ ID NO. 6 encodes the 5' end sequence of the HA gene for A/Beijing/32/92 (sequence range 1-461). SEQ ID NO. 7 is the corresponding amino acid sequence (beginning at the start codon "ATG" [nucleotide 21] of SEQ ID NO. 6). The amino acid sequence of the 61 K signal peptide is set forth in SEQ ID NO. 7 as amino acids 1-18.

SEQ ID NO. 8 encodes the 5' end sequence of the HA gene for A/Texas/36/91 (sequence range 1-481). SEQ ID NO. 9 is the corresponding amino acid sequence (beginning at the start codon "ATG" [nucleotide 21] of SEQ ID NO. 8). The amino acid sequence of the 61 K signal peptide is set forth in SEQ ID NO. 9 as amino acids 1-18.

SEQ ID NO. 10 encodes the 5' end sequence of the HA gene for B/Panama/45/90 (sequence range 1-434). SEQ ID NO. 11 Is the corresponding amino acid sequence (beginning at the start codon "ATG" [nucleotide 21] of SEQ ID NO. 10). The amino acid sequence of the 61 K signal peptide is set forth In SEQ ID NO. 11 as amino acids 1-18.

in SEQ ID NOs 6, 8, and 10, nucleotides 1-20 are the 3' end of the polyhedrin promoter, nucleotides 21-74 encode the 61 K signal peptide, and nucleotides 75 to the end encode the 5' end of the HA gene.

### Example 4: Expression of Recombinant HA In Insect cells.

The chimeric recombination plasmids containing cloned HA genes were purified and 2 µg was mixed with 1 µg AcNPV wild type DNA. The DNAs were co-precipitated with calcium and transfected into *S*. *frugiperda* cells using standard procedures (Smith, Summers, and Fraser, Mol. and Cell. Biol. 3:2158-2165 (1983)). Recombinant baculoviruseswere identified on the basis of plaque morphology then further purified by additional rounds of plaque-purification. Plaque-purified recombinant baculoviruses are screened for expression of rHA and a single baculovirus expression vector was selected for further development.

*S. frugiperda* cells were infected with a baculovirus vector containing the HA gene from the Influenza strain: B/Panama/45/90. At 24, 48, and 72 hours post infection, 1 X 10⁶ cells were pulsed with 25 µCl [³⁵S]methionine for 15 minutes to label proteins being synthesized. The cells were collected and the proteins separated on an 11 % polyacrylamide gel in the presence of 0.1 % SDS. The radiolabeled proteins were detected by exposure to X-OMAT-AR film. The location of protein standards and their size in kilodaltons, (kd) indicated that the 85 kd recombinant HA protein is one of the major proteins being synthesized in the cells at 48 hours and 72 hours post infection.

### Example 5: Production and Purification of Recombinant HA

The baculovirus expression vector A8611, which contains the gene for influenza A/Beijing/353/89, produced essentially as described above for A/Beijing/32/92 hemagglutinin under the control of the polyhedrin promoter, was used to Infect *S*. *frugiperda* cells. Cells were grown at 27°C to a density of 1 x 10⁶ cells/mL in TNMFH media (Glbco BRL, Gaithersburg, MD) supplemented with 10% fetal bovine serum, and Infected at a multiplicity of infection (MOI) of 1 with the A8611 recombinant baculovirus. During infection the Influenza A/Beijing/353/89 hemagglutinin is produced under the transcriptional control of the baculovirus polyhedrin promoter. Cells are harvested 72 hours post-infection by centrifugation for 15 minutes at 3,400 x g, and washed by resuspension in serum-free TNMFH media followed by centrifugation for 30 minutes at 10,400 x g. The supernatant is decanted, and infected cell pellets are stored at -70°C.

A process was developed in which the recombinant HA is selectively extracted from the infected cells under conditions that do not denature the antigen. Unless noted, all extraction steps are performed at 4°C. The cell pellet from 0.5 L of culture (approximately 5 x 10⁸ cells) was disrupted for 2 minutes in 40 mL of ice-cold 30 mM Tris-HCl, pH 8.4, 25 mM LiCl, 1% (v/v) Tween-20, 1 mg/mL leupeptin, using a Polytron™ homogenizer (Brinkmann Instruments Inc. Westbury, NY). The homogenate was centrifuged for 30 minutes at 9,200 x g. The supernatant was discarded, and the pellet collected. This step removes soluble and peripheral membrane proteins from the insect cells without extraction of integral membrane proteins like rHA. To extract the rHA the pellet was homogenized for 2 minutes at a setting of 4 in 40 mL of ice-cold 30 mM Tris, 10 mM ethanolamine, pH 11, 25 mM LiCl, 2% Tween-20. After a 60 minute incubation on ice, the pH of the homogenate was adjusted to 8.4 with 1 N HCl, and insoluble material was removed by centrifugation for 30 minutes at 9,200 x g. The supernatant containing the soluble rHA was decanted, and the pH was checked and, if necessary, adjusted to 8.4 at room temperature. The Insoluble material was resuspended in 40 mL of water for analysis. The HA integral membrane protein was solubilized under the high pH, Tween-20 detergent conditions and remains in solution after the pH, is dropped.

Proteins were analyzed by SDS polyacrylamide gel electrophoresis. Samples were disrupted in a boiling water bath for 10 minutes in the presence of 2% sodium dodecyl sulfate (SDS) and 5% betamercaptoethanol, then electrophoresed on an 11 % polyacrylamide gel in the presence of 0.1 % SDS, then stained with Coomassie blue.

A chromatography purification process was developed to purify recombinant HA which results in a highly purified recombinant HA antigen that is non-denatured and suitable as a component of an influenza vaccine for human use. The following procedure was used to purify the A/Beijing/353/89 HA from *S. frugiperda* cells infected with the recombinant virus A8611.

The chromatography gel matrices used to purify HA from 0.5 L of infected *S. frugiperda* cells were 30 mL Pharmacia DEAE Sepharose Fast Flow (in a Pharmacia C16/20 column) and a4 mL Pharmacia Lentil Lectin Sepharose 4B (in a Pharmacia C10/10 column). The outlet of the DEAE column is connected to the inlet of the lentil lectin column, and the S/N 2 cell extract prepared as described above was applied to the coupled columns at a flow rate of 1 mL/ minute. The columns were washed with 30 mM Tris-HCl, pH 8.4, 25 mM LiCl, 0.5% Tween-20 until the UV absorption at 280 nm of the lentil lectin effluent returns to baseline. Under these conditions most of the contaminating proteins bind to DEAE but recombinant HA flows through the column. The remaining contaminants pass through the lectin column and glycosylated rHA binds to the lentil lectin affinity matrix. The DEAE column is disconnected, and the lectin column is washed with another 40 mL of 30 mM Tris-HCl, pH 8.4, 25 mM LiCl, 0.5% Tween-20. Next, the lectin column is washed with 40 mL of 30 mM Tris-HCl, pH 8.4, 25 mM LiCl, 0.4% (v/v) sodium deoxycholate (DOC). This step replaces the Tween-20 detergent with a detergent, like DOC, that can be removed from the protein by dialysis. Recombinant HA is then eluted from the lectin column with approximately 20 mL of 40 mL of 30 mM Tris-HCl, pH 8.4, 25 mM LiCl, 0.4% (v/v) sodium deoxycholate containing 0.3 M a-D-methyl mannoside. Results are analyzed by 11 % PAGE.

Due to the genetic variability of influenza HA proteins, the details of the above purification process may vary with each unique recombinant HA protein. For example, the rHA may bind to the DEAE ion exchange column instead of flowing through. Should this occur, the rHA would be removed from the DEAE column with by washing the column with buffer containing higher concentration of LiCl, NaCl, or other salts.

To remove the DOC detergent and other buffer components, the eluate from the lectin column containing the purified rHA was dialyzed against phosphate buffered saline, pH 7.5 (PBS). The purified recombinant HA was at least 95% pure as determined by analysis on SDS polyacrylamide gels.

### Example 6: Analysis of rHA Protease Resistance.

Mature HA assembles into trimeric structures which are resistant to a variety of proteases, including trypsin, that degrade HA monomers (Murphy and Webster, 1990). Resistance to trypsin treatment can therefore be used as an assay for functional trimer formation. The following procedure was used to study resistance of rHA to protease treatment.

Two aliquots of purified rHA (A/Beijing/353/89) at 60 µg/mL were incubated on ice for 30 minutes in 30 mM Tris-HCl, pH 8.4, 150 mM NaCl, in the presence and absence of 50 µg/mL TPCK-treated trypsin. The reaction was stopped by the addition of 57.4 mM phenyl methyl suffonyl fluoride in isopropanol to a final concentration of 1 mM. Aliquots of each sample were denatured by boiling in 3% SDS under reducing conditions, electrophoresed on 11.5% polyacrylamide gels, and transferred to nitrocellulose filter using standard Western blotting procedures. The HA polypeptides were detected using guinea pig anti-HA serum prepared against purified rHA and a goat anti-guinea pig IgG alkaline phosphatase conjugate.

Untreated rHA migrates at the size of the HA precursor (HA0). Protease treatment results in two major bands that migrate at the sizes predicted for Influenza hemagglutinin HA1 and HA2. The results show that trypsin cleaves the rHA protein once to produce two polypeptides that are the sizes predicted for HA1 and HA2. No further proteolytic processing occurs. These results demonstrate that rHA purified by the above process is resistant to degradation by protease. This property is consistent with purified rHA being in the form of trimers.

### Example 7: Immunogenicity of rHA using standardized Mouse Potency Assay.

One approach to measure immunogenicity of an antigen is to determine the quantity necessary to induce a detectable antibody response in mice (mouse potency assay). A standardized mouse potency assay is usedto measure the immunogenicity of rHAO vaccine. Groups of 5-10 mice are immunized once with vaccine containing serial dilutions of rHA, i.e., 0.500 µg, 0.1 µg, 0.02 µg, and 0.004 µg purified rHA. Sera are collected 28 days post immunization and antibodies against the rHA antigen measured in a standard enzyme-linked immunological solid-phase assay (ELISA) in 96 well microtiter plates. A mouse has seroconverted if the OD450 at a 1:100 dilution of the 28 day antisera is greater than three standard deviations above the mean of the OD450 of mouse pre-immune sera. The effective dosage of vaccine needed to seroconvert 50% of the mice (ED50) is a measure of the immunogenicity of the antigen.

For example, four groups of 10 mice are immunized once with either 0.1 µg, 0.02 µg, 0.004 µg, or 0.0008 µg (5-fold dilutions) of rHAO vaccine. Sera are collected 28 days post immunization and measured against each rHAO antigen in the vaccine for seroconversion in an ELISA assay. The dosage needed to seroconvert 50% of the mice (ED₅₀) is calculated and a minimum ED₅₀ established for each rHAO antigen.

Preliminary data shows that a single dose of 0.004 µg of rHAO will seroconvert at least 50% of the mice.

### Example 8: Administration of rHA in combination with an Adjuvant and comparison with available Influenza vaccines.

The mouse potency of purified rHA from influenza A/Beijing/353/89 was tested with alum or without alum (neat) and compared to a commercial Influenza vaccine, FLUZONE® (Connaught Laboratories, Inc. Swiftwater, PA) which contains the A/Beijing/353/89 strain of influenza. Vaccine was administered in a dosage of 0.5 µg, 0.1 µg, 0.02 µg, and 0.04 µg. The mice were boosted at day 28 with the doses of purified rHA described above. On day 42 sera were collected and titered in an ELISA assay for IgG anti-HA antibodies.

The results are shown in Figure 3. In the absence of adjuvant, only a dosage of 0.5 µg induced production of significant antibody titer (200,000). In the presence of adjuvant, dosages of as little as 0.004 µg of rHAO produced significant antibody. The animals immunized with rHA (neat) produced approximately the same levels of anti-HA antibodies as the commercial vaccine. Alum increased the immunogenicity of rHA, and anti-HA titers were generated that were 10-fold or higher than without adjuvant.

In summary comparison of the immunogenicity of purified rHAOs with an influenza whole virion vaccine, (FLUZONE®, Connaught Laboratories, Inc., Swiftwater, PA), demonstrates that rHAO elicits a similar immune response in mice over a period of 42 days. Adsorption of the rHAO to alum significantly increases the immunogenicity of the purified rHAO in mice, as measured by the assay described in Example 7. The combination with alum elicits IgG hemagglutinin antibodies that are higher than the Fluzone® influenza vaccines.

### Example 9: Hemagglutination Inhibition Studies.

Hemagglutination inhibition (HAI) antibodies bind to three of four known epitopes on hemagglutinin and block the ability of influenza to agglutinate red blood cells (Wilson et al, "Structure of the hemagglutinin membrane glycoprotein of influenza virus at 3A° resolution". Nature, 289:366-378 (1981)). These antigenic determinants are clustered around the sialic acid receptor binding site on hemagglutinin trimers. Antibodies against these sites will neutralize virus infectivity (Weis, et al., "Structure of the influenza virus hemagglutinin complexed with its receptor, sialic acid", Nature 333:426-431 (1988)). The titer and specificity of HAI antibodies are an important measure of the potential for an influenza vaccine to protect against infection with like and related strains of Influenza.

Studies were conducted in mice comparing the ability of purified rHAO from A/Beijing/353/89 and FLUZONE® (Connaught Laboratories, inc., Swiftwater, PA) to elicit HAI antibodies. Groups of 5 mice were injected on days 0 and 28 with 0.5 µg, 0.1 µg, 0.02 µg, or 0.004 µg of rHAO or three times these quantities of FLUZONE® hemagglutinin so that equal levels of recombinant or viral A/Beijing/353/89 hemagglutinin were administered. For example, mice in the highest dose group were immunized with 1.5 µg of FLUZONE® hemagglutinin (0.5 µg of hemagglutinin from each strain) and 0.5 µg rHAO. The presence of additional hemagglutinin antigen in FLUZONE® from two other influenza strains may result In some cross-reactive antibodies.

Anti-hemagglutinin antibodies (hemagglutinin IgG) were measured in a standard dilutional ELISA against purified rHAO. HAI antibodies were measured against 4 hemagglutinin units of the following antigens: whole influenza A/ Beijing/353/89 virus (A/Bei), purified rHAO A/Beijing/353/69 antigen, and FLUZONE®. The HAI titer is the reciprocal of the highest dilution of antisera which inhibits the agglutination of chicken red blood cells by 50%.

Table 3 summarizes serum hemagglutinin IgG and HAI titers In the mice at day 42. High levels of anti-hemagglutinin antibodies were produced with the recombinant rHAO vaccine. These were about ten fold higher titers than FLUZONE®. Most significant is that the rHAO vaccine produced good titers of antibodies that block agglutination of red blood cells by the A/Beijing/353/89 virus and rHAO antigens. Thus, the rHAO vaccine produced HAI antibodies that recognized equally well the immunogen and the influenza A/Beijing virus. The lower HAI titers against FLUZONE® may be due to the inability of the antisera to block agglutination by the other two strains of hemagglutinin in the FLUZONE® vaccine. In contrast, FLUZONE® immunized mice produce high HAI antibodies when measured only against itself. The HAI liters against influenza A/Beijing/353/89 virus and the rHAO antigen were considerably reduced. Similar patterns were observed in the mice in the lower dose groups.

**Table 3. HAI Titers against rHAO and FLUZONE^{®}**

| rHAO A/Bei (day 42) | | | | | FLUZONE^{®} (day 42) | | | |
|---|---|---|---|---|---|---|---|---|
| HA IgG | | HAI | | | HA IgG | HAI | | |
| Mouse # | rHAO | A/Bei | rRAO | FLUZONE | rHAO | A/Bei | rHAO | FLUZONE |
| 1 | 4,096,000 | 1,920 | 960 | 15 | 256,000 | <10 | <10 | 600 |
| 2 | 4,096,000 | 480 | 480 | 15 | 512,000 | 120 | 120 | 600 |
| 3 | 8,192,000 | 1,920 | 960 | 15 | 256,000 | 60 | 60 | 300 |
| 4 | 4,096,000 | 960 | 960 | 30 | 128,000 | 30 | 30 | 400 |
| 5 | 4,096,000 | 1,920 | 960 | 60 | 512,000 | 80 | 80 | 400 |
| **MEAN** | **4,915,000** | **1,440** | **864** | **27** | **332,800** | **58** | **58** | **460** |

These data also suggest that there are ganatic differences betwin the influenza A/Beijing/359/89 stain in FLUZONE@ and this same strain of influenza obtained from the FDA and passaged once in eggs prior to using the HAI assay. The fact that antibodies produced in response to the recombinant HAO cloned from influenza A/Beijing/ 353/89 blocks agglutination of red blood cells by this strain of influenza as well as itself is good evidence that there were no genetic changes during the cloning process that effected the sialic acid receptor binding site on the purified rHAO antigen.

### Example 10: Formulation and Clinical Efficacy of a 1993/1994 Influenza Vaccine.

A series of human clinical trials was conducted to characterize the safety and immunogenicity in humans of an experimental influenza vaccine containing recombinant HA and to obtain preliminary data regarding the protective efficacy of such a vaccine against natural infection during an epidemic season. The results demonstrate that vaccines containing the recombinant influenza hemagglutinin (rMAO), produced in accordance with the methods described herein surprisingly caused fewer local adverse reactions and provided an equivalent or superior protective immune response when compared to a commercially available, licensed attenuated flu vaccine produced in eggs.

### MATERIALS AND METHODS

**Vaccines.** The recombinant HA vaccines used in this study contained full length uncleaved HA (HAO) glycoprotein from the influenza A/Beijing/32/92 (H3N2) virus. Recombinant HAO (rHAO) was produced in cultures of Lepidopteran (insect) cells following exposure to a baculovirus vector containing cDNA inserts encoding the HA gene. The expressed protein was purified under non-denaturing conditions to >95%, as measured by quantitative scanning densitometry of the bulk antigen electrophoresed on sodium dodecyl sulfate-polyacrylamide gels. The identity of the peptide was confirmed by amino acid analysis, N-terminal sequencing and Western blot analysis with antiinfluenza A/Beijing/ 32/92 sera. The rHAO vaccines contained a specified amount of the synthetic HA antigen eitherdissolved in a phosphate-buffered saline solution oradsorbed to aluminum phosphate (alum) adjuvant in the form of a gel suspension. The licensed trivalent subvirion vaccine used in this study contained 15 µg/dose of each the HAs from influenza A/ Texas/36/91 (N1N1), A/Beijing/32/92 (H3N2) and B/Panama,45/90 viruses (FLUZONE™ attenuated flu vaccine produced in eggs, Connaught Laboratories, Swiftwater, PA).

**Clinical Studies.** Identical study protocols were approved by the institutional Review Boards of Saint Louis University and the University of Rochester. Healthy adults aged 18 to 45 years were enrolled at both institutions. Subjects were randomly assigned to receive one of the following five vaccine preparations in a double-blinded manner: (1) 15 µg rHAO, (2) µg rHAO plus alum, (3) 90 µg rHAO, (4) licensed trivalent inactivated influenza vaccine, or (5) saline placebo. Vaccines were administered by intramuscular injection in a volume of 0.5 ml. To allow for an initial assessment of the safety of the three vaccine preparations containing rHAO, the first 25 subjects to be vaccinated were randomized (i.e., 5 persons per study arm) independently of the other subjects and closely monitored by phone contact for 48 hours post-vaccination before proceeding with the remaining vaccinations. All subjects were instructed to fill out a daily report card of adverse reactions, including both local and systemic symptoms, during the first 6 days post-vaccination. Symptoms were self-graded as mild, moderate or severe in nature. Oral temperatures were taken and recorded by participants if they felt feverish. If present, localized swelling or erythema at the injection site was graded according to whether the area was less than or greater than the size of a quarter in diameter, respectively. All vaccinations were performed during the last week of November and first week of December, 1993. Serum specimens were obtained from each subject at the time of vaccination, 3 weeks post-vaccination, and once again in late March or April 1994 at least 2 to 3 weeks after influenza viruses were no longer circulating in the local communities. Volunteers at each institution were instructed to contact the study center if they experienced an influenza-like illness during the winter influenza epidemic season. An influenza-like illness was defined as the presence of any respiratory symptom(s) of two days or greater duration accompanied by fever and/or systemic symptoms of myalgias or chills. Subjects who reported influenza-like symptoms had nasal and pharyngeal swabs obtained for virus culture and identification. Clinical specimens were given coded identification numbers and processed in a blinded fashion.

**Serology.** For each type of serologic assay, all specimens from both institutions were tested in one batch by a single laboratory. Hemagglutination Inhibition (HAI) antibodies to influenza A/Beijing/32/93 (H3N2) virus antigen were measured in sera by a standard microtiter assay, following removal of nonspecific inhibitor with preceptor destroying enzyme and of cold, agglutinins by hemadsorption at 4° C. The titer was defined as the highest serum dilution that completely prevented hemagglutination by 4 antigen units of virus, using 1:4 as the starting dilution. Serum HA-specific immunoglobulin G (IgG) antibodies were measured by enzyme-linked immunosorbent assay (ELISA), using purified rHAO from influenza A/Beijing/32/92 (H3N2) as the coating antigen. The sequence of reagents from solid phase outward consisted of (1) purified rHAO antigen, (2) serum specimen, (3) alkaline phosphate-conjugated goat anti-human IgG, and (4) p-nitrophenyl phosphate disodium substrate. The ELISA titer was expressed as the highest dilution at which the optical density of the antigen-containing well was at least twice that of the corresponding control well without antigen. Neutralizing antibodies were measured using the microneutralization assay previously described by Treanor, J.J., and Betts, R.F., J. Infecf. Dis. 168:455-459 (1993). In brief, serial dilutions of heat-inactivated sera were mixed with approximately 100 TCID₅₀ of influenza A/Beijing/32/92 (H3N2) virus and incubated at 37°C for 1 hr. The virus-sera mixture was then adsorbed to confluent monolayers of Madin-Darby canine kidney (MDCK) cells in 96-well plates for 1 hr at room temperature. The plates were washed to remove residual inoculum, refed serum-free Dulbecco's MEM with 2 µg/ml trypsin, and incubated in 5% CO₂ at 33°C for 72 hr. Cells were then fixed with methanol, and viral replication was assessed using a panel of murine monoclonal antibodies specific for the matrix and nucleoproteins of influenza A virus (Centers for Disease Control, Atlanta, GA), followed by alkaline phosphatase-conjugated anti-mouse IgG. The endpoint titer of the sera was defined as the highest dilution resulting in greater than 50% reduction in signal compared with nonneutralized control wells.

**Virology.** Viral cultures of nasopharyngeal swab specimens were performed at each institution by standard techniques. Specimens were inoculated in either MDCK or rhesus monkey kidney cells and incubated at 33°C for 14 days. Hemadsorption of cell monolayers was tested with 0.4% guinea plg erythrocytes. Influenza viruses were identified in hemadsorption positive cultures by HAI using H3-specific antisera (Centers for Disease Control).

**Statistical Analyses.** Reciprocal HAI, ELISA IgG and neutralizing antibody titers were logarithmically transformed for statistical analysis. A significant response to vaccination was defined as a fourfold or greater rise in antibody titer between the pre-vaccination and 3-week post-vaccination serum specimens. Laboratory evidence of influenza A (H3N2) virus infection was defined as the isolation of virus from nasopharyngeal secretions and/or a four-fold or greater increase in serum HAI antibody titer between the 3-week post-vaccination (preseason) specimen collected in December and the corresponding postseason specimen collected the following spring. Differences between vaccine groups were **analyzed** using Fisher's exact test to compare the proportions of subjects with adverse reactions, significant antibody responses or laboratory-confirmed influenza illness or infection, and analysis of variance (ANOVA) to compare post-vaccination mean reciprocal log₂ antibody titers. The modified Bonferroni's inequality and Tukey-Kramer tests were applied where appropriate to account for multiple possible comparisons.

### RESULTS

**Reactogenicity.** The rHAO vaccines used In this study were safe and well-tolerated. The frequency of adverse reactions did not appear to be influenced by changing the dose of rHAO antigen from 15 µg to 90 µg, but may have been slightly increased by the addition of alum. Localized erythema, pain and tenderness at the injection site were each reported significantly more frequently by recipients of licensed subvirion vaccine than by recipients of either 15 µg or 90 µg rHAO in saline. With the exception of one individual who experienced moderately severe pain, tenderness and stiffness in the arm following immunization with licensed vaccine, all symptoms were graded as mild in nature and were generally 1-2 days in duration. Localized erythema and/or induration, when present, was invariably less than the area of a quarter in size.

**Immunogenicity.** Baseline titers of serum HAI antibody to influenza A/Beijing/32/92 (H3N2) virus were less than or equal to 1:8 in 64 (50%) of the 127 subjects enrolled. Most subjects in each of the four vaccine groups had HA-specific serologic responses measured by HAI and ELISA (Table 4). Post-vaccination titers of serum HAI antibody were greater than or equal to 1:32 in all vaccine recipients with the exceptions of two persons given 15 µg rHAO and one given the licensed vaccine. Vaccination was likewise associated with the production of neutralizing antibody in the majority of volunteers. Mean rises In antibody titers and seroconversion rates tended to be slightly lower following immunization with 15 µg rHAO than with licensed vaccine, although these differences were not statistically significant. Antibody response to rHAO were not enhanced by the addition of alum. Subjects Immunized with 90 µg rHAO achieved post-vaccination mean HAI and ELISA IgG antibody titers that were two- to five-fold higher than in any of the other three vaccine groups (differences were statistically significant when comparing serum HAI titers).

**Protective Efficacy.** During the period of surveillance, there were a total of 28 influenza-like illnesses reported by 26 subjects. Four of these individuals (three of whom had received placebo and one of whom had been immunized with 15 µg rHAO) had influenza A (H3N2) virus isolated from nasopharyngeal cultures. Significant increases in HAI antibody titer to influenza A/Beijing/32/92 (H3N2) between preseason and postseason serum specimens were also present in three of the four culture-confirmed cases, but not in any other individuals who reported illness. The Ione rHAO recipient who subsequently developed laboratory-confirmed influenza illness had the positive culture obtained 31 days after immunization, and had seroconverted from a prevaccination HAI titer of less than 1:4 to a post-vaccination (preseason) titer of 1:32. Two additional placebo recipients and one volunteer immunized with licensed vaccine had serologic evidence of infection with influenza A (H3N2) virus during the epidemic season in the absence of clinical illness. Compared to all vaccinated subjects (or to all subjects who received any rHAO vaccine) as one group, a significantly larger proportion of placebo recipients had laboratory-confirmed influenza A (H3N2) Illness (p<.05) or infection (p<.005).

The above findings indicate that influenza vaccines containing purified rHAO antigen, prepared as described in the above-identified patent application, are welt-tolerated and capable of eliciting protective immune responses in human subjects. Even at a dose of 90 µg, the rHAO evaluated in this study was no more reactogenic than saline placebo, and caused significantly fewer local adverse reactions than did a licensed trivalent subvirion vaccine containing half as much (i.e., 45 µg) total HA antigen.

Neutralizing, HA-specific antibody responses to the 15 µg rHAO preparation were comparable to those elicited by subvirion vaccine, and were significantly improved by raising the dose of rHAO to 90 µg.

Overall rates of infection and illness resulting from natural exposure to the circulating epidemic strain of influenza A (H3N2) virus were significantly lower among vaccinated subjects than among placebo recipients. The data suggest that protective immunity conferred by rHAO, particularly when administered at high doses, is comparable or superior to that induced by currently available vaccines.

**Table 4: Serum antibody response in young adult subjects following immunization with vaccines containing purified recombinant hemagglutinin (rHAO) from influenza A/Beijing 32/92 (H3N2), licensed trivalent trivalent subviron containing 15 µg HA from A/Beijing/32/92 (H3N2), or saline placebo.**

| | HAI antibody | | | ELISA | IgG HA antibody | Neutralizing Microneutralization | antibody | |
|---|---|---|---|---|---|---|---|---|
| Vaccine (Number in group | HAI titer Pre | %with ≥ 4x rise Post | % with post ≥ 1:32 | ELISA Pre | titer Post | %with ≥4xrise | titerPre | %with≥ 4x rise Post |
| rHAO 15 | 3.7±0.3 | 9.0±.0.6* | 85 | 92 | 8.7±0.312. | 86 | 5.7±0.3 | 85 |
| µg (26) | | | | | 0±0.3 | | 10.0±0.4 | |
| rHAO 15 | 4.3±0.5 | 8.6±0.4* | 88 | 100 | 9.4±0.411. | 76 | 6.4±0.4 | 76 |
| µg plus | | | | | 5±0.4 | | 9.3±0.2 | |
| alum (26) | | | | | | | | |
| rHAO 90 | 3.3±0.4 | 11.1±0.3 | 100 | 100 | 8.5±0.4 | 100 | 5.7±0.3 | 96 |
| µg (26) | | | | | 13.1±0.4 | | 10.2±0.4 | |
| Licensed | 3.7±0.4 | 9.3±0.5* | 100 | 96 | 8.1±0.4 | 92 | 5.8±0.3 | 96 |
| subviron | | | | | 12.0±0.4 | | 9.9±0.4 | |
| (26) | | | | | | | | |
| Placebo | 3.7±0.5 | 3.8±0.5* | 0 | 38 | 9.1±0.39 | 0 | 5.3±0.4 | 8 |
| (24) | | | | | 9.1±0.3 | | 5.4±0.4 | |

| | | | | | | | | |
|---|---|---|---|---|---|---|---|---|
| HAI, hemagglutination inhibition; HA, hemagglutinin; ELISA, enzyme-linked immunosorbent assay. Post-vaccination serum specimens were obtained three weeks after immunization. Antibody liters are expressed as means reciprocal log₂±SEM. Statistical comparisons are made between the mean postvaccination HAI titer of the designated group and that of the 90 µg rHAO vaccine group by analysis of variance with Dunnett's test for multiple comparisons. *, P<0.01; +, P<0.05; #, P<0.01 | | | | | | | | |

### Example 11: Method for making an improved HAO cloning vector.

An improved cloning vector for expression of mature HA wherein the gene encoding the HA was located immediately downstream of the sequence encoding the chitinase signal peptide was designed.

### Linear pMGS27 with Single-stranded Tails was created

In the pMGS12 plasmid, HA was cloned into Sma1 or *Kpn*1 sites immediately downstream from the chitinase signal peptide. The nucleic and amino acid sequences are shown respectively as SEQ ID NO. 22 and SEQ ID NO. 28:

This region was changed by oligo directed mutagenesis to create pMGS27 (changed bases were underlined) (SEQ ID NO. 24): Plasmid pMGS27 was linearized with Pst1 cut (residues 6-35 of SEQ ID NO. 24 shown): then treating the linear pMGS27 with T4 DNA polymerase plus dATP to create single stranded tails as shown below (residues 23-36 and complement of residues 6-18 of SEQ ID NO. 24): - Target HA Gene was Cloned into pMGS27

Step 1. PCR primers were synthesized.

Forward oligo (SEQ ID NO. 25):
5' GTC GCC GTG TCC AAC GCG (5' end 20 bases of the mature HA)

Reverse oligo (complement of SEQ ID NO. 26):
(3' end 20 bases of the mature HA) ATT AA CCGGTCTCTCCGG 5'
PCR of the HA gene

PCR of the target HA gene with the two oligos was used to obtain (SEQ II7 NO. 25 and SEQ ID NO. 26):

### Anneal target HA gene into pMGS27 and transform E.coli

Linear pMGS27 and the T4 DNA polymerase treated PCR fragment of the HA gene were mixed. The two molecules anneal to each other, to form a circular plasmid which is ready to be used for transforming *E*. *coli.* The diagram includes SEQ ID NOS. 25 and 26, residues 23-36 and 6-18 of SEQ ID NO. 24.

As shown above, there is no extra amino acid in between the signal peptide and the mature HA.

### Example 12: Preparation and efficacy of a Trivalent Types A and B 1995-1996 Influenza Virus Vaccine.

Influenza virus vaccine, purified recombinant hemagglutinin, trivalent, types A and B

Influenza virus vaccine, purified recombinant hemagglutinin, trivalent, types A and B (A/Texas/36/92\1 (H1 N1), A/Johanesburg/33/94 (H3N2), and B/Harbin/7/94) is a non-infectious subunit derived from purified, recombinant influenza hemagglutinin antigens (HA). The HA genes were cloned from the Center for Disease Control/Food and Drug Administration recommended strains of influenza A and B viruses as described above and the identity of each cloned gene determined by DNA sequence analysis. Baculovirus expression vectors containing the cloned HA genes from influenza virus strains A/Texas/36/91 (H1N1), A/Johanesburg/33/94 (H3N2), B/Harbin/7/94 were used to produce the recombinant HA antigens in cultured insect cells. The recombinant HA proteins are full length, uncleaved hemagglu-tinins (rHAO) with a molecular weight of approximately 69,000. The rHAO were produced in a *Spodoptera frugiperda* (Lepidopteran) cell line maintained in a serum-free culture medium. The trivalent vaccines is composed of purified (greater than 95% pure, more probably greater than 99% pure) rHAO from the two influenza A strains and one B strain mixed in equal proportions. The vaccine is supplied for clinical use as purified types A and B rHAO proteins in phosphate buffered saline solution without added preservative.

Animal studies with monovalent, bivalent and trivalent rHAO vaccines have demonstrated that they are free of significant toxicity. There are no detectable toxic or adventitious agents in the vaccine. General safety and immunogenicity studies of A/Beijing/32/92 and A/Texas/36/91 rHAO were conducted in mice and guinea pigs. No adverse reactions were noted. In mice, a single immunization with 15 micrograms of rHAO antigens without adjuvant induces in two to three weeks high levels of anti-HA IgG antibodies, hemagglutinin inhibition (HAI) antibodies and neutralizing antibodies.

In one study, groups of ten mice were immunized with 15 micrograms of purified rHAO A/Beijing/32/92 (H3N2) made in cells adapted to media containing 10% fetal bovine serum or rHAO made in insect cells adapted to media containing 10% fetal bovine serum or rHAO made in insect cells adapted to a serum-free medium (rHAO-SF). Two and three weeks post injection the mice were bled and serum samples prepared. Each sera were measured for anti-HA IgG and HAI antibodies. Both rHAO and rHAO-SF antigens elicit similar titers of anti-HA and HAI antibodies. Both rHAO and rHAO-SF antigens elicit similar titers of anti-HA and HAI antibodies. Two weeks following the single immunization, most of the mice have significant titers of HAI antibodies and by week three 8/10 mice in each group had HAI titers of 32 or greater. These and other biochemical and immunological studies demonstrate that rHAO produced in serum-free insect cell culture is indistinguishable from rHAO manufactured under serum-containing fermentation conditions.

A study was conducted to compare the 1994-1995 formulation of the trivalent rHAO influenza vaccine with a licensed purified virus surface antigen vaccine, Fluvlrin® (an attenuated influenza viral vaccine produced by culturing in eggs). Each vaccine contained 15 micrograms rHAO orviral HA per 0.5 ml from A/Texas/36/91 (H1N1),A/Shangdong/ 9/93 (H3N2), and B/Panama/45/90 influenza strains. Both the recombinant rHAO and

**Table 5:**

| **Comparison of trivalent rHAO vaccine with Fluvirin®.** | | | | |
|---|---|---|---|---|
| **GMT (n=10 mice)** | **Trivalent rHAO Fluvirin® Influenza vaccine GMT (n=10 mice)** | | | |
| **Virus strain** | **anti-HA IgG** | | **anti-HA IgG** | |
| **used as antigen** | **week 0** | **week 3** | **week 0** | **week 3** |
| A/Texas/36/91 (H1N1) | <1000 | 103,000 | <1000 | 11,200 |

## Claims

1. A substantially pure, recombinant, glycosylated influenza haemagglutinin (rHAo) produced by a baculovirus expression system in cultured insect cells, **characterised in that** the haemagglutinin protein is purified to at least 95% and **in that** the protein is immunogenic, induces a protective immune response when used as a vaccine and further comprises a baculovirus chitinase signal peptide.

2. A haemagglutinin according to claim 1 **characterised in that** the haemagglutinin protein further comprises a second protein which is fused to the haemagglutinin.

3. A haemagglutinin according to claim 2, **characterised in that** the second protein is selected from the group consisting of a hepatitis B viral protein, a HIV protein, a carcinoembryonic antigen, and a neuraminidase.

4. A haemagglutinin according to any preceding claim wherein the chitinase signal peptide is coupled directly to the haemagglutinin protein without intervening amino acids.

5. A haemagglutinin according to any preceding claim wherein the influenza is selected from the group consisting of influenza A virus strains and influenza B virus strains.

6. A haemagglutinin according to claim 5, wherein the virus infects humans.

7. A vaccine composition comprising a haemagglutinin according to any preceding claim, and
(i) a pharmaceutically acceptable carrier, or
(ii) an adjuvant, or
(iii) a pharmaceutically acceptable carrier and an adjuvant.

8. A vaccine composition according to claim 7 wherein the pharmaceutically acceptable carrier is a polymeric delivery system.

9. A method for making the hemagglutinin protein of claim 2, said method comprising infecting cultured insect cells with a vector containing the following sequences in 5' -> 3' order: a polyhedron promoter from a baculovirus, an ATG translational start codon, a coding sequence for a chitinase signal peptide, the coding sequence for hemagglutinin from a strain of influenza selected from the group consisting of an influenza A strain and an influenza B strains, coding sequence for the second protein, a translational termination codon, and a polyhedron RNA polyadenylation signal, and culturing the cells in a nutrient media.

10. A recombinant influenza hemagglutinin fusion protein expressed in a baculovirus expression system cultured in insect cells prepared by the method of claim 9 and purified to at least 95% and comprising a baculovirus chitinase signal peptide.

11. The method of claim 9 further comprising isolating the HAO influenza haemagglutinin fusion protein from cells to a purity of at least 95%.

12. The method of claim 11, wherein the mature, glycosylated hemagglutinin fusion protein is isolated by separating membrane-bound proteins including the mature, glycosylated hemagglutinin fusion protein from non-membrane proteins at alkaline pH, washing the membrane bound proteins to elute the mature, glycosylated hemagglutinin fusion protein, separating the eluted mature, glycosylated hemagglutinin fusion protein by an anion exchange resin with a change in pH, and separating the anion exchange passaged mature, glycosylated hemagglutinin fusion protein by a cation exchange resin with a change in salt concentration.

13. A method for making a vector containing the following 5'-> 3' sequences: a polyhedrin promoter from a baculovirus, an ATG translational start codon, a coding sequence for a chitinase signal peptide, the coding sequences from hemagglutinin from a strain of influenza selected from the group consisting of Influenza A strains and Influenza B strains, a translational termination codon, and a polyhedrin RNA polyadenalytion signal comprising harvesting virus from the cell media and isolating either viral RNA, for Influenza A strains, or mRNA, for influenza B strains;
synthesizing cDNA using either a universal primer (5'AGCAAAAGGAGG-3' (SEQ ID No.1)) for the viral RNA from the Influenza A strains or random primers for the mRNA from influenza B strains, wherein the 5' and 3' primers have restriction enzyme sites at the ends that are not found within the hemagglutinin genes;
amplifying the Influenza A or B primers and Influenza cDNA mixed with the hemagglutinin gene segments to produce double-stranded DNA fragments containing entire mature hemagglutinin coding sequences;
identifying the signal peptide of the hemagglutinin genes then amplifying the hemaglutinin genes minus the hemagglutinin signal peptide; and
cloning the hemagglutinin genes minus the signal peptide into a vector containing the AcNPV polyhedrin promoter and a coding sequence for a baculovirus chitinase signal peptide.

14. The method of claim 13 wherein the hemagglutinin genes are cloned into the vector using PCR so that the vector encodes the signal peptide coupled directly to the hemagglutinin without any intervening amino acids.

15. The method of claim 13 further comprising transfecting the vector into insect cells, and selecting cells for hemagglutinin expression.

## Patentansprüche

1. Ein im Wesentlichen reines, rekombinantes, glycosyliertes Influenza-Hämagglutinin (rHAo), was durch ein Baculovirus-Expressionssystem in kultivierten Insektenzellen hergestellt wird, **dadurch gekennzeichnet, dass** das Hämagglutinin-Protein zu mindestens 95% gereinigt ist und dass das Protein immunogen ist, eine schützende Immunantwort induziert, wenn es als ein Impfstoff verwendet wird, und ferner ein Baculovirus-Chitinase-Signalpeptid umfasst.

2. Ein Hämagglutinin gemäß Anspruch 1, **dadurch gekennzeichnet, dass** das Hämagglutinin-Protein ferner ein zweites Protein umfasst, das mit dem Hämagglutinin fusioniert ist.

3. Ein Hämagglutinin gemäß Anspruch 2, **dadurch gekennzeichnet, dass** das zweite Protein ausgewählt ist aus der Gruppe bestehend aus einem viralen Hepatitis B Protein, einem HIV Protein, einem carcinoembryonalem Antigen und einer Neuraminidase.

4. Ein Hämagglutinin gemäß einem der vorhergehenden Ansprüche, wobei das Chitinase-Signalpeptid direkt an das Hämagglutinin-Protein gekoppelt ist ohne dazwischen liegenden Aminosäuren.

5. Ein Hämagglutinin gemäß einem der vorhergehenden Ansprüche, wobei die Influenza ausgewählt ist aus der Gruppe bestehend aus Influenza A Virusstämmen und Influenza B Virusstämmen.

6. Ein Hämagglutinin gemäß Anspruch 5, wobei das Virus Menschen infiziert.

7. Eine Impfstoff Zusammensetzung umfassend ein Hämagglutinin gemäß einem der vorhergehenden Ansprüchen und (i) einen pharmazeutisch annehmbaren Trägerstoff oder (ii) ein Adjuvans oder (iii) einen pharmazeutisch annehmbaren Trägerstoff und ein Adjuvans.

8. Eine Impfstoff Zusammensetzung gemäß Anspruch 7, wobei der pharmazeutisch annehmbare Trägerstoff ein polymeres Abgabesystem ist.

9. Ein Verfahren zur Herstellung des Hämagglutinins gemäß Anspruch 2, wobei das Verfahren das Infizieren von, kultivierten Insektenzellen mit einem Vektor, der die folgenden Sequenzen in 5'→3' Richtung enthält: ein Polyhedrin-Promotor aus einem Baculovirus, ein ATG translationales Startcodon, eine kodierende Sequenz für ein Chitinase-Signalpeptid, die kodierende Sequenz für Hämagglutinin von einem Influenzastamm, ausgewählt aus der Gruppe bestehend aus einem Influenza A Stamm und einem Influenza B Stamm, der kodierende Sequenz für das zweite Protein, ein translationales Terminationscodon und ein Polyhedrin-RNA-Polyadenylierungssignal und Kultivieren der Zellen in einem Nährmedium umfasst.

10. Ein rekombinantes Influenza-Hämagglutinin-Fusionsprotein, das in einem Baculovirus-Expressionssystem kultiviert in Insektenzellen exprimiert wird und durch das Verfahren gemäß Anspruch 9 hergestellt wird und bis zu mindestens 95% gereinigt ist und ein Baculovirus-Chitinase-Signalpeptid umfasst.

11. Das Verfahren gemäß Anspruch 9 ferner umfassend das Isolieren des HAO Influenza-Hämagglutinin-Fusionsproteins aus Zellen bis zu einer Reinheit von mindestens 95%.

12. Das Verfahren gemäß Anspruch 11, wobei das reife, glycosylierte Hämagglutinin-Fusionsprotein isoliert wird, indem Membran-gebundene Proteine, einschließlich der reifen, glycolsylierten Hämagglutinin-Fusionsproteine von nicht-Membran-gebundenen Proteinen bei einem alkalischen pH abgetrennt werden, die Membran-gebundenen Proteine gewaschen werden, um die reifen, glycosylierten Hämagglutinin-Fusionsproteine zu eluieren, das eluierte, reife, glycosylierte Hämagglutinin-Fusionsprotein durch ein Anionenaustausch-Harz mit einer Änderung des pH abgetrennt wird, und das reife, glycosylierte Hämagglutinin-Fusionsprotein nach dem Anionenaustausch durch ein Kationenaustausch-Harz mit einer Änderung der Salzkonzentration abgetrennt wird.

13. Ein Verfahren zur Herstellung eines Vektors, der die folgenden 5'→3' Sequenzen enthält: ein Polyhedrin-Promotor aus einem Baculovirus, ein ATG translationales Startcodon, eine kodierende Sequenz für ein Chitinase-Signalpeptid, die kodierende Sequenz für Hämagglutinin aus einem Influenzastamm, ausgewählt aus der Gruppe bestehend aus einem Influenza A Stamm und einem Influenza B Stamm, ein translationales Terminationscodon und ein Polyhedrin-RNA-Polyadenylierungssignal umfassend das Ernten von Viren aus Zellmedien und das Isolieren von entweder viraler RNA, für Influenza A Stämme, oder mRNA, für Influenza B Stämme;
Synthetisieren von cDNA entweder mittels Verwendung eines Universalprimers (5'AGCAAAAGGAGG-3' (SEQ ID No.1)) für die virale RNA von Influenza A Stämmen oder von Random-Primern für mRNA von Influenza B Stämmen, wobei die 5' und 3' Primer Restriktionsenzymstellen an den Enden aufweisen, die nicht innerhalb der Hämagglutinin-Gene zu finden sind;
Amplifizieren der Influenza A oder B Primer und Influenza cDNA vermischt mit dem Hämagglutinin-Gensegment um doppelsträngige DNA Fragmente herzustellen, welche die komplette reife, kodierende Hämagglutinin-Sequenz enthalten;
Identifizieren des Signalpeptids der Hämagglutinin-Gene und dann Amplifizieren der Hämagglutinin-Gene abzüglich des Signalpeptids; und
Klonieren der Hämagglutinin-Gene abzüglich des Signalpeptids in einen Vektor, der den AcNPV Polyhedrin Promotor und eine kodierende Sequenz für ein Baculovirus-Chitinase-Signalpeptid enthält.

14. Das Verfahren gemäß Anspruch 13, wobei die Hämagglutinin-Gene unter Verwendung von PCR so in einen Vektor kloniert werden, dass der Vektor für ein Signalpeptid kodiert, das direkt an das Hämagglutinin gekoppelt ist, ohne dazwischen liegenden Aminosäuren.

15. Das Verfahren gemäß Anspruch 13, ferner umfassend Transfizieren des Vektors in Insektenzellen und Selektieren von Zellen auf Hämagglutinin-Expression.

## Revendications

1. Hémagglutinine de la grippe glycosylée, recombinante et essentiellement pure (rHAo) produite par un système d'expression baculovirus dans des cellules d'insecte en culture, **caractérisée en ce que** la protéine hémagglutinine est purifiée à au moins 95 % et **en ce que** la protéine est immunogène, induit une réponse immunitaire protectrice lorsqu'elle est utilisée en tant que vaccin et comprend en outre un peptide signal de la chitinase de baculovirus.

2. Hémagglutinine selon la revendication 1, **caractérisée en ce que** la protéine hémagglutinine comprend en outre une seconde protéine qui est fusionnée à l'hémagglutinine.

3. Hémagglutinine selon la revendication 2, **caractérisée en ce que** la seconde protéine est sélectionnée dans le groupe consistant en une protéine virale de l'hépatite B, une protéine du VIH, un antigène carcino-embryonnaire et une neuraminidase.

4. Hémagglutinine selon l'une quelconque des revendications précédentes, où le peptide signal de la chitinase est directement couplé à la protéine hémagglutinine sans acides aminés intermédiaires.

5. Hémagglutinine selon l'une quelconque des revendications précédentes, où la grippe est sélectionnée dans le groupe consistant en des souches du virus de la grippe A et des souches du virus de la grippe B.

6. Hémagglutinine selon la revendication 5, où le virus infecte les humains.

7. Composition vaccinale comprenant une hémagglutinine selon l'une quelconque des revendications précédentes, et
(i) un véhicule pharmaceutiquement acceptable, ou
(ii) un adjuvant, ou
(iii) un véhicule pharmaceutiquement acceptable et un adjuvant.

8. Composition vaccinale selon la revendication 7, où le véhicule pharmaceutiquement acceptable est un système de délivrance polymérique.

9. Procédé pour fabriquer la protéine hémagglutinine selon la revendication 2, ledit procédé comprenant l'infection de cellules d'insecte en culture avec un vecteur contenant les séquences suivantes, dans l'ordre 5' → 3' : un promoteur de la polyhédrine d'un baculovirus, un codon d'initiation de la traduction ATG, une séquence codant pour un peptide signal de la chitinase, la séquence codant pour une hémagglutinine issue d'une souche de la grippe sélectionnée dans le groupe consistant en des souches de la grippe A et des souches de la grippe B, une séquence codant pour la seconde protéine, un codon de terminaison de la traduction et un signal de polyadénylation d'un ARN de la polyhédrine et la mise en culture des cellules dans un milieu nutritif.

10. Protéine de fusion d'hémagglutinine de la grippe recombinante exprimée dans un système d'expression baculovirus cultivé dans des cellules d'insecte, préparée par le procédé selon la revendication 9 et purifiée à au moins 95 % et comprenant un peptide signal de la chitinase de baculovirus.

11. Procédé selon la revendication 9, comprenant en outre l'isolation de la protéine de fusion d'hémagglutinine de la grippe HAO à partir des cellules à une pureté d'au moins 95 %.

12. Procédé selon la revendication 11, dans lequel la protéine de fusion d'hémagglutinine glycosylée mature est isolée en séparant les protéines liées à la membrane, comprenant la protéine de fusion d'hémagglutinine glycosylée mature, des protéines non membranaires à un pH alcalin, en lavant les protéines liées à la membrane afin d'éluer la protéine de fusion d'hémagglutinine glycosylée mature, en séparant la protéine de fusion d'hémagglutinine glycosylée mature éluée par une résine échangeuse d'anions avec une modification du pH, et en séparant la protéine de fusion d'hémagglutinine glycosylée mature ayant été soumise à un échange d'anions par une résine échangeuse de cations avec une modification de la concentration en sel.

13. Procédé pour fabriquer un vecteur contenant les séquences 5' → 3' suivantes : un promoteur de la polyhédrine d'un baculovirus, un codon d'initiation de la traduction ATG, une séquence codant pour un peptide signal de la chitinase, les séquences codant pour une hémagglutinine issue d'une souche de la grippe sélectionnée dans le groupe consistant en des souches de la grippe A et des souches de la grippe B, un codon de terminaison de la traduction et un signal de polyadénylation d'un ARN de la polyhédrine, comprenant la récolte du virus à partir du milieu cellulaire et l'isolation de l'ARN viral, pour les souches de la grippe A, ou de l'ARNm, pour les souches de la grippe B ;
la synthèse d'un ADNc en utilisant une amorce universelle (5'-AGCAAAAGGAGG-3' (SEQ ID NO: 1)) pour l'ARN viral des souches de la grippe A ou des amorces aléatoires pour l'ARNm des souches de la grippe B, où les amorces 5' et 3' possèdent des sites d'enzyme de restriction aux extrémités qui ne sont pas observés au sein des gènes de l'hémagglutinine ;
l'amplification des amorces de la grippe A ou B et de l'ADNc de la grippe mélangés avec des segments de gène de l'hémagglutinine afin de produire des fragments d'ADN double brin contenant des séquences codant pour une hémagglutinine mature entière ;
l'identification du peptide signal des gènes de l'hémagglutinine puis l'amplification des gènes de l'hémagglutinine moins le peptide signal de l'hémagglutinine ; et
le clonage des gènes de l'hémagglutinine moins le peptide signal dans un vecteur contenant le promoteur de la polyhédrine de l'AcNPV et une séquence codant pour un peptide signal de la chitinase de baculovirus.

14. Procédé selon la revendication 13, dans lequel les gènes de l'hémagglutinine sont clonés dans le vecteur en utilisant une PCR de sorte que le vecteur code pour le peptide signal directement couplé à l'hémagglutinine sans aucun acide aminé intermédiaire.

15. Procédé selon la revendication 13, comprenant en outre la transfection du vecteur dans des cellules d'insecte et la sélection des cellules pour l'expression de l'hémagglutinine.
